# EUROPEAN PATENT SPECIFICATION

(11) **EP 4 164 963 B1**
(45) Date of publication and mention of the grant of the patent: **30.09.2026**
(21) Application number: 21746844.6
(22) Date of filing: 16.06.2021
(51) Int. Cl.: B65D 81/28

(54) **PACKAGES COMPRISING ANTI-MICROBIAL COATINGS FOR PREVENTING CONTAMINATION, E.G. AFTER FIRST USE OF THE PRODUCT**
VERPACKUNGEN MIT ANTIMIKROBIELLEN BESCHICHTUNGEN ZUR VERHINDERUNG VON KONTAMINATION, Z. B. NACH DER ERSTEN VERWENDUNG DES PRODUKTS
EMBALLAGES COMPRENANT DES REVÊTEMENTS ANTIMICROBIENS POUR EMPÊCHER LA CONTAMINATION, PAR EXEMPLE APRÈS LA PREMIÈRE UTILISATION DU PRODUIT

(30) Priority: 16.06.2020 US 202063039666 P; 14.12.2020 US 202063125231 P
(43) Date of publication of application: 19.04.2023
(73) Proprietor: SiO2 Medical Products, Inc., Auburn, AL 36832 (US)
(72) Inventor: WEIKART, Christopher, Auburn, Alabama 36832 (US); CLARK, Becky L., Auburn, Alabama 36832 (US); STEVENSON, Adam, Auburn, Alabama 36832 (US); FELTS, John T., Auburn, Alabama 36832 (US); TAHA, Ahmad, Auburn, Alabama 36832 (US); ABRAMS, Robert S., Auburn, Alabama 36832 (US)
(74) Representative: Maiwald GmbH
(86) International application number: PCT/US2021/037667
(87) International publication number: WO 2021/257728

(56) References cited:
- EP-A1- 1 466 668
- WO-A1-96/36371
- DE-A1- 102017 105 246
- US-A1- 2002 051 754
- US-A1- 2006 246 149
- US-A1- 2012 225 185

## Description

### PRODUCT

The present application claims priority to United States Provisional Patent Application No. 63/039,666, filed on June 16, 2020, and United States Provisional Patent Application No. 63/125,231, filed on December 14, 2020.

### FIELD OF THE INVENTION

The present invention relates to the technical field of multi-dose packages containing products that are used over a period of time, rather than all at once. The packages include but are not limited to multi-dose medicine containers, multi-dose eye drop bottles, multi-dose nasal spray bottles, multi-dose inhalers, cosmetic containers, skin cream or ointment containers, fragrance containers, and the like. In particular, the interior surfaces of these multi-dose packages may be treated with an anti-microbial coating that prevents contamination of the container contents, once the container has been opened.

The present invention also relates to the technical field of barrier coated surfaces, for example interior surfaces of fragrance and/or cosmetic packages, including for example the multdose containers described above. Examples of suitable fluids include fragrances, perfumes, dry powder cosmetics, moist cosmetics, solutions, and liquids. The present invention also relates to a fragrance and/or cosmetic package or other vessel and to a method for making a fragrance and/or cosmetic package with a pH protective coating or layer between the contents and the barrier coating or layer.

The present disclosure also relates to improved methods for processing multi-dose packages, fragrance, and/or cosmetic packages, for example multiple identical multi-dose, fragrance, and/or cosmetic packages. The resulting packages are also claimed. Such multi-dose, fragrance, and/or cosmetic packages are used in large numbers, and must be relatively economical to manufacture and yet highly reliable in storage and use.

### BACKGROUND OF THE INVENTION

One important consideration for multi-dose packages is that the product within the package remains uncontaminated by microbes, such as bacteria and the like. Many products are aseptic or sterile when packaged but, after one or more uses, can become contaminated with bacteria. As a result, many multi-dose product packages have a limited shelf-life after first opening/use.

To avoid contamination, it is typically recommended that a user of a multi-dose cosmetic package wash his/her hands prior to application. However, in the United States, there are no requirements that a cosmetic manufacturer print a post-opening shelf life or expiration date on a cosmetic package. In Europe, cosmetic products with a lifespan longer than 30 months must show a "period after opening" (POA) time, i.e. a post-opening shelf-life, which is the time (usually in months) when the product will remain in good condition after the consumer has opened and used the product for the first time. The POA is typically provided as a number - which identifies the number of months - positioned on a symbol of an open cream jar. This symbol is sometimes used on cosmetic products in the United States as well. Similarly, in Europe, any cosmetic product that has a lifespan less than 30 months (which are less common) must show a "best before the end of" date, which is typically shown using an "egg timer" symbol followed by the date, or the words, which can be abbreviated to BBE or Exp, followed by the date.

For instance, many cosmetics packages for creams and lotions require a user to dip his/her fingers into the container to extract an amount of the product, which directly introduces microorganisms, e.g. bacteria, fungi, etc., into the product. To combat this, cosmetic manufacturers will often include preservatives in the product. However, preservatives break down over time and/or can cause adverse reactions by certain users.

The shelf life for certain products, including for example eye-area products and cosmetics, e.g. eye drops, mascara, eye liner, etc., is also often limited because the applicators are susceptible to microbial infection during use, which increases the risk of causing an eye infection. For example, manufacturers usually recommend discarding mascara three to six months after purchase. The same may be true of products applied to the lips, e.g. lip glosses, lipsticks, etc.

A recent study of used lipstick, lip gloss, eyeliner, and mascara products showed that about 79-90% of those products were contaminated with significant levels of microbial contamination, with bacterial loads ranging between 10² and 10³ CFU per mL. Detected bacteria included Staphylococcus aureus, Escherichia coli and Citrobacter freundii. Enterobacteriaceae and fungi were also detected in the used products. See Bashir et al., "Microbiological study of used cosmetic products: highlighting possible impact on consumer health," J. Appl. Microbiol., vol. 128(2), pp. 598-605 (2020).

Embodiments of the present disclosure are therefore directed to a multi-dose (also referred to as multi-use) product packages comprising an anti-microbial coating to protect the product from microbial, e.g. bacterial, fungal, etc., contamination during use and to extend the shelf-life of the product package after first opening/use.

Another important consideration for many multi-dose product packages, cosmetic, and/or fragrance packages, is that the contents of the package have a substantial pre-opening shelf life. During this pre-opening shelf life, it may be desirable to isolate the product from the vessel wall containing it, or from barrier layers or other functional layers applied to the vessel wall to avoid leaching material from the vessel wall, barrier layer, or other functional layers into the prefilled contents or vice versa.

Traditional glass multi-dose, fragrance, and/or cosmetic packages or other vessels are prone to breakage or degradation during manufacture, filling operations, shipping and use, which means that glass particulates may enter the multi-dose, fragrance, and/or cosmetic. The presence of glass particles has led to many FDA Warning Letters and to product recalls. As a result, some companies have turned to plastic multi-dose, fragrance, and/or cosmetic packages, which provide greater dimensional tolerance and less breakage than glass, but their use may remain limited due to their gas (oxygen) permeability: plastic allows small molecule gases to permeate into (or out of) the article, decreasing the pre-opening shelf-life of the package. The permeability of plastics to gases is significantly greater than that of glass and, in many cases, plastics have been unacceptable for that reason.

Fragrance and/or cosmetic compositions contained in the novel packages of the present invention may be suitable for application to a wide variety of substrates but particularly to the skin and hair. The compositions, in particular cosmetic compositions, may comprise a mascara composition, such as that of the type commonly used to enhance lenghthening and beautiful curvature on eyelashes, or a crème, such as a face or body crème or a hair crème. Such mascara compositions or crèmes may contain one or more waxes, a film-forming polymer, a silicone, a natural or synthetic latex or pseudolatex, and agents for suspending the latex and the silicone and/or a thickening agent, among other compositional materials or admixtures.

Other cosmetic compositions, more particularly fragrance compositions, may comprise a fragrance oil, an entrapment material and greater than 50% volatile solvent, wherein the fragrance oil comprises both "top note" and "middle and base note" perfume raw materials wherein the weight ratio of the two types of fragrance raw materials is in the range from about 1:20 to about 20:1. More particularly, the fragrance character of the "top note" perfume raw materials remains detectable on the substrate for at least 2 hours after application.

A prior art container comprising an anti-bacterial coating containing silver ions is disclosed in US 2012/225185 A1.

### SUMMARY OF THE INVENTION

The invention relates to a package according to appended claim 1. An aspect of the invention is a vessel having a lumen defined at least in part by a wall, the wall having an interior surface facing the lumen, an outer surface, and a coating set on the interior surface comprising an anti-microbial coating and optionally a barrier coating.

Embodiments of the present disclosure are directed to a package comprising: a vessel comprising one or more walls that enclose at least a portion of a lumen; a fluid within the lumen, the fluid being present in an amount that is configured for a plurality of doses or applications, optionally wherein the fluid is a drug or medical product, optionally wherein the fluid is a cosmetic product, optionally wherein the fluid is a skin care product; an anti-microbial coating on an interior surface of the one or more walls, wherein the anti-microbial coating is in contact with the fluid; and wherein the anti-microbial coating is effective to inhibit the growth of microbes, such as bacteria, in the fluid within the lumen.

Embodiments of the present disclosure are directed to a package comprising: a vessel comprising one or more walls that enclose at least a portion of a lumen; an aseptic or sterile fluid within the lumen, the fluid being present in an amount that is configured for a plurality of doses or applications, optionally wherein the fluid is a drug or medical product, optionally wherein the fluid is a cosmetic product, optionally wherein the fluid is a skin care product; an anti-microbial coating on an interior surface of the one or more walls, wherein the anti-microbial coating is in contact with the fluid; and wherein the anti-microbial coating is effective to inactivate or kill bacteria introduced into the lumen.

Embodiments of the present disclosure are directed to a package comprising: a vessel comprising one or more walls that enclose at least a portion of a lumen; an aseptic or sterile fluid within the lumen, the fluid being present in an amount that is configured for a plurality of doses or applications, optionally wherein the fluid is a drug or medical product, optionally wherein the fluid is a cosmetic product, optionally wherein the fluid is a skin care product; an applicator for the fluid, wherein the applicator is susceptible to bacterial contamination upon use; and an antimicrobial coating on an interior surface of the one or more walls, wherein the anti-microbial coating is in contact with the fluid; wherein the anti-microbial coating is effective to increase the shelf-life of the package after first use, optionally by at least one week (also referred to herein as the post-opening shelf life), optionally at least two weeks, optionally at least one month, optionally at least two months, optionally at least three months, optionally at least four months, optionally at least five months, optionally at least six months, optionally at least nine months, optionally at least one year.

Embodiments of the present disclosure are directed to a multi-dose eye drop bottle comprising: a vessel comprising one or more walls that enclose at least a portion of a lumen; a dropper tip at the opening of the lumen; an ophthalmic medical fluid within the lumen; and an anti-microbial coating on an interior surface of the one or more walls and in contact with the fluid; wherein the anti-microbial coating is effective to inactivate or kill bacteria introduced into the lumen.

Embodiments of the present disclosure are directed to a multi-dose eye drop bottle comprising: a vessel comprising one or more walls that enclose at least a portion of a lumen; a dropper tip at the opening of the lumen; an ophthalmic medical fluid within the lumen; and an anti-microbial coating on an interior surface of the one or more walls and in contact with the fluid; and wherein the anti-microbial coating is effective to increase the shelf-life of the multi-dose eye drop bottle after first use (also referred to herein as the post-opening shelf life), optionally by at least one week, optionally at least two weeks, optionally at least one month, optionally at least two months, optionally at least three months, optionally at least four months, optionally at least five months, optionally at least six months, optionally at least nine months, optionally at least one year.

Many additional and alternative aspects and embodiments of the invention are also contemplated, and are described in the specification and claims that follow. Some optional features include the following:

The package may further comprise an applicator for the fluid, and wherein the applicator is susceptible to bacterial contamination.

The package may comprise a dropper tip, a dropper cap, a dropper, or a plunger-operated applicator.

The vessel may comprise a nasal spray applicator, such as spray top, such as wherein the vessel is a nasal spray bottle.

The vessel may be a multi-dose eye dropper bottle.

The fluid may be a liquid formulation of a drug, optionally a liquid formulation of a drug configured for ocular or nasal administration, optionally a liquid formulation of a drug configured for ocular administration, optionally a liquid formulation of a drug configured for nasal administration.

The fluid may comprise an ophthalmic drug formulation.

The fluid may comprise a locally-acting nasal drug, optionally a nasal decongestant.

The vessel may be a multi-dose inhaler, e.g. a pressurized metered dose inhaler.

The anti-microbial coating may be effective to increase the shelf-life of the package after first use (also referred to herein as the post-opening shelf life), optionally wherein the anti-microbial coating is effective to increase the shelf-life of the package after first use by at least one week, optionally at least two weeks, optionally at least one month, optionally at least two months, optionally at least three months, optionally at least four months, optionally at least five months, optionally at least six months, optionally at least nine months, optionally at least one year.

The vessel may be cosmetics container, optionally a mascara bottle or tube, optionally an eye liner bottle or tube, optionally a lip gloss bottle or tube.

The applicator may be a makeup applicator, optionally an eyelash brush.

The fluid may be a mascara composition.

The fluid may be a liquid or gel eye liner and the applicator is an eye liner brush.

The fluid may be a lip gloss and the applicator is a lip gloss brush or pad.

The vessel may be a contact lens container.

The fluid may be a contact lens solution.

The vessel may be a bottle and the fluid is a contact lens solution or a saline solution.

THe fluid may be configured to be applied to a person's skin, and optionally wherein the fluid is a cream, ointment, or topical medication.

The fluid may be an edible food product, optionally a spreadable food product, optionally a spreadable condiment.

The anti-microbial coating comprises zinc oxide, titanium dioxide, or silver oxide, optionally wherein the anti-microbial coating comprises zinc oxide, optionally wherein the anti-microbial coating comprises titanium dioxide, optionally wherein the anti-microbial coating comprises silver oxide.

The anti-microbial coating is applied by PECVD, ALD, PEALD, sputtering, evaporation, or sintering, optionally wherein the anti-microbial coating is applied by PECVD, ALD, or PEALD, optionally wherein the anti-microbial coating is applied by PECVD, optionally wherein the anti-microbial coating is applied by ALD, optionally wherein the anti-microbial coating is applied by PEALD.

The anti-microbial coating may consist essentially of a plurality of atomic monolayers, optionally wherein the anti-microbial coating or layer is deposited by atomic layer deposition, optionally by plasmaassisted atomic layer deposition.

The anti-microbial coating may have a thickness between about 1 nm and about 1000 nm, optionally between about 2 nm and about 1000 nm, optionally between about 5 nm and about 1000 nm, optionally between about 10 nm and 1000 nm, optionally between about 1 nm and about 500 nm, optionally between 2 nm and about 500 nm, optionally between about 5 nm and about 500 nm, optionally between about 10 nm and 500 nm, optionally between about 1 nm and about 250 nm, optionally between about 2 nm and about 250 nm, optionally between about 5 nm and about 250 nm, optionally between about 10 nm and 250 nm, optionally between about 1 nm and about 100 nm, optionally between about 2 nm and about 100 nm, optionally between about 5 nm and about 100 nm, optionally between about 10 nm and 100 nm, optionally between about 1 nm and about 50 nm, optionally between about 2 nm and about 50 nm, optionally between about 5 nm and about 50 nm, optionally between about 10 nm and about 50 nm.

The anti-microbial coating may comprise zinc oxide (ZnO) applied by PECVD from a feed gas comprising zinc acetate, diethyl zinc, or a combination thereof, and an oxidant.

The anti-microbial coating may comprise titanium dioxide (TiO₂) applied by PECVD from a feed gas comprising titanium tetra chloride and an oxidant.

The anti-microbial coating may comprise silver oxide (Ag₂O) applied by PECVD from a feed gas comprising an organosilver compound and an oxidant, optionally wherein the organosilver compound has the composition:

Ag(Hfac)(PR₃)

in which Hfac is 1,1,1,5,5,5-hexafluoroacetylacetonate, P is phosphine, and R is methyl, ethyl, or a combination thereof.

The anti-microbial coating may comprise zinc oxide (ZnO) applied by ALD or PEALD using feed gases comprising zinc acetate, diethyl zinc, or a combination thereof, and an oxidant.

The anti-microbial coating may comprise titanium dioxide (TiO2) applied by ALD or PEALD using feed gases comprising titanium tetra chloride, titanium isopropoxide, or a combination thereof, and an oxidant.

The anti-microbial coating may comprise silver oxide (Ag2O) applied by PECVD using feed gases comprising an organosilver compound and an oxidant, optionally wherein the organosilver compound has the composition:

Ag(Hfac)(PR₃)

in which Hfac is 1,1,1,5,5,5-hexafluoroacetylacetonate, P is phosphine, and R is methyl, ethyl, or a combination thereof.

The oxidant may be selected from O₂, O₃, H₂O, H₂O₂, N₂O, NO₂, air, or a combination thereof, optionally in which the oxidant is O₂.

The vessel wall may further comprise a barrier coating, and optionally in which the barrier coating may be part of a coating set comprising a tie coating or layer, a barrier coating or layer, and a pH protective coating or layer.

The tie coating or layer can comprise SiOₓC_{y} or Si(NH)ₓC_{y}. In either formulation, x is from about 0.5 to about 2.4 and y is from about 0.6 to about 3. The tie coating or layer has an interior surface facing the lumen and an outer surface facing the wall interior surface.

The barrier coating or layer can comprise SiOₓ, wherein x is from 1.5 to 2.9. The barrier layer can be from 2 to 1000 nm thick. It can have an interior surface facing the lumen and an outer surface facing the interior surface of the tie coating or layer. The barrier coating or layer optionally is effective to reduce the ingress of atmospheric gas into the lumen compared to an vessel without a barrier coating or layer.

The pH protective coating or layer can comprise SiOₓC_{y} or Si(NH)ₓC_{y}, where x is from about 0.5 to about 2.4 and y is from about 0.6 to about 3. The pH protective coating or layer can have an interior surface facing the lumen and an outer surface facing the interior surface of the barrier coating or layer.

In an embodiment, in the presence of a fluid composition contained in the lumen and having a pH between 5 and 9, the calculated shelf life of the package can be more than six months at a storage temperature of 4°C.

At least a portion of the wall of the vessel comprises a thermoplastic polymer, optionally in which the vessel wall is made of a thermoplastic polymer.

The anti-microbial coating and/or the barrier coating or layer is from 4 nm to 500 nm thick.

The pH protective coating or layer comprises SiOₓC_{y}.

The pH protective coating or layer is applied by PECVD of a precursor feed comprising an acyclic siloxane, a monocyclic siloxane, a polycyclic siloxane, a polysilsesquioxane, a monocyclic silazane, a polycyclic silazane, a polysilsesquiazane, a silatrane, a silquasilatrane, a silproatrane, an azasilatrane, an azasilquasiatrane, an azasilproatrane, or a combination of any two or more of these precursors.

The pH protective coating or layer is applied by PECVD of a precursor feed comprising octamethylcyclotetrasiloxane (OMCTS).

The pH protective coating or layer as applied is between 10 and 1000 nm thick.

The rate of erosion of the pH protective coating or layer, if directly contacted by a fluid composition having a pH of 8, is less than 20% of the rate of erosion of the barrier coating or layer, if directly contacted by the same fluid composition under the same conditions.

The pH protective coating or layer is at least coextensive with the barrier coating or layer.

The fluid composition removes the pH protective coating or layer at a rate of 1 nm or less of pH protective coating or layer thickness per 44 hours of contact with the fluid composition.

An FTIR absorbance spectrum of the pH protective coating or layer has a ratio greater than 0.75 between:
- the maximum amplitude of the Si-O-Si symmetrical stretch peak between about 1000 and 1040 cm-1, and
- the maximum amplitude of the Si-O-Si assymmetric stretch peak between about 1060 and about 1100 cm-1.

The silicon dissolution rate by a 50 mM potassium phosphate buffer diluted in water for injection, adjusted to pH 8 with concentrated nitric acid, and containing 0.2 wt. % polysorbate-80 surfactant from the vessel is less than 170 ppb/day.

The total silicon content of the pH protective coating or layer and barrier coating or layer, upon dissolution into 0.1 N potassium hydroxide aqueous solution at 40°C from the vessel, is less than 66 ppm.

The calculated shelf life (total Si / Si dissolution rate) is more than 2 years.

The pH protective coating or layer shows an O-Parameter measured with attenuated total reflection (ATR) of less than 0.4, measured as: $O - Parameter = \frac{Intensity at 1253 cm - 1}{Maximum intensity in the range 1000 to 1100 cm - 1 .}$

The pH protective coating or layer shows an N-Parameter measured with attenuated total reflection (ATR) of less than 0.7, measured as: $N - Parameter = \frac{Intensity at 840 cm - 1}{Intensity at 799 cm - 1}$

The tie coating or layer is applied by PECVD of a precursor feed comprising octamethylcyclotetrasiloxane (OMCTS), tetramethyldisiloxane (TMDSO), or hexamethyldisiloxane (HMDSO).

The tie coating or layer is on average between 5 and 200 nm thick.

The tie coating or layer is at least coextensive with the barrier coating or layer.

The barrier coating or layer is between 10 and 200 nm thick

Also described is a multi-dose, fragrance, and/or cosmetic package having a lumen defined at least in part by a wall, the wall having an interior surface facing the lumen, an outer surface, and a coating set on the interior surface that comprises a barrier coating and at least one additional coating layer.

The problem of permeability may be addressed by adding a barrier coating or layer to a plastic fragrance and/or cosmetic package where it contacts fluid contents of the package. One such barrier layer is a very thin coating of SiOx, as defined below, applied by plasma enhanced chemical vapor deposition. But SiOx barrier layers deposited on a package by PECVD are etched off by aqueous contents of the package having pH-values greater than 4, particularly at higher pH values. This reduces the pre-opening shelf life of the package as its barrier efficacy is reduced.

Containers for cosmetic compositions also have a number of challenges that must be overcome as a result of the complexity of the cosmetic composition itself. Cosmetic compositions include a mascara composition, such as that of the type commonly used to enhance lenghthening and beautiful curvature on eyelashes, or a crème, such as a face or body crème or a hair crème. Such mascara compositions or crèmes may contain one or more waxes, a film-forming polymer, a silicone, a natural or synthetic latex or pseudolatex, and agents for suspending the latex and the silicone and/or a thickening agent, among other compositional materials or admixtures. The embodiments of the present invention provide a suitable solute block for the underlying plastic to avoid any detrimental effects or interactions with the compositions contained within the container. The embodiments of the present invention may also reduce the need for additives in the compositions, whether cosmetic or fragrance compositions, by providing an inert environment.

The coating set may comprise a barrier coating or layer and any one or more of a tie coating or layer and a pH protective coating or layer.

The tie coating or layer may comprise or consist of SiOₓC_{y}H_{z} or SiNₓC_{y}H_{z} in which x is from about 0.5 to about 2.4 as measured by X-ray photoelectron spectroscopy (XPS), y is from about 0.6 to about 3 as measured by XPS, and z is from about 2 to about 9 as measured by at least one of Rutherford backscattering spectrometry (RBS) or hydrogen forward scattering (HFS). The tie coating or layer has an outer surface facing the wall surface and an interior surface.

The barrier coating or layer may comprise or consist of SiOx, in which x is from about 1.5 to about 2.9 as measured by XPS. The barrier coating or layer is positioned between the interior surface of the tie coating or layer and the lumen.

The pH protective coating or layer may comprise or consist of SiOₓC_{y}H_{z}, in which x is from about 0.5 to about 2.4 as measured by XPS, y is from about 0.6 to about 3 as measured by XPS, and z is from about 2 to about 9 as measured by at least one of RBS or HFS. The pH protective coating or layer is positioned between the barrier coating or layer and the lumen.

The pH protective coating or layer and tie coating or layer together may be effective to keep the barrier coating or layer at least substantially undissolved as a result of attack by a fluid contained in the lumen having a pH greater than 5 for a period of at least six months.

Also described is the use of such a vessel for storing a multi-dose, fragrance (e.g. perfume), and/or cosmetic fluid having a pH greater than 5.

A process for making such a vessel is also described, comprising or consisting of the steps of: forming a tie coating or layer on the vessel interior wall; forming a barrier coating or layer over at least a portion of the tie coating or layer; and forming a pH protective coating or layer positioned between the barrier coating or layer and the lumen.

In any embodiment of the invention, the tie coating or layer optionally can be applied by plasma enhanced chemical vapor deposition (PECVD). In any embodiment of the invention, the barrier coating or layer optionally can be applied by PECVD. In any embodiment of the invention, the pH protective coating or layer optionally can be applied by PECVD.

In any embodiment of the invention, for the pH protective coating or layer, x optionally can be from about 1 to about 2 as measured by XPS, y optionally can be from about 0.6 to about 1.5 as measured by XPS, and z optionally can be from about 2 to about 5 as measured by RBS or HFS.

In any embodiment of the invention, the pH protective coating or layer may be applied by PECVD of a precursor feed comprising an organosilicon precursor. In any embodiment of the invention, the organosilicon precursor comprises or consists of hexamethyldisiloxane (HMDSO), trimethylsilane (TriMS), tetramethylsilane (TetraMS), tetramethyldisiloxane (TMDSO), octamethylcyclotetrasiloxane (OMCTS) or a combination of two or more of them.

In any embodiment of the invention, the precursor feed for the pH protective coating or layer may comprise or consist of:
- from 0.5 to 10 standard volumes of the organosilicon precursor;
- from 0.1 to 10 standard volumes of oxygen; and
- from 1 to 100 standard volumes of a carrier gas.

In any embodiment of the invention, the pH protective coating or layer optionally can be from about 10 to about 1000 nm thick.

In any embodiment of the invention, the pH protective coating or layer contacting the fluid composition optionally can be from about 10 to about 1000 nm thick after contact with a fluid contained in the lumen having a pH greater than 5 for a period of two years.

In any embodiment of the invention, the rate of erosion of the pH protective coating or layer, if directly contacted by a fluid contained in the lumen having a pH greater than 5, optionally can be less than 20% of the rate of erosion of the barrier coating or layer, if directly contacted by the same fluid under the same conditions.

In any embodiment of the invention, the vessel may have a pre-opening shelf life, while directly contacted by a fluid contained in the lumen having a pH greater than 5, of at least two years.

In any embodiment of the invention, the pre-opening shelf life optionally can be based on storage of the vessel containing the fluid at 20°C. In any embodiment of the invention, the pre-opening shelf life optionally can be based on storage of the vessel containing the fluid at 40°C.

In any embodiment of the invention, a fluid contained in the lumen having a pH greater than 5 optionally can remove the pH protective coating or layer at a rate of 1 nm or less of pH protective coating or layer thickness per 88 hours of contact with the fluid.

In any embodiment of the invention, an FTIR absorbance spectrum of the pH protective coating or layer may have a ratio greater than 0.75 between:
- the maximum amplitude of the Si-O-Si symmetrical stretch peak between about 1000 and 1040 cm-1, and
- the maximum amplitude of the Si-O-Si asymmetric stretch peak between about 1060 and about 1100 cm-1.

In any embodiment of the invention, the silicon dissolution rate by a 50 mM potassium phosphate buffer diluted in water, adjusted to pH 8 with concentrated nitric acid, and containing 0.2 wt. % polysorbate-80 surfactant, from the vessel optionally can be less than 170 ppb/day.

In any embodiment of the invention, the total silicon content of the pH protective coating or layer, barrier coating or layer, and tie coating or layer, as measured by dissolution of the pH protective coating or layer, barrier coating or layer, and tie coating or layer into 0.1 N potassium hydroxide aqueous solution at 40°C from the vessel, optionally can be less than 66 ppm.

In any embodiment of the invention, the pre-opening calculated shelf life optionally can be more than 2 years.

In any embodiment of the invention, after formation of a groove by focused ion beam through the pH protective coating or layer, the barrier coating or layer, the tie coating or layer, and into the lumen wall, and exposure of the groove with 1N aqueous potassium hydroxide (KOH) solution maintained at 40°C in the lumen for 6.5 hours, the barrier coating or layer optionally can be detectable by XPS and optionally can have atomic percentages of oxygen and silicon within 10 atomic percent of their values before treatment of the groove with the KOH solution.

In any embodiment of the invention, the pH protective coating or layer optionally can show an O-Parameter measured with attenuated total reflection (ATR) of less than 0.4, measured as: $O - Parameter = \frac{Intensity at 1253 cm - 1}{Maximum intensity in the range 1000 to 1100 cm - 1 .}$

In any embodiment of the invention, the pH protective coating or layer optionally can show an N-Parameter measured with attenuated total reflection (ATR) of less than 0.7, measured as: $N - Parameter = \frac{Intensity at 840 cm - 1}{Intensity at 799 cm - 1} .$

In any embodiment of the invention, the pH protective coating or layer optionally can be applied by PECVD at a power level per of more than 22,000 kJ/kg of mass of polymerizing gases in the PECVD reaction chamber. In any embodiment of the invention, the pH protective coating or layer optionally can be applied by PECVD at a power level per of from 1 to 200 W. In any embodiment of the invention, for formation of the pH protective coating or layer the ratio of the electrode power applied by PECVD to the plasma volume optionally can be from 5 W/ml to 75 W/ml.

In any embodiment of the invention, for the tie coating or layer, x optionally can be from about 1 to about 2 as measured by X-ray photoelectron spectroscopy (XPS), y optionally can be from about 0.6 to about 1.5 as measured by XPS, and z optionally can be from about 2 to about 5 as measured by Rutherford backscattering spectrometry (RBS) or hydrogen forward scattering (HFS).

In any embodiment of the invention, the tie coating or layer optionally can be applied by PECVD of a precursor feed comprising an organosilicon precursor. In any embodiment of the invention, the organosilicon precursor optionally can be tetramethylsilane (TetraMS), trimethylsilane (TriMS), hexamethyldisiloxane (HMDSO), octamethylcyclotetrasiloxane (OMCTS), tetramethyldisiloxane (TMDSO), or a combination of two or more of these.

In any embodiment of the invention, the precursor feed for the tie coating or layer optionally comprises or consists of:
- from 0.5 to 10 standard volumes of the organosilicon precursor;
- from 0.1 to 10 standard volumes of oxygen; and
- from 1 to 120 standard volumes of a carrier gas.

In any embodiment of the invention, the tie coating or layer optionally can be on average from about 5 to about 200 nm thick.

Any embodiment of the invention optionally can further comprise a lubricity coating or layer applied between the pH protective coating or layer and the lumen.

In any embodiment of the invention, the vessel optionally can contain a fragrance and/or cosmetic composition having a pH greater than 5 in the lumen, and the package may have a pre-opening shelf life of at least six months.

In any embodiment of the invention, the vessel may contain a liquid fragrance, a dry powder cosmetic product, or a moist cosmetic product.

In any embodiment of the invention, the composition contained in said vessels may be a mascara composition, such as that of the type commonly used to enhance lenghthening and beautiful curvature on eyelashes, or a crème, such as a face or body crème or a hair crème, or a fragrance / perfume, among other cosmetic or fragrance compositions. Such mascara compositions or crèmes may contain one or more waxes, a film-forming polymer, a silicone, a natural or synthetic latex or pseudolatex, and agents for suspending the latex and the silicone and/or a thickening agent, among other compositional materials or admixtures. Embodiments of the present invention provide a suitable solute block for the underlying plastic to avoid any detrimental effects or interactions with the compositions contained within the container. Embodiments of the present invention also reduce the need for additives in the compositions, whether cosmetic or fragrance compositions, by providing an inert environment.

Many additional and alternative aspects and embodiments of the invention are also contemplated, and are described in the specification and claims that follow.

### BRIEF DESCRIPTION OF THE DRAWINGS

FIG. 1 is a perspective view of a multi-dose eye drop bottle according to an embodiment of the invention.
FIG. 2 is a perspective view of a multi-dose eye drop bottle according to an embodiment of the invention.
FIG. 3 is a schematic sectional view of a multi-dose eye drop bottle according to an embodiment of the invention, including an enlarged detail view of a portion of the vessel wall and coating.
FIG. 4 is a schematic sectional view of a multi-dose eye drop bottle according to an embodiment of the invention, including an enlarged detail view of a portion of the vessel wall and coatings.
FIG. 5 is a perspective view of a nasal spray bottle according to an embodiment of the invention.
FIG. 6 is a perspective view of a nasal spray bottle according to an embodiment of the invention.
FIG. 7 is a schematic sectional view of a nasal spray bottle according to an embodiment of the invention, including an enlarged detail view of a portion of the vessel wall and coating.
FIG. 8 is a schematic sectional view of a nasal spray bottle according to an embodiment of the invention, including an enlarged detail view of a portion of the vessel wall and coatings.
FIG. 9 is a perspective view of a mascara bottle according to an embodiment of the invention.
FIG. 10 is a perspective view of a mascara bottle according to an embodiment of the invention.
FIG. 11 is a schematic sectional view of a mascara bottle according to an embodiment of the invention, including an enlarged detail view of a portion of the vessel wall and coating.
FIG. 12 is a schematic sectional view of a mascara bottle according to an embodiment of the invention, including an enlarged detail view of a portion of the vessel wall and coatings.
FIG. 13 is a perspective view of a small dose medicine bottle package according to an embodiment of the invention.
FIG. 14 is a perspective view of a small dose medicine bottle package according to an embodiment of the invention.
FIG. 15 is a schematic sectional view of a small dose medicine bottle package according to an embodiment of the invention, including enlarged detail views of a portion of the vessel wall and coating and a portion of the applicator wall and coating.
FIG. 16 is a schematic sectional view of a small dose medicine bottle package according to an embodiment of the invention, including enlarged detail views of a portion of the vessel wall and coatings and a portion of the applicator wall and coatings.
FIG. 17 is a perspective view of a pump bottle according to an embodiment of the invention.
FIG. 18 is a perspective view of a pump bottle according to an embodiment of the invention.
FIG. 19 is a schematic sectional view of a pump bottle according to an embodiment of the invention, including an enlarged detail view of a portion of the vessel wall and coating.
FIG. 20 is a schematic sectional view of a pump bottle according to an embodiment of the invention, including an enlarged detail view of a portion of the vessel wall and coatings.
FIG. 21 is a perspective view of a contact lens case according to an embodiment of the invention.
FIG. 22 is a schematic sectional view of a contact lens case according to an embodiment of the invention, including an enlarged detail view of a portion of the vessel wall and coating.
FIG. 23 is a schematic sectional view of a multi-dose inhaler according to an embodiment of the invention.
FIG. 24 is a schematic sectional view of a vessel according to any embodiment of the invention.
FIG. 25 is an enlarged detail view of a portion of the vessel wall and coatings of FIG. 1.
FIG. 26 is a plot of silicon dissolution versus exposure time at pH 6 for a glass container versus a plastic container having a SiOₓ barrier layer coated in the inside wall.
FIG. 27 is a plot of silicon dissolution versus exposure time at pH 7 for a glass container versus a plastic container having a SiOₓ barrier layer coated in the inside wall.
FIG. 28 is a plot of silicon dissolution versus exposure time at pH 8 for a glass container versus a plastic container having a SiOₓ barrier layer coated in the inside wall.
FIG. 29 is a plot of the SiOₓ coating thickness necessary initially to leave a 30 nm residual coating thickness when stored with solutions at different nominal pH values from 3 to 9.
FIG. 30 shows the silicon dissolution rates at pH 8 and 40°C of various PECVD coatings.
FIG. 31 is a plot of the ratio of Si-O-Si symmetric/asymmetric stretching mode versus energy input per unit mass (W/FM or KJ/kg) of a PECVD coating using as the reactive precursor gases OMCTS and oxygen.
FIG. 32 is a plot of silicon shelf life (days) versus energy input per unit mass (W/FM or KJ/kg) of a PECVD coating using as the reactive precursor gases OMCTS and oxygen.
FIG. 33 is a Fourier Transform Infrared Spectrophotometer (FTIR) absorbance spectrum of a PECVD coating.
FIG. 34 is a Fourier Transform Infrared Spectrophotometer (FTIR) absorbance spectrum of a PECVD coating.
FIG. 35 is a Fourier Transform Infrared Spectrophotometer (FTIR) absorbance spectrum of a PECVD coating.
FIG. 36 is a Fourier Transform Infrared Spectrophotometer (FTIR) absorbance spectrum of a PECVD coating.
FIG. 37 is a Fourier Transform Infrared Spectrophotometer (FTIR) absorbance spectrum of a PECVD coating, originally presented as FIG. 5 of U.S. Pat. No.8,067,070, annotated to show the calculation of the O-Parameter referred to in that patent.
FIG. 38 is a schematic view of a syringe with a trilayer coating according to FIGS. 1, 2, and 3, showing a cylindrical region and specific points where data was taken.
FIG. 39 is a Trimetric map of the overall trilayer coating thickness versus position in the cylindrical region of a syringe illustrated by FIGS. 18, 1, 2, and 3.
FIG. 40 is a photomicrograhic sectional view showing the substrate and coatings of the trilayer coating at position 2 shown in FIG. 18.
FIG. 41 is another Trimetric map of the overall trilayer coating thickness versus position in the cylindrical region of a syringe illustrated by FIGS. 18, 1, 2, and 3.
FIG. 42 is a plot of coating thickness, representing the same coating as FIG. 21, at Positions 1, 2, 3, and 4 shown in FIG. 18.
FIG. 43 is a schematic illustration of a syringe, showing points on its surface where measurements were made in a working example.
FIG. 44 is a photograph showing the benefit of the present trilayer coating in preventing pinholes after attack by an alkaline reagent, as discussed in the working examples.
FIG. 44A is an enlarged detail view of the indicated portion of FIG. 24.

In the context of the present invention, the following definitions and abbreviations are used:
RF is radio frequency.

The term "at least" in the context of the present invention means "equal or more" than the integer following the term. The word "comprising" does not exclude other elements or steps, and the indefinite article "a" or "an" does not exclude a plurality unless indicated otherwise. Whenever a parameter range is indicated, it is intended to disclose the parameter values given as limits of the range and all values of the parameter falling within said range.

"First" and "second" or similar references to, for example, layers, processing stations or processing devices refer to the minimum number of layers, processing stations or devices that are present, but do not necessarily represent the order or total number of layers, processing stations and devices or require additional layers, processing stations and devices beyond the stated number. These terms do not limit the number of processing stations or the particular processing carried out at the respective stations. For example, a "first" layer in the context of this specification can be either the only layer or any one of plural layers, without limitation. In other words, recitation of a "first" layer allows but does not require an embodiment that also has a second or further layer.

For purposes of the present invention, an "organosilicon precursor" is a compound having at least one of the linkages: 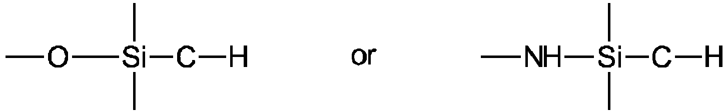 which is a tetravalent silicon atom connected to an oxygen or nitrogen atom and an organic carbon atom (an organic carbon atom being a carbon atom bonded to at least one hydrogen atom). A volatile organosilicon precursor, defined as such a precursor that can be supplied as a vapor in a PECVD apparatus, is an optional organosilicon precursor. Optionally, the organosilicon precursor is selected from the group consisting of a linear siloxane, a monocyclic siloxane, a polycyclic siloxane, a polysilsesquioxane, an alkyl trimethoxysilane, a linear silazane, a monocyclic silazane, a polycyclic silazane, a polysilsesquiazane, and a combination of any two or more of these precursors.

The feed amounts of PECVD precursors, gaseous reactant or process gases, and carrier gas are sometimes expressed in "standard volumes" in the specification and claims. The standard volume of a charge or other fixed amount of gas is the volume the fixed amount of the gas would occupy at a standard temperature and pressure (without regard to the actual temperature and pressure of delivery). Standard volumes can be measured using different units of volume, and still be within the scope of the present disclosure and claims. For example, the same fixed amount of gas could be expressed as the number of standard cubic centimeters, the number of standard cubic meters, or the number of standard cubic feet. Standard volumes can also be defined using different standard temperatures and pressures, and still be within the scope of the present disclosure and claims. For example, the standard temperature might be 0°C and the standard pressure might be 760 Torr (as is conventional), or the standard temperature might be 20°C and the standard pressure might be 1 Torr. But whatever standard is used in a given case, when comparing relative amounts of two or more different gases without specifying particular parameters, the same units of volume, standard temperature, and standard pressure are to be used relative to each gas, unless otherwise indicated.

The corresponding feed rates of PECVD precursors, gaseous reactant or process gases, and carrier gas are expressed in standard volumes per unit of time in the specification. For example, in the working examples the flow rates are expressed as standard cubic centimeters per minute, abbreviated as sccm. As with the other parameters, other units of time can be used, such as seconds or hours, but consistent parameters are to be used when comparing the flow rates of two or more gases, unless otherwise indicated.

A "vessel" in the context of the present invention can be any type of vessel with at least one opening and a wall defining an inner or interior surface. The substrate can be the wall of a vessel having a lumen. The substrate surface can be part or all of the inner or interior surface of a vessel having at least one opening and an inner or interior surface. A vessel can be of any shape, with a vessel having a substantially cylindrical wall adjacent to at least one of its open ends being preferred.

In some embodiments, the package and vessel may be configured to store multiple doses of a fluid product. For instance, the vessel may be a multi-dose eye dropper bottle or a nasal spray bottle, configured for multiple applications/uses over a period of time. Alternatively, the vessel may be a cosmetic container, such as a mascara bottle or tube or a liquid/gel eye liner bottle or tube or a lip gloss bottle or tube. Alternatively, the vessel may be a container configured to store a cream that is applied to the skin, e.g. a can or canister of a topical cream or ointment. Alternatively, the vessel may be a container configured to store a fragrance, e.g. a perfume.

In some embodiments, the package or vessel may comprise an applicator for the fluid product.

An "applicator" in the context of the present invention can be any type of device that is used to apply the fluid product to an intended location and can include droppers, spray nozzles, brushes, and the like. In some embodiments, for instance, the vessel may comprise a dropper tip or a spray tip. The dropper tip or spray tip is typically inserted into the opening to the lumen, thereby closing it off such that the fluid exits the lumen only through the dropper tip or spray tip. In other embodiments, the applicator may be removed from the vessel during use. For instance, the applicator may include a makeup brush such as an eyelash brush, an eye liner brush or pad, or a lip gloss brush. Or the applicator may comprise a dropper cap that is unscrewed from the vessel for use and then re-attached to the vessel after use.

In other embodiments, rather than being part of the package of the present disclosure, the applicator may come from an external source. For instance, a topical cream may be applied by hand, with a user's hand thus serving as the applicator. Or a food product may be applied by a common kitchen utensil, such as a spoon, fork, or knife.

The terms "pre-opening shelf life" and "post-opening shelf life" are used in the present disclosure to refer to two different things. The pre-opening shelf life of a package is the shelf life of the package prior to a first opening of the package, i.e. during which the product within the lumen of the vessel remains sealed in the lumen by one or more closures and/or seals. The post-opening shelf life of a package is the shelf life of the package after the package has been opened (and typically the product dispensed for the first time) and subjected to potential microbial/bacterial contamination. The pre-opening shelf life of a product may be extended by the application of one or more coatings or layers that reduce gas ingress and/or egress through the vessel walls and/or that provide a solute block to avoid detrimental effects or interactions between the vessel wall and the composition contained within the container. The post-opening shelf life of a product, on the other hand, may be extended by the application of one or more anti-microbial coatings.

The term "at least" in the context of the present invention means "equal or more" than the integer following the term. Thus, a vessel in the context of the present invention has one or more openings. If the vessel has two openings, they can be of same or different size. If there is more than one opening, one opening can be used for the gas inlet for a PECVD coating method according to the present invention, while the other openings are either capped or open.

A "hydrophobic layer" in the context of the present invention means that the coating or layer lowers the wetting tension of a surface coated with the coating or layer, compared to the corresponding uncoated surface. Hydrophobicity is thus a function of both the uncoated substrate and the coating or layer. The same applies with appropriate alterations for other contexts wherein the term "hydrophobic" is used. The term "hydrophilic" means the opposite, i.e. that the wetting tension is increased compared to reference sample. The present hydrophobic layers are primarily defined by their hydrophobicity and the process conditions providing hydrophobicity. A coating or layer or treatment is defined as "hydrophobic" if it lowers the wetting tension of a surface, compared to the corresponding uncoated or untreated surface. Hydrophobicity is thus a function of both the untreated substrate and the treatment.

"Wetting tension" is a specific measure for the hydrophobicity or hydrophilicity of a surface. An optional wetting tension measurement method in the context of the present invention is ASTM D 2578 or a modification of the method described in ASTM D 2578. This method uses standard wetting tension solutions (called dyne solutions) to determine the solution that comes nearest to wetting a plastic film surface for exactly two seconds. This is the film's wetting tension. The procedure utilized is varied herein from ASTM D 2578 in that the substrates are not flat plastic films, but are tubes made according to the Protocol for Forming PET Tube and (except for controls) coated according to the Protocol for coating Tube Interior with Hydrophobic Coating or Layer (see Example 9 of EP2251671 A2).

These values of w, x, y, and z are applicable to the empirical composition Si_{w}OₓC_{y}H_{z} throughout this specification. The values of w, x, y, and z used throughout this specification should be understood as ratios or an empirical formula (for example for a coating or layer), rather than as a limit on the number or type of atoms in a molecule. For example, octamethylcyclotetrasiloxane, which has the molecular composition Si₄O₄C₈H₂₄, can be described by the following empirical formula, arrived at by dividing each of w, x, y, and z in the molecular formula by 4, the largest common factor: Si₁O₁C₂H₆. The values of w, x, y, and z are also not limited to integers. For example, (acyclic) octamethyltrisiloxane, molecular composition Si₃O₂C₈H₂₄,is reducible to Si₁O_{0.67}C_{2.67}H₈. Also, although SiOₓC_{y}H_{z} is described as equivalent to SiOₓC_{y}, it is not necessary to show the presence of hydrogen in any proportion to show the presence of SiOₓC_{y}.

The atomic ratio can be determined by XPS. Taking into account the H atoms, which are not measured by XPS, the coating or layer may thus in one aspect have the formula Si_{w}OₓC_{y}H_{z} (or its equivalent SiOₓC_{y}), for example where w is 1, x is from about 0.5 to about 2.4, y is from about 0.6 to about 3, and z is from about 2 to about 9. Typically, such coating or layer would hence contain 36% to 41% carbon normalized to 100% carbon plus oxygen plus silicon.

The word "comprising" does not exclude other elements or steps.

The indefinite article "a" or "an" does not exclude a plurality.

### DETAILED DESCRIPTION

The present invention will now be described more fully, with reference to the accompanying drawings, in which several embodiments are shown. This invention can, however, be embodied in many different forms and should not be construed as limited to the embodiments set forth here. Rather, these embodiments are examples of the invention, which has the full scope indicated by the language of the claims. Like numbers refer to like or corresponding elements throughout. The following disclosure relates to all embodiments unless specifically limited to a certain embodiment.

### Multi-Use Packages

A variety of products, and in particular fluid products, are sold in multi-use or repeateduse packages. Such products include drug or medical products, including for example liquid formulations configured for ocular administration and contained in multi-use eye drop bottles, liquid formulations configured for nasal administration and contained in nasal spray bottles, liquid formulations configured for a metered-dose inhaler and contained in a metered-dose inhaler canister, and drug formulations contained in bottles having small dosage applicators, such as medicines for infants, toddlers, or pets. Such products also include topical medications, skin care products, or cosmetic products, such as creams/crèmes (which are considered fluids in the context of the present disclosure) and/or ointments. Such products also include cosmetics such as makeups, liquid/gel eye liners, lip glosses, and mascaras, which may contain for instance one or more pigments, one or more oils, and one or more waxes. Such products may also include contact lens solutions, which are packaged in multi-use bottles, and reusable contact lens containers/cases into which contact lens solutions are introduced. Such products may also include food products, including for example mayonnaise, mustard, and the like, as well as solid food products that are packed in a liquid such as pickles, olives, and the like.

Such packages typically include a vessel having one or more walls that enclose and at least partially define a lumen, an opening to the lumen, and a closure, such as a cap, sealing the opening. A fluid product is stored in the lumen. Some of these packages further include an applicator for applying the fluid product. While the fluid product within the lumen may be aseptic or sterile when the package is sold, once the package is opened and the product used, contaminants may enter the fluid reservoir of the vessel, such as through the applicator.

For instance, multi-dose eye drop bottles typically include dropper tip that seals and communicates with the lumen. During use, however, the dropper tip may become contaminated with bacteria, e.g. if it is held too close to the eye. Nasal spray bottles typically include a spray cap, e.g. that may be operated by an integrated pump. During use, however, the spray cap is often inserted into the nose, whereby it can pick up bacteria that may be introduced into the fluid reservoir of the vessel. Small-dose medicines, such as those for infants, toddlers, or pets may have either a removable dropper cap or an independent dropper or plunger-operated applicator. Although these applicators are typically inserted into the mouth of the recipient, they may not be thoroughly washed between uses. Accordingly, bacteria from the mouth may be introduced into the fluid reservoir during a subsequent use. Metered-dose inhalers include an actuator having a mouthpiece applicator that is inserted into the mouth of a user, whereby it can come into contact with bacteria, which may subsequently be introduced into the fluid reservoir of the canister. Cosmetics such as makeups or mascaras may be provided in bottles, tubes, canisters, or the like, which may also contain a makeup applicator. In the case of mascaras, for instance, the package typically comprises a cap that includes an eyelash brush which is inserted into the lumen of the vessel to close the vessel opening. Similarly, liquid/gel eyeliners and lip glosses are typically provided in bottle or tube with a cap that includes an appropriately configured applicator brush which is inserted into the lumen of the vessel to close the vessel opening. Should the applicator brush collect bacteria, that bacteria will almost immediately be introduced into the fluid reservoir.

Although the packages for skin creams and ointments may not have an applicator, they are typically applied by hand. Accordingly, it is quite easy for bacteria or other contaminants to become introduced into the fluid reservoir. Similarly, though most food packages do not include a specific applicator, the contents may be removed from the container either by a common kitchen utensil, such as a knife, spoon, or fork, or by a user's hands (in the case of solid food products that are stored in a liquid). It is very easy for contaminants to enter the fluid reservoirs of food products in this manner.

The introduction of bacteria into the lumen of the vessel may often lead to spoilage of the product contained therein, which may prevent a user from being able to use the entire contents of the package.

Embodiments of the present disclosure are directed to methods of inhibiting the growth of microbes, including bacteria and fungi, in a fluid contained within a multi-use package, such as any of the products and packages described above. Embodiments of the present disclosure are also directed to methods of inactivating or killing microbes, including bacteria and fungi, in a fluid contained within a multi-use package, such as any of the products and packages described above. Related to either of the above mechanisms (or both), embodiments of the present disclosure are also directed to methods of increasing the post-opening shelf life of a multi-use package, such as any of the products and packages described above. In some embodiments, for example, the post-opening shelf life of any of the above-described products and packages may be increased by at least one week, optionally at least two weeks, optionally at least one month, optionally at least two months, optionally at least three months, optionally at least four months, optionally at least five months, optionally at least six months, optionally at least nine months, optionally at least one year. By inhibiting and/or inactivating/killing microbes that enter the vessel lumen, embodiments of the present disclosure are also directed to methods of reducing the amount of preservatives and/or excipients used in a product contained in a multi-use package.

### Anti-Microbial Coatings

The anti-microbial coatings of the present disclosure may inhibit the growth of microbes, such as bacteria. In other words, the anti-microbial coatings may reduce the amount of bacteria present in the fluid product within the lumen at a defined period of time after the introduction of bacteria relative to the same vessel without the anti-microbial coating. The period of time selected for this determination may vary depending on how quickly the bacteria grows and may be on the order of hours, days, weeks, or months. In some embodiments, the period of time selected for a comparative analysis of the amount of bacteria present within the lumen may be, for instance, between one and seven days (e.g. one day after introduction, two days after introduction, three days after introduction, four days after introduction, five days after introduction, six days after introduction, or seven days after introduction) or between one and four weeks (e.g. one week after introduction, two weeks after introduction, three weeks after introduction, or four weeks after introduction).

In some embodiments, the comparative testing may be performed after a period of use or a replicated period of use that corresponds with a sample expiration date of the product, e.g. 3 months, 6 months, 12 months, 18 months, 24 months, or 36 months. An example of a specific testing method may be found, for example, in Bashir et al., "Microbiological study of used cosmetic products: highlighting possible impact on consumer health," J. Appl. Microbiol., vol. 128(2), pp. 598-605 (2020), the entirety of which is incorporated by reference herein.

Specific bacteria that may be reduced, and desirably eliminated or substantially eliminated, include *Salmonella, Escherichia coli (E.coli), C.freundii, Pseudomonas aeruginosa (P.aeruginosa), Staphylococcus aureus (S.aureus), Staphylococcus epidermis, Escherichia hermannii, Bacillus cereus, Enterobacter* species, and *Candida* species. In some embodiments, pathogenic bacteria such as *E*. *Coli, C.freundii, P.aeruginosa,* and/or *S.aureus* may be demonstrably reduced by anti-microbial coatings of the present disclosure. In some embodiments, the presence of *S.aureus* and *P.aeruginosa,* the two most common pathogenic contaminants of cosmetic products such as mascara, eyeliner, and lip gloss, may be demonstrably reduced by anti-microbial coatings of the present disclosure.

The anti-microbial coatings of the present disclosure may be effective to inactivate or kill microbes, such as bacteria, that are introduced into the lumen. For example, silver ions have been shown in other applications to react with certain enzymes to inactivate them, leading to cell death. Embodiments of the anti-microbial coatings of the present disclosure may therefore be used to destroy microbial contaminants that enter the fluid reservoir within the lumen, such as through contamination of an applicator, thereby reducing the amount of bacteria (as opposed to simply inhibiting its growth). As such, embodiments of the present disclosure may be effective to re-establish aseptic or sterile conditions within a fluid reservoir that has been contaminated.

Regardless of the mechanism, anti-microbial coatings of the present disclosure may be effective to to increase the shelf-life of a multi-dose package after first use of the product. Because of the risk of contamination once opened, and particularly the risk of contamination caused by use of the product (e.g. through an applicator), many products have limited shelf-lives after opening. For instance, it is often recommended that a multi-dose eye drop bottle be used for no longer than, for example, four weeks after opening. In other words, many multi-dose eye drop bottles have a shelf-life after first use of about four weeks. By inhibiting the growth of and/or inactivating microbial contaminants, anti-microbial coatings of the present disclosure may extend that post-opening (i.e., post-first use) shelf-life for a commercially significant amount of time. For instance, embodiments of the anti-microbial coatings of the present disclosure may increase the shelf-life of the package after first use by at least one week, optionally at least two weeks, optionally at least one month, optionally at least two months, optionally at least three months, optionally at least four months, optionally at least five months, optionally at least six months, optionally at least nine months, optionally at least one year.

In some embodiments, the packages of the present disclosure may enable a reduction in the amount, or elimination, of anti-bacterial preservatives in the fluid formulation. Again, using eye drops as an example, the inclusion of excipients such as preservatives in an ophthalmic formulation is generally undesirable. Accordingly, the ability to reduce or eliminate those excipients would result in an improved ophthalmic fluid.

The anti-microbial coatings of the present disclosure may comprise any of a variety of metal oxides, including in particular zinc oxide, titanium dioxide, and silver oxide. The antimicrobial coatings may be applied by any of variety of processes, including for example sputtering, evaporation, or sintering. In some preferred embodiments, the anti-microbial coatings may be applied by plasma enhanced chemical vapor deposition (PECVD) or atomic layer deposition (ALD), including for instance plasma-enhanced atomic layer deposition (PEALD).

Given that the vessels and packages of the present disclosure are manufactured in high quantites and, being disposable command relatively low prices, it is desirable that the antimicrobial coatings of the present disclosure be thin, e.g. on the nanometer scale. It is also desirable that the anti-microbial coating be applied consistently across at least a portion of the interior surface of the vessel that contacts the fluid, and preferably across the entire interior surface of the vessel. Finally, it is desirable that the anti-microbial coatings can be applied to the wall of the vessel without damage to the vessel itself, e.g. deformation or melting of a thermoplastic vessel wall. The application of an anti-microbial coating by PECVD or ALD (including PEALD) in accordance with the present disclosure provide for a thin and consistent coating across the desired surface and can be applied without damage to the thermoplastic vessel wall.

In some embodiments, for example, the anti-microbial coating comprises zinc oxide (ZnO) applied by PECVD from a feed gas comprising zinc acetate, diethyl zinc, or a combination thereof, and an oxidant. Alternatively, the anti-microbial coating may comprise zinc oxide (ZnO) applied by ALD or PEALD using feed gases comprising zinc acetate, diethyl zinc, or a combination thereof, and an oxidant.

In some embodiments, for example, the anti-microbial coating comprises titanium dioxide (TiO2) applied by PECVD from a feed gas comprising titanium tetra chloride and an oxidant. Alternatively, the anti-microbial coating may comprise titanium dioxide (TiO2) applied by ALD or PEALD using feed gases comprising titanium tetra chloride, titanium isopropoxide, or a combination thereof, and an oxidant.

In some embodiments, for example, the anti-microbial coating comprises silver oxide (Ag2O) applied by PECVD from a feed gas comprising an organosilver compound and an oxidant, optionally wherein the organosilver compound has the composition: Ag(Hfac)(PR₃), in which Hfac is 1,1,1,5,5,5-hexafluoroacetylacetonate, P is phosphine, and R is methyl, ethyl, or a combination thereof. Alternatively, the anti-microbial coating may comprise silver oxide (Ag2O) applied by PECVD using feed gases comprising an organosilver compound and an oxidant, optionally wherein the organosilver compound has the composition: Ag(Hfac)(PR₃), in which Hfac is 1,1,1,5,5,5-hexafluoroacetylacetonate, P is phosphine, and R is methyl, ethyl, or a combination thereof.

In any of these embodiments, the oxidant may be selected from O₂, O₃, H₂O, H₂O₂, N₂O, NO₂, air, or a combination thereof.

In some embodiments, the anti-microbial coating may have a thickness between about 1 nm and about 1000 nm, optionally between about 2 nm and about 1000 nm, optionally between about 5 nm and about 1000 nm, optionally between about 10 nm and 1000 nm, optionally between about 1 nm and about 500 nm, optionally between 2 nm and about 500 nm, optionally between about 5 nm and about 500 nm, optionally between about 10 nm and 500 nm, optionally between about 1 nm and about 250 nm, optionally between about 2 nm and about 250 nm, optionally between about 5 nm and about 250 nm, optionally between about 10 nm and 250 nm, optionally between about 1 nm and about 100 nm, optionally between about 2 nm and about 100 nm, optionally between about 5 nm and about 100 nm, optionally between about 10 nm and 100 nm, optionally between about 1 nm and about 50 nm, optionally between about 2 nm and about 50 nm, optionally between about 5 nm and about 50 nm, optionally between about 10 nm and about 50 nm.

In some embodiments, the anti-microbial coating may be applied as a relatively consistent layer across the interior surface of the vessel wall.

### Examplary Embodiments

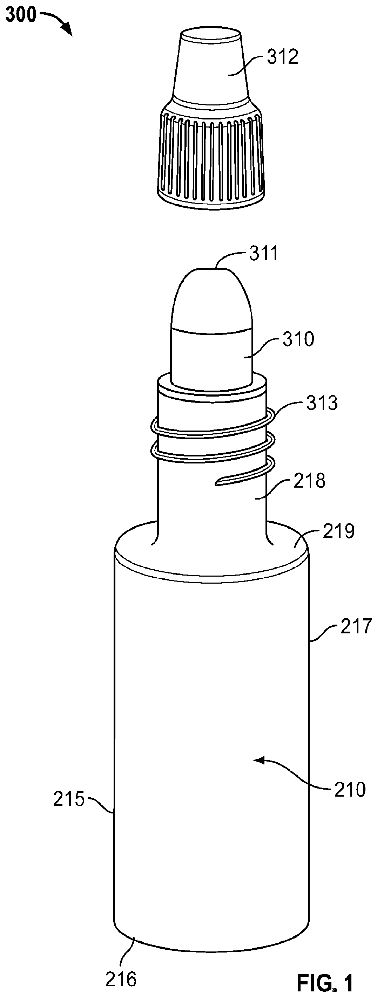

Examples of a multi-use package according to an embodiment of the present disclosure, and in particular embodiments of a multi-dose eye drop bottle 300, are shown in Figures 1-4. The multi-dose eye drop bottle 300 comprises a vessel 210 having a wall 214, and more particularly one or more sidewalls 215 and a bottom wall 216, the wall (e.g. the one or more sidewalls and the bottom wall together) defining and at least partially enclosing a lumen 212. The sidewall 215 may comprise a main body portion 217 and a neck portion 218 having a reduced diameter relative to the main body portion, with the main body portion and the neck portion being connected by a transition region 219. Opposite the bottom wall 216 is an opening 220 through which a fluid stored within the lumen 212 may be dispensed from the vessel 210.

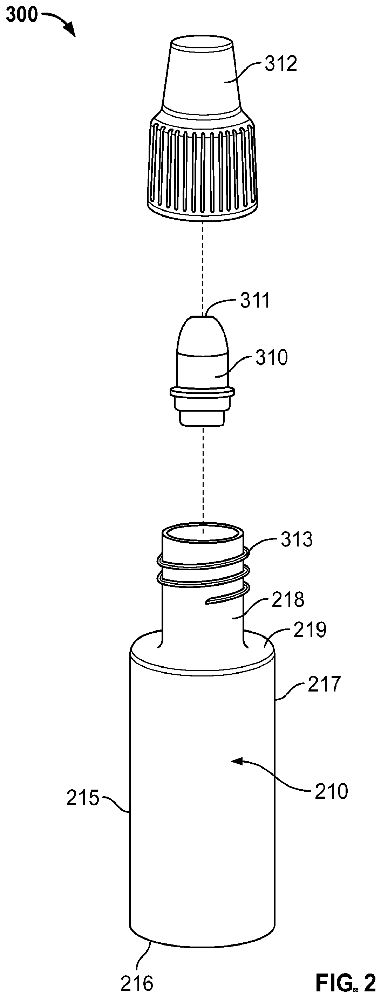

In the multi-dose eye drop bottle 300 shown in Figure 1, the vessel comprises an integral dropper tip 310 which compriess opening 311, with the opening being sized and configured to expel the fluid in small volume defined doses, e.g. droplets. In the multi-dose eye drop bottle 300 shown in Figure 2, on the other hand, the vessel 210 comprises a relatively large opening that is sealed by the insertion of a dropper tip element 310. The dropper tip element 310 comprises an opening 311 that is sized and configured to expel the fluid from the lumen of the vessel in small volume defined doses, e.g. droplets. Typically each droplet may be less than 0.3 mL, optionally less than 0.2 mL, optionally less than 0.1 mL. In some embodiments, for instance, each droplet may be abou 0.05 mL or less.

The multi-dose eye drop bottle 300 may also comprise a cap 312, the cap being configured to seal the opening 311 in between uses (i.e., when the product is not being dispensed). The cap 312 may be secured to the vessel 210 in any of a variety of manners. In the illustrated embodiment, for example, the exterior surface of the neck portion 218 may comprise a threaded portion 313 configured to mate with a threaded portion on the interior surface of a cap 312 in order to secure the cap to the neck portion of the vessel 210.

The multi-dose eye drop bottle 300 further comprises a fluid 350, for example an ophthalmic medical fluid, within the lumen 312. In some embodiments, the ophthalmic medical fluid 350 may be a drug-containing solution. In some embodiments, for example, the ophthalmic medical fluid 350 may comprise any one or more of the following: alcaftadine ophthalmic, atropine ophthalmic, azelastine ophthalmic, bepotastine ophthalmic, betaxolol ophthalmic, bimatoprost ophthalmic, brimonidine and timolol ophthalmic, brimonidine ophthalmic, brinzolamide and brimonidine ophthalmic, brinzolamide ophthalmic, bromfenac ophthalmic, carteolol ophthalmic, cenegermin ophthalmic, cetirizine ophthalmic, chloramphenicol ophthalmic, cromolyn ophthalmic, cyclopentolate and phenylephrine ophthalmic, cyclopentolate ophthalmic, cyclosporine ophthalmic, cysteamine ophthalmic, dexamethasone ophthalmic, diclofenac ophthalmic, difluprednate ophthalmic, dipivefrin ophthalmic, dorzolamide and timolol ophthalmic, dorzolamide ophthalmic, echothiophate iodide ophthalmic, emedastine ophthalmic, epinastine ophthalmic, fluocinolone ophthalmic, fluorometholone ophthalmic, flurbiprofen ophthalmic, ganciclovir ophthalmic, homatropine ophthalmic, hydrocortisone ophthalmic, hydroxyamphetamine and tropicamide Ophthalmic, ketorolac ophthalmic, ketotifen ophthalmic, latanoprost and netarsudil ophthalmic, latanoprost ophthalmic, latanoprostene bunod ophthalmic, levobunolol ophthalmic, levocabastine ophthalmic, lidocaine ophthalmic, lifitegrast ophthalmic, lodoxamide ophthalmic, loteprednol ophthalmic, metipranolol ophthalmic, naphazoline and antazoline ophthalmic, naphazoline and pheniramine ophthalmic, naphazoline and zinc ophthalmic, naphazoline ophthalmic, nedocromil ophthalmic, nepafenac ophthalmic, netarsudil ophthalmic, ocular lubricant, olopatadine ophthalmic, oxymetazoline ophthalmic, pemirolast ophthalmic, phenylephrine ophthalmic, physostigmine ophthalmic, pilocarpine ophthalmic, povidone and tetrahydrozoline ophthalmic, povidone-iodine ophthalmic, prednisolone ophthalmic, proparacaine ophthalmic, rimexolone ophthalmic, scopolamine ophthalmic, sodium chloride, hypertonic ophthalmic, tafluprost ophthalmic, tetracaine ophthalmic, tetrahydrozoline and zinc ophthalmic, tetrahydrozoline ophthalmic, timolol ophthalmic, travoprost ophthalmic, triamcinolone ophthalmic, trifluridine ophthalmic, tropicamide ophthalmic, unoprostone ophthalmic, vidarabine ophthalmic, or a combination thereof.

In some embodiments, the ophthalmic medical fluid 350 may contain one or more humectants (substances that help retain moisture), one or more lubricants, one or more electrolytes, such as potassium, or a combination thererof. In some embodiments, the ophthalmic medical fluid 350 may comprise one or more decongestants, one or more antihistamines, one or more mast cell stabilizers, or any combination thereof. In some embodiments, the ophthalmic medical fluid 350 may comprise one or more steroids. In some embodiments, the ophthalmic medical fluid 350 may have a viscosity similar to that of water while in other embodiments, the ophthalmic medical fluid may be a more viscous gel or ointment.

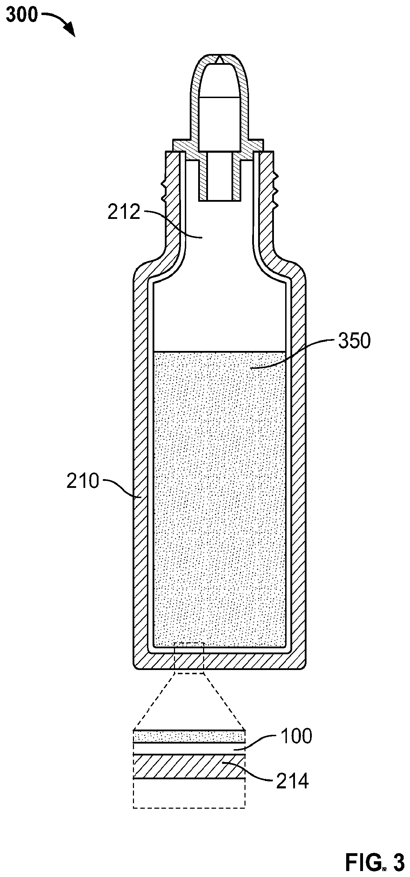

As shown in Figure 3, embodiments of the multi-dose eye drop bottle 300 may comprise an anti-microbial coating 100 on at least a portion of the interior surface of the wall 214 (e.g. the interior surfaces of the sidewall 215 and the bottom wall 216), i.e. the surfaces that are in contact with the fluid 350 stored within the lumen 212. Note that Figure 3 is not intended to be drawn to scale and that the anti-microbial coating 100 may be applied as a very thin coating relative to the thickness of the vessel wall 214.

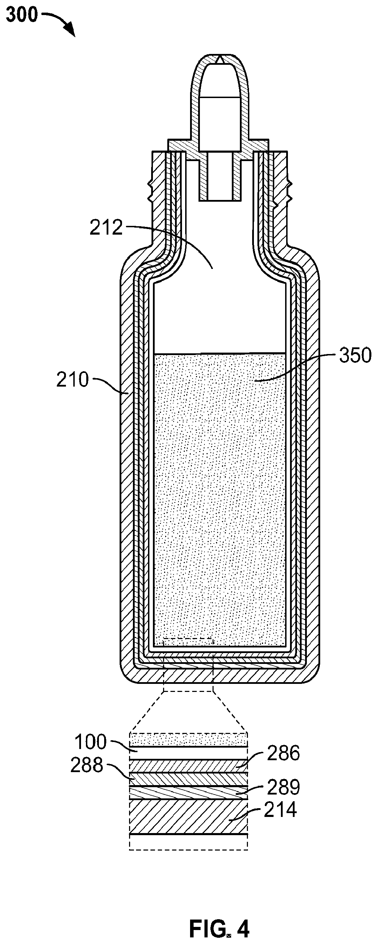

As shown in Figure 4, embodiments of the multi-dose eye drop bottle 300 may comprise a coating set 285 that includes a barrier coating or layer 288 and optionally one or more of a tie coating or layer 289 and a pH protective coating or layer 286. Like the anti-microbial coating 100 described above, this coating set 285 may be applied to at least a portion of the interior surface of the vessel wall 214, e.g. the interior surfaces of the sidewalls 215 and the bottom wall 216 of the vessel. This coating set 285 may be provided in addition to the antimicrobial coating 100, e.g. as illustrated in Figure 4, or alone, i.e. independent of an anti-microbial coating (not illustrated). Generally, when applied in combination with an anti-microbial coating 100, the anti-microbial coating is applied as the innermost layer, i.e. the layer that is in contact with the fluid 350 stored within the lumen 212. Note that Figure 4 is not intended to be drawn to scale and that the various coatings may be applied as very thin coatings relative to the thickness of the vessel wall 214.

In some embodiments of the multi-dose eye drop bottle 300, the cap 312 may initially seal the vessel 210 in a manner that prevents moisture and/or atmospheric gas (e.g. oxygen) and/or bacteria from entering into the lumen 212 (for example through the incorporation of one or more gaskets which may be compressed between the body of the cap and the vessel). That initial seal is then broken by the end user upon the first opening of the multi-dose eye drop bottle 300, which typically corresponds with the first use of (i.e., dispensing of product from) the bottle. In other embodiments, the multi-dose eye drop bottle 300 may comprise a seal, e.g. a film, foil, or laminate, which extends over the opening of the vessel and which may typically be sealed to an upper surface or top flange of the neck portion 218 so as to prevent moisture and/or atmospheric gas (e.g. oxygen) and/or bacteria from entering into the lumen 212. That seal is removed by the end user upon the first opening of the multi-dose eye drop bottle 300, which typically corresponds with the first use of (i.e., dispensing of product from) the bottle. In some embodiments, the multi-dose eye drop bottle 300 may comprise both initial seals. Regardless, however, where the multi-dose eye drop bottle 300 is in its initial sealed state, moisture and/or atmospheric gas (e.g. oxygen) may still enter into the lumen 212 of the vessel through the vessel walls 215, 216, which can result in deterioration of the fluid 350 contained within the lumen before the package is ever opened by the end user. Embodiments of the present invention therefore may comprise an oxygen barrier coating 288 that reduces the ingress of oxygen into the lumen 212 compared to a vessel without the oxygen barrier coating. The resulting increased oxygen barrier properties may serve to increase the pre-opening shelf life of the package.

In use, the multi-dose eye drop bottle 300 is typically inverted and a particular number of droplets of the ophthalmic medical fluid 350 is/are dispensed through the opening 311 of the dropper tip 310 into the user's eye. In doing so, the multi-dose eye drop bottle 300 is typically placed in close proximity to the eye and may come into contact with the user's eye, eyelid, eyelashes, etc. As a result, the dropper tip 310 may come into contact with bacteria, which can then enter into the lumen 212 of the vessel 210 in which the remainder of the ophthalmic medical fluid is stored for future use, thereby contaminating the ophthalmic medical fluid 350. Embodiments of the present invention comprise an anti-microbial coating 100 that is effective to inhibit the growth of microbes such as bacteria in the ophthalmic medical fluid 350 contained within the lumen 212 of the vessel 210 (e.g. as compared to a vessel without the anti-microbial coating) and/or to inactivate or kill bacteria introduced into the lumen of the multi-dose eye drop bottle 300, and/or to increase the shelf-life of the multi-dose eye drop bottle package after first use.

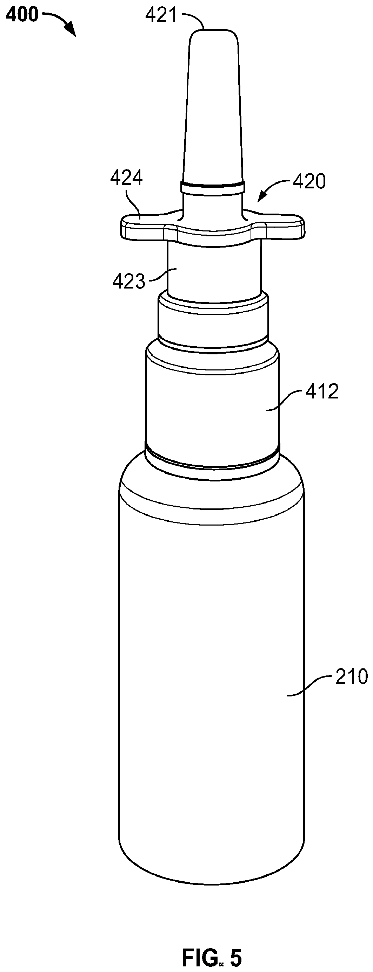

Another example of a multi-use package according to an embodiment of the present disclosure, and in particular a nasal spray bottle 400, is shown in Figures 5 to 8. The nasal spray bottle 400 comprises a vessel 210 having a wall 214, and more particularly one or more sidewalls 215 and a bottom wall 216, the wall (e.g. the one or more sidewalls and the bottom wall together) defining and at least partially enclosing a lumen 212. The sidewall 215 may comprise a main body portion 217 and a neck portion 218 having a reduced diameter relative to the main body portion, with the main body portion and the neck portion being connected by a transition region 219. Opposite the bottom wall 216 is an opening through which a fluid stored within the lumen may be dispensed from the vessel.

The nasal spray bottle 400 may also comprise a cap 412, which is securable to the vessel 210, and in particular which may be securable to the neck portion 218 of the vessel. The cap 412 may be secured to the vessel 210 in any of a variety of manners. In the illustrated embodiment, for example, the exterior surface of the neck portion 218 may comprise a threaded portion 413 configured to mate with a threaded portion on the interior surface of a cap 412 in order to secure the cap to the neck portion of the vessel.

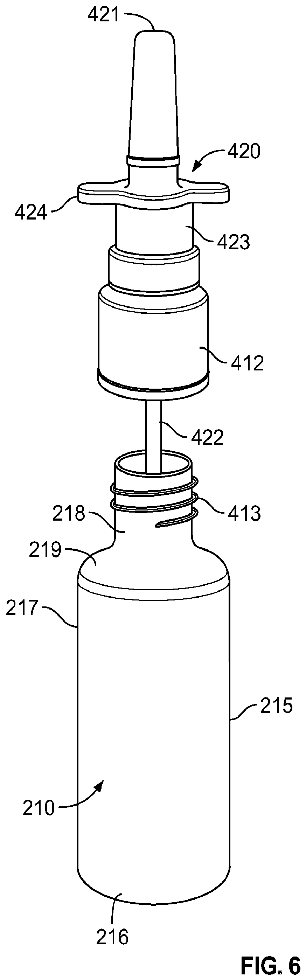

The nasal spray bottle 400 may further comprise a spray applicator 420. In some embodiments, including that illustrated in Figures 5 and 6 for example, the spray applicator 420 may form part of and/or be attached to (and/or a portion of the spray applicator may be integral with) the cap 412. The spray applicator 420 may comprise an outlet 421 configured to dispense a small volume dose of a medical fluid. Typically the small volume dose may be less than 0.5 mL, optionally less than 0.4 mL, optionally less than 0.3 mL, optionally less than 0.2 mL, optionally less than 0.1 mL. The spray applicator 420 may also comprise a dip tube 422 which extends into the lumen of the vessel 212 and desirably into close proximity with the bottom wall 216 of the vessel, and through which the medical fluid stored in the lumen of the vessel travels en route to the outlet 421. The spray applicator 420 further comprises an actuating element by which a user may dispense a small volume dose of medical fluid. In some embodiments, including that illustrated in Figures 5-6 for example, the actuating element may be a piston 423 that is manually operated by a user pushing down on a finger flange 424.

In some embodiments, the nasal spray bottle 400 may comprise a cap, also referred to as a hood, that covers the outlet of the spray applicator 421 when the bottle is not in use.

The nasal spray bottle further comprises a fluid 450, for example a medical fluid, within the lumen 212. In some embodiments, the medical fluid 450 may be a drug-containing solution. In some embodiments, the nasal spray bottle 400 may be configured so that the drug-containing solution is delivered to the user's brain and/or into the user's bloodstream, such as by intranasal delivery. In other embodiments, the nasal spray bottle 400 may be configured so that the drug-containing solution is locally targeted at the nasal passage, e.g. delivered as a topical administration. In some embodiments, for example, the nasally-delivered medical fluid 450 may comprise any one or more of the following: Azelastine Nasal, Azelastine and Fluticasone Nasal, Beclomethasone Nasal, Budesonide Nasal, Butorphanol Nasal, Calcitonin Nasal, Ciclesonide Nasal, Corticosteroid Nasal, Cromolyn Nasal, Cyanocobalamin Nasal, Desmopressin Nasal, Epinephrine Nasal, Fentanyl Citrate Nasal, Flunisolide Nasal, Fluticasone Nasal, Ipratropium Nasal, Ketorolac Nasal, Levocabastine Nasal, Metoclopramide Nasal, Mometasone Nasal, Nafarelin Nasal, Naphazoline Nasal, Nicotine Nasal, Olopatadine Nasal, Oxymetazoline Nasal, Phenylephrine Nasal, Sodium Chloride Nasal, Testosterone Nasal, Tetrahydrozoline Nasal, Triamcinolone Nasal, Xylometazoline Nasal.

In some embodiments, the medical solution 450 may contain one or more decongestants, such as pseudoephedrine, phenylephrine, propylhexedrine, phenylpropanolamine, levomethamphetamine, ephedrine, oxymetazoline, anphazoline, oxymetazoline, synephrine, tetryzoline, tramazoline, xylometazoline, and/or a corticosteroid (such as beclomethasone dipropionate, budesonide, ciclesonide, dexamethasone, flunisolide, fluticasone, fluticasone furoate, fluticasone propionate, azelastine/fluticasone, mometasone furoate, prednisolone, tixocortol, triamcinolone, and/or triamcinolone acedtonide); one or more antihistamines; one or more expectorants; saline; or a combination thereof. In some embodiments, the medical solution 450 may contain one or more migraine drugs. In some embodiments, the medical solution 450 may contain one or more antiasthma drugs. In some embdoiments, the medical solution 450 may contain one or more peptide drugs, e.g. for hormone treatment. In some embodiments, the medical solution 450 may contain one or more steroids. In some embodiments, the medical solution 450 may contain one or more anaesthetic agents.

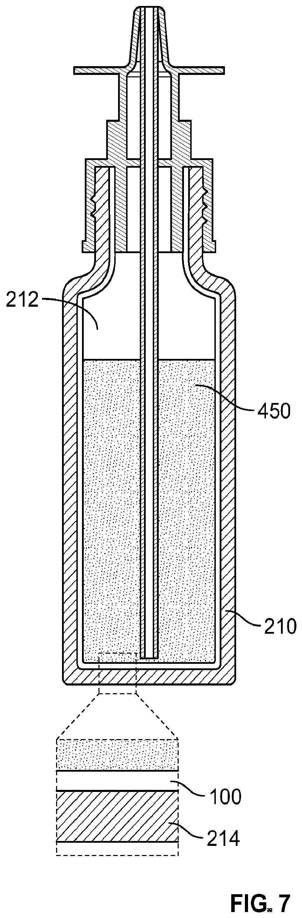

As shown in Figure 7, embodiments of the nasal spray bottle 400 comprise an antimicrobial coating 100 on at least a portion of the interior surface of the vessel wall 214 (e.g. on the interior surfaces of the sidewall 215 and the bottom wall 215), i.e. the surfaces that are in contact with the fluid 450 stored within the lumen 212. Note that Figure 7 is not intended to be drawn to scale and that the anti-microbial coating 100 may be applied as a very thin coating relative to the thickness of the vessel wall 214.

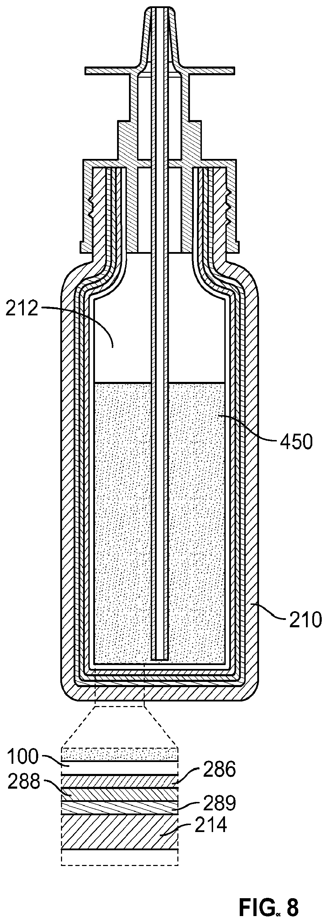

As shown in Figure 8, embodiments of the nasal spray bottle 400 may also comprise a coating set 285 including a barrier coating or layer 288 and optionally one or more of a tie coating or layer 289 and a pH protective coating or layer 286. Like the anti-microbial coating 100 described above, this coating set 285 may be applied to at least a portion of the interior surface of the vessel wall 214, e.g. to the interior surfaces of the sidewall 215 and/or the bottom wall 216 of the vessel. This coating set 285 may be provided in addition to the anti-microbial coating 100, e.g. as illustrated in Figure 8, or alone, i.e. independent of an anti-microbial coating (not illustrated). Generally, when applied in combination with an anti-microbial coating 100, the antimicrobial coating is applied as the innermost layer, i.e. the layer that is in contact with the fluid 450 stored within the lumen 212. Note that Figure 8 is not intended to be drawn to scale and that the various coatings may be applied as very thin coatings relative to the thickness of the vessel wall 214.

In some embodiments of the nasal spray bottle 400, the cap 412 or a combination of the cap and the hood may initially seal the opening of the vessel 210 in a manner that prevents moisture and/or atmospheric gas (e.g. oxygen) and/or bacteria from entering into the lumen 212. That initial seal is then broken by the end user upon the first opening of the nasal spray bottle 400, which typically corresponds with the first use of (i.e., dispensing of product from) the bottle. In other embodiments, the nasal spray bottle 400 may comprise a seal, e.g. a film, foil, or laminate, which extends over the opening of the vessel 210 and which may typically be sealed to an upper surface or top flange of the neck portion 218 so as to prevent moisture and/or atmospheric gas (e.g. oxygen) and/or bacteria from entering into the lumen 212. That seal is removed by the end user upon the first opening of the nasal spray bottle 400, which typically corresponds with the first use of (i.e., dispensing of product from) the bottle. In those embodiments, the seal may be covered by a separate cap and the cap comprising the spray applicator 420 may be detached from the vessel and/or provided separately. Regardless, however, where the nasal spray bottle 400 is in its initial sealed state, moisture and/or atmospheric gas (e.g. oxygen) may still enter into the lumen 212 of the vessel 210 through the vessel wall 214, which can result in deterioration of the fluid 450 contained within the lumen before the package is ever opened by the end user. Embodiments of the present invention therefore may comprise an oxygen barrier coating 288 that reduces the ingress of oxygen into the lumen compared to a vessel without the oxygen barrier coating. The resulting increased oxygen barrier properties may serve to increase the pre-opening shelf life of the package.

In use, the nasal spray bottle 400 is typically held near a user or patient's nose with at least the tip of the spray applicator 420 being placed into the user or patient's nostril, and the actuator is actuated one or more times in order to dispense the medical fluid into the nostril. In use therefore the spray applicator 420 may come into contact with the inside of the user or patient's nasal passage and with bacteria present therein, which can then enter into the lumen 212 of the vessel 210 in which the remainder of the medical fluid is stored for future use, thereby contaminating the medical fluid 450. Embodiments of the present invention comprise an antimicrobial coating 100 that is effective to inhibit the growth of microbes such as bacteria in the medical fluid 450 contained within the lumen 212 of the vessel (e.g. as compared to a vessel without the anti-microbial coating) and/or to inactivate or kill bacteria introduced into the lumen of the nasal spray bottle 400, and/or to increase the shelf-life of the nasal spray bottle package after first use.

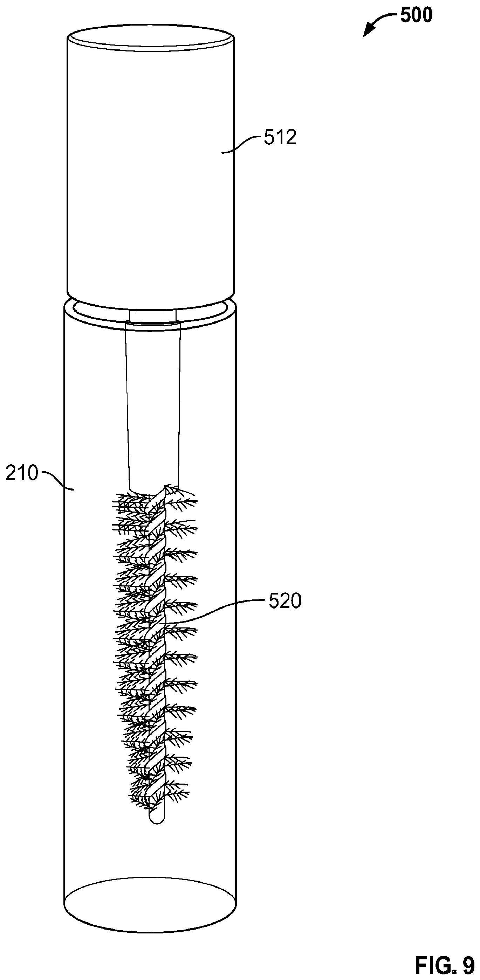

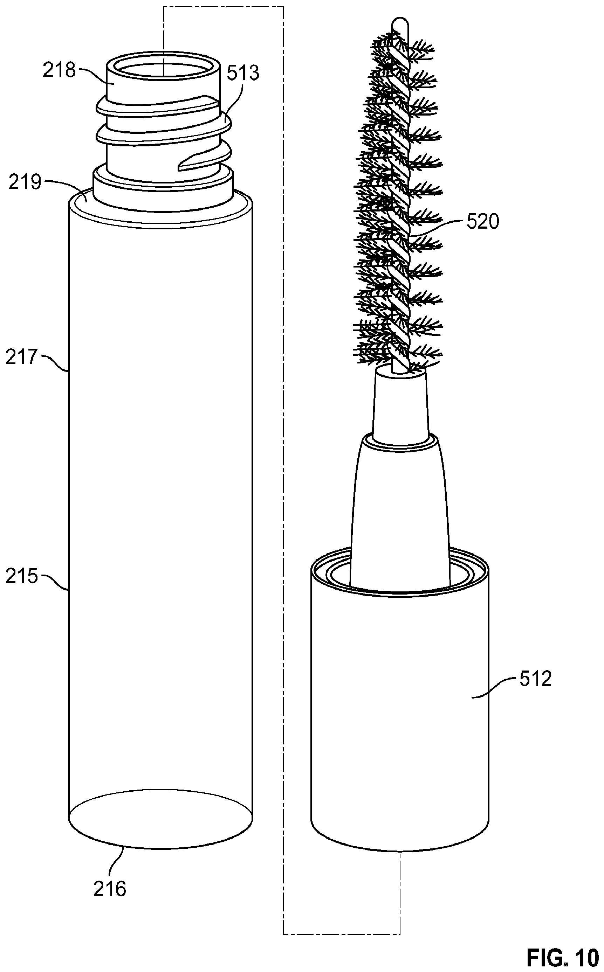

Another example of a multi-use package according to an embodiment of the present disclosure, and in particular an embodiment of a mascara bottle 500, also referred to as a mascara tube, is shown in Figures 9-12. The mascara bottle 500 comprises a vessel 210 having a wall 214, and more particularly one or more sidewalls 215 and a bottom wall 216, the wall (e.g. the one or more sidewalls and the bottom wall together) defining and at least partially enclosing a lumen 212. The sidewall 215 may comprise a main body portion 217 and a neck portion 218 having a reduced diameter relative to the main body portion, with the main body portion and the neck portion being connected by a transition region 219. Opposite the bottom wall 216 is an opening through which a fluid stored within the lumen, e.g. a mascara composition, may be dispensed from the vessel.

The mascara bottle 500 may also comprise a cap 512, the cap being configured to seal the opening in between uses (i.e., when the product is not being dispensed). The cap 512 may be secured to the vessel 210 in any of a variety of manners. In the illustrated embodiment, for example, the exterior surface of the neck portion 218 of the vessel may comprise a threaded portion 513 configured to mate with a threaded portion on the interior surface of a cap 512 in order to secure the cap to the neck portion of the vessel.

The mascara bottle 500 may also comprise an eyelash brush 520, also referred to as a wand, which is used to apply the mascara composition. In the mascara bottle 500 shown in Figures 9-10, the eyelash brush 520 is attached to cap 512. In other embodiments, however, the eyelash brush 520 may be separate from the cap 512. The eyelash brush 520 is sized and configured to be inserted through the opening of the vessel and into the lumen 212 of the vessel. When attached to the cap 512, the eyelash brush 520 may be inserted into the lumen 212 of the vessel 210 so as to take up am amount of the mascara composition contained therein and then the eyelash brush may be removed from the lumen of the vessel and used to apply the amount of mascara composition to the user's eyelashes. That process may be repeated a number of times until a desired amount of mascara has been applied. When the desired amount has been applied, the eyelash brush 520 may be inserted into the lumen 212 of the vessel and then the cap 512 may be rotated in order to secure the cap to the vessel 210.

The mascara bottle 500 further comprises a fluid 550, for example mascara composition, within the lumen 212. In some embodiments, the mascara composition 550 may include one or more pigments, one or more waxes and/or oils, and one or more film-forming polymers. In some embodiments, for example, the mascara composition 550 may include a carbon black, iron oxide, titanium dioxide, and/or ultramarine blue pigment to darken lashes; one or more polymers such as polyvinylpyrrolidone (PVP), ceresin, gum tragacanth, methyl ceullulose, etc., to form a film that coats lashes; one or more thickening waxes or oils such as lanolin, mineral oil, linseed oil, eucalyptus oil, sesame oil, oil of turpentine, paraffin, petrolatum, castor oil, carnauba wax, beeswax, palm wax, and candelilla wax. In some embodiments, the mascara composition 550 may comprise a base of wax selected from beeswax, paraffin, carnauba wax, palm wax, and a combination thereof, a pigment that includes carbon black and/or iron oxide, one or more film-forming polymers. Some embodiments of mascara compositions 550 may also contain nylon and/or rayon microfibers to provide the eyelashes with more length. Some embodiments of mascara compositions 550 may also contain a moisturizer and/or vitamins to condition eyelashes.

Some mascara compositions 550 may be free or substantially free of water, thereby providing what is considered a waterproof and hydrophobic mascara, while other mascara compositions may comprise an emulsion of water and oil(s), and be considered hydrophilic. An example waterproof mascara composition may contain petroleum distillate, polyethylene, carnauba wax, pentaerythrityl hydrogenated rosinate, and tall oil glycerides. An example hydrophilic mascara may contain, for example, water, glyceryl stearate, ammonium acrylates copolymer, polyvinyl alcohol, and alcohol.

In some embodiments, the mascara bottle 500 may be configured such that the lumen 212 of the vessel contains between about 3 mL and about 15 mL of fluid, alternatively between about 5 mL and about 12 mL of fluid, alternatively between about 7 mL and about 12 mL of fluid, alternatively between about 4 mL and about 12 mL, alternatively between about 4 mL and about 10 mL of fluid, alternatively about 4 mL of fluid, alternatively about 10 mL of fluid.

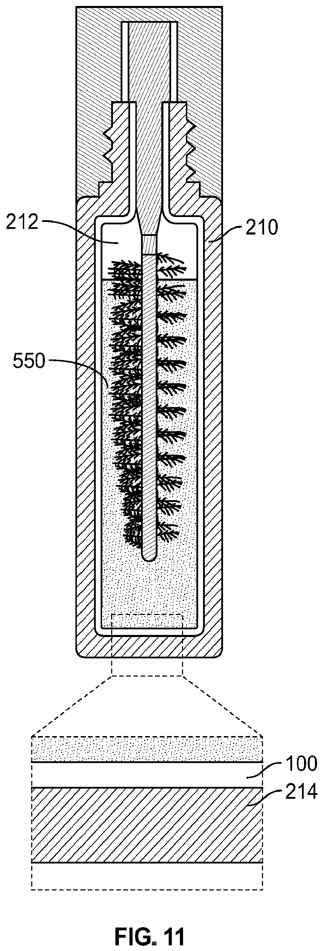

As shown in Figure 11, embodiments of the mascara bottle 500 comprise an antimicrobial coating 100 on at least a portion of the interior surface of the vessel wall 214 (on the interior surfaces of the sidewalls 215 and/or bottom wall 216), i.e. the surfaces that are in contact with the fluid 550 stored within the lumen 212. Note that Figure 11 is not intended to be drawn to scale and that the anti-microbial coating 100 may be applied as a very thin coating relative to the thickness of the vessel wall 214.

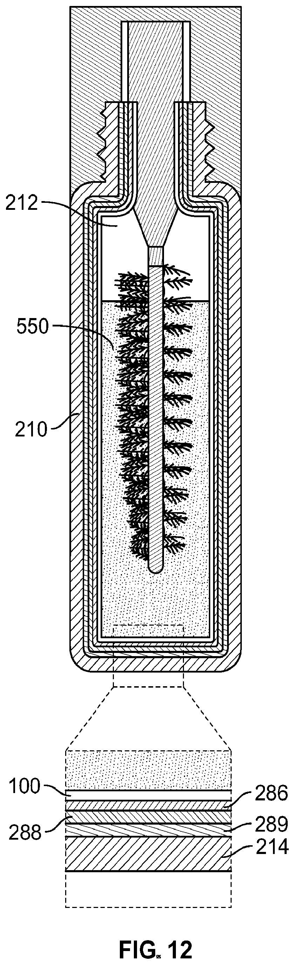

As shown in Figure 12, embodiments of the mascara bottle 500 may comprise a coating set 285 that includes a barrier coating or layer 288 and optionally one or more of a tie coating or layer 289 and a pH protective coating or layer 286. Like the anti-microbial coating 100 described above, this coating set 285 may be applied to at least a portion of the interior surface of the vessel wall 214, e.g. the interior surfaces of the sidewall 215 and/or bottom wall 216 of the vessel. This coating set 285 may be provided in addition to the anti-microbial coating 100, e.g. as illustrated in Figure 12, or alone, i.e. independent of an anti-microbial coating (not illustrated). Generally, when applied in combination with an anti-microbial coating 100, the anti-microbial coating is applied as the innermost layer, i.e. the layer that is in contact with the fluid 550 stored within the lumen. Note that Figure 12 is not intended to be drawn to scale and that the various coatings may be applied as very thin coatings relative to the thickness of the vessel wall 214.

In some embodiments of the mascara bottle 500, the cap 512 may initially seal the opening of the vessel 210 in a manner that prevents moisture and/or atmospheric gas (e.g. oxygen) and/or bacteria from entering into the lumen 212 (for example through the incorporation of one or more gaskets which may be compressed between the body of the cap and the vessel). That initial seal is then broken by the end user upon the first opening of the mascara bottle 500, which typically corresponds with the first use of (i.e., dispensing of product from) the bottle. In other embodiments, the mascara bottle 500 may comprise a seal, e.g. a film, foil, or laminate, which extends over the opening of the vessel and which may typically be sealed to an upper surface or top flange of the neck portion 218 so as to prevent moisture and/or atmospheric gas (e.g. oxygen) and/or bacteria from entering into the lumen 212. That seal is removed by the end user upon the first opening of the mascara bottle 500, which typically corresponds with the first use of (i.e., dispensing of product from) the bottle. In some embodiments, the mascara bottle 500 may comprise both initial seals. However, where the mascara bottle 500 is in its initial sealed state, moisture and/or atmospheric gas (e.g. oxygen) may still enter into the lumen 212 of the vessel 210 through the vessel wall 214, which can result in deterioration of the fluid 450 contained within the lumen 212 before the package is ever opened by the end user. Embodiments of the present invention therefore may comprise an oxygen barrier coating 288 that reduces the ingress of oxygen into the lumen 212 compared to a vessel without the oxygen barrier coating. The resulting increased oxygen barrier properties may serve to increase the pre-opening shelf life of the package.

During use, the eyelash brush 520 is typically placed into contact with the user's eyelashes and in close proximity to the user's face. As a result, the eyelash brush 520 may come into contact with bacteria, which can then enter into the lumen 212 of the vessel 210 in which the remainder of the mascara composition is stored for future use, thereby contaminating the mascara composition 550. Embodiments of the present invention comprise an anti-microbial coating 100 that is effective to inhibit the growth of microbes such as bacteria in the mascara composition 550 contained within the lumen 212 of the vessel 210 (e.g. as compared to a vessel without the anti-microbial coating) and/or to inactivate or kill bacteria introduced into the lumen of the mascara bottle 500, and/or to increase the shelf-life of the mascara bottle package after first use.

Another, non-illustrated example of a multi-use package according to an embodiment of the present disclosure is a liquid or gel eyeliner package (e.g., as opposed to a pencil eyeliner). Liquid or gel eyeliner is provided in a package that is similar to the mascara bottle/tube shown and described above. The primary difference - apart from the composition of the fluid itself - is the configuration of the applicator. Rather than an eyelash brush such as that shown in Figures 9-12, the applicator may comprise an eyeliner brush or pad. Like the eyelash brush shown in Figures 9-12, the eyeliner brush or pad typically extends from the underside of the cap and is inserted into and stored within the lumen of the vessel when the cap is secured to the vessel. The primary difference is in the configuration of the brush itself, as an eyeliner brush will typically have a fine, sharp-tipped brush that extends from the end of the applicator and which creates a fine, precise line on the skin around the eye as opposed to an eyelash brush which extends around the periphery/circumference of at least a portion of the applicator and is used to apply mascara to the eyelashes. The eyeliner fluid stored in the lumen of the vessel may include one or more film formers, one or more thickeners (e.g. waxes such as Japan wax, natural gums, clays), and/or one or more pigments (e.g. iron oxides, ultramarine, chromium oxide, titanium dioxide). One or more of the antimicrobial coating or layer 100 and/or the coating set 285 may be provided on the interior surface(s) of the eyeliner vessel in the same manner as described above with respect to mascara bottles/tubes and will function in the same manner.

Another, non-illustrated example of a multi-use package according to an embodiment of the present disclosure is a lip gloss package. Lip gloss may be provided in a package that is similar to the mascara bottle/tube shown and described above and the eyeliner bottle/tube described above. Once again, the primary difference - apart from the composition of the fluid itself - is the configuration of the applicator. Rather than an eyelash brush such as that shown in Figures 9-12, or an eyeliner brush, the applicator may comprise a lip brush, sometimes called a wand. Like the eyelash brush shown in Figures 9-12 and the eyeliner brush described above, the lip brush typically extends from the underside of the cap and is inserted into and stored within the lumen of the vessel when the cap is secured to the vessel. The primary difference is in the configuration of the brush itself, as a lip brush will typically have a spongy material at the tip of the applicator, which is used to brush the lip gloss onto a user's lips. The spongy tip (sometimes referred to as a "doe foot") may be provided in any of a variety of shapes, including curved, spatula, flame, and drop configurations. The lip gloss fluid stored in the lumen of the vessel may include one or more waxes (e.g. lanolin), one or more oils, and/or one or more pigments. One or more of the antimicrobial coating or layer 100 and/or the coating set 285 may be provided on the interior surface(s) of the lip gloss vessel in the same manner as described above with respect to mascara bottles/tubes and will function in the same manner.

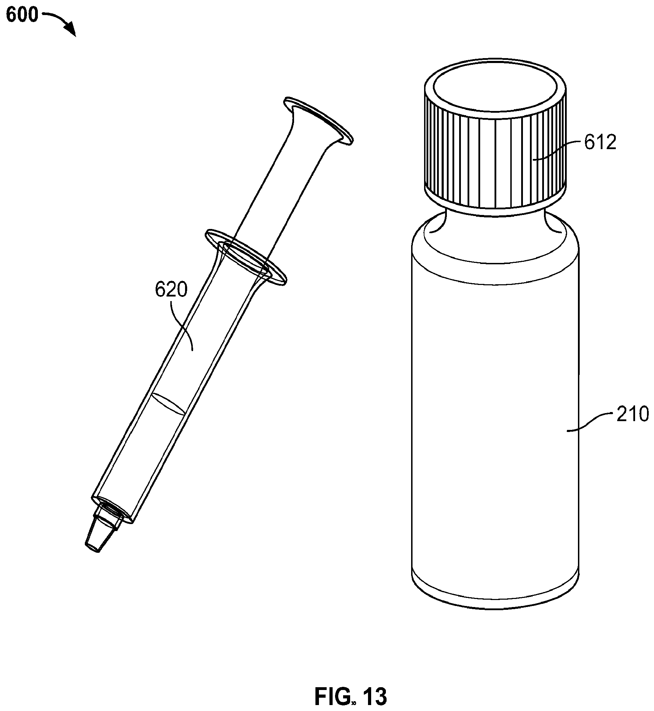

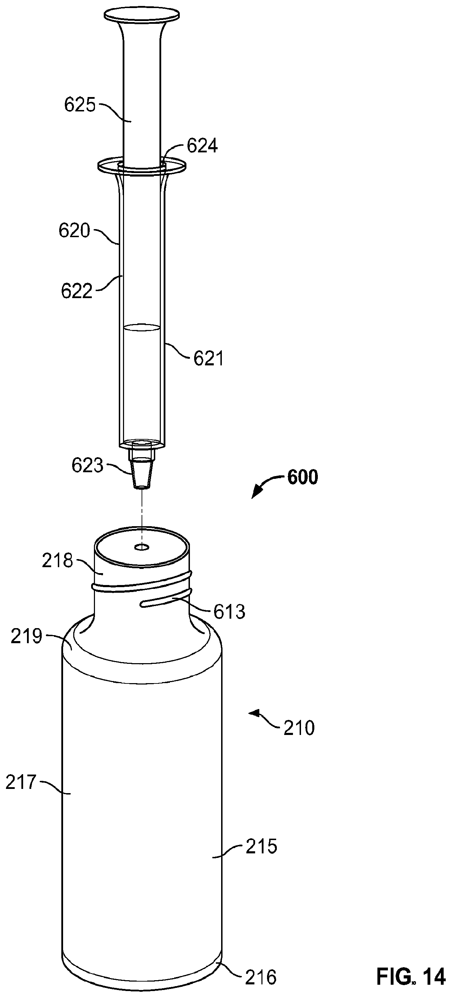

Another example of a multi-use package according to an embodiment of the present disclosure, and in particular an embodiment of a small-dose medicine package 600, is shown in Figures 13-16. The small-dose medicine package 600 comprises a vessel 210 having a wall 214, and more particularly one or more sidewalls 215 and a bottom wall 216, the wall (e.g. the one or more sidewalls and the bottom wall together) defining and at least partially enclosing a lumen 212. The sidewall 215 may comprise a main body portion 217 and a neck portion 218 having a reduced diameter relative to the main body portion, with the main body portion and the neck portion being connected by a transition region 219. Opposite the bottom wall 216 is an opening through which a fluid stored within the lumen, e.g. the small-dose medical fluid, may be dispensed from the vessel 210.

The small-dose medicine bottle 600 may also comprise a cap 612, the cap being configured to seal the opening in between uses (i.e., when the product is not being dispensed). The cap 612 may be secured to the vessel 210 in any of a variety of manners. In the illustrated embodiment, for example, the exterior surface of the neck portion 218 of the vessel may comprise a threaded portion 613 configured to mate with a threaded portion on the interior surface of a cap 612 in order to secure the cap to the neck portion of the vessel.

Small-dose medicine packages 600, such as those for infants, toddlers, or pets often include an applicator 620, such as a dropper (which can be integrated into a cap and provided as a dropper cap or which can be an independent component) or a plunger-operated applicator, as is illustrated in Figures 13-16.

A plunger-operated applicator 620 may comprise a plastic barrel 621 having a main body 622 portion, a narrowed dispensing tip 623 at a first end, and an opening 624 at the opposite end. A plastic plunger 625 is inserted into the barrel 621 through the opening 624 and is slidable within the barrel. In some embodiments, the barrel 621 may contain a series of ridges and/or markings that correspond with various dosage measurements, whereas in other embodiments the applicator 620 may be configured for only a single dosage size. During use, the dispensing tip 623 of the applicator 620 is inserted into the lumen 212 of the vessel 210 and a desired dosage of medical fluid 650 is pulled into the barrel 621of the applicator by suction produced by movement of the plunger 625 rearward away from the dispensing tip 623. The properly measured dosage of medical fluid is then dispensed through the dispensing tip 623 of the applicator 620 directly to its intended location, typically the mouth of an infant, toddler, or pet.

A dropper-type applicator is used in substantially the same manner, with a desired dosage of the fluid being pulled from the lumen 212 of the vessel 210 into the dropper through suction caused by a user slowly releasing pressure on a rubber bulb that is attached to an opening at the non-dispensing end of the dropper and the dosage of fluid then being dispensed through the dispensing end of the dropper by application of pressure to the rubber bulb. Although these applicators 620 are typically inserted into the mouth of the recipient, they may not be thoroughly washed between uses. Accordingly, bacteria from the mouth may be introduced into the medical fluid contained within the lumen 212 of the vessel 210 either immediately after use (e.g. if the applicator 620 is stored in the vessel lumen) or during a subsequent use (when the applicator is re-introduced into the lumen).

The small-dose medicine package 600 further comprises a fluid 650, for example a medicinal formulation within the lumen 212 of the vessel 210. In some embodiments, the fluid 650 may comprise an analgesic drug such as acetaminophen or ibuprofen. In some embodiments, the fluid 650 may comprise a prescription drug.

In some embodiments, the applicator 620 may be configured to hold and dispense up to about 10 mL of fluid, alternatively up to about 7 mL of fluid, alternatively up to about 5 mL of fluid, alternatively up to about 2 mL of fluid.

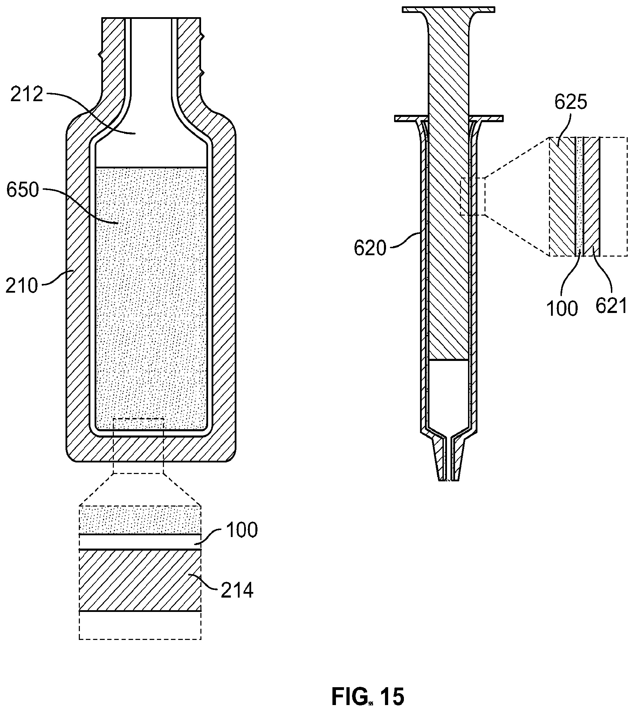

As shown in Figure 15, embodiments of the vessel 210 of the small-dose medicine package 600 comprise an anti-microbial coating 100 on at least a portion of the interior surface of the vessel wall 214 (e.g. on the interior surfaces of the sidewall 215 and/or the bottom wall 216), i.e. the surfaces that are in contact with the fluid 650 stored within the lumen 212. Note that Figure 15 is not intended to be drawn to scale and that the anti-microbial coating 100 may be applied as a very thin coating relative to the thickness of the vessel wall 214.

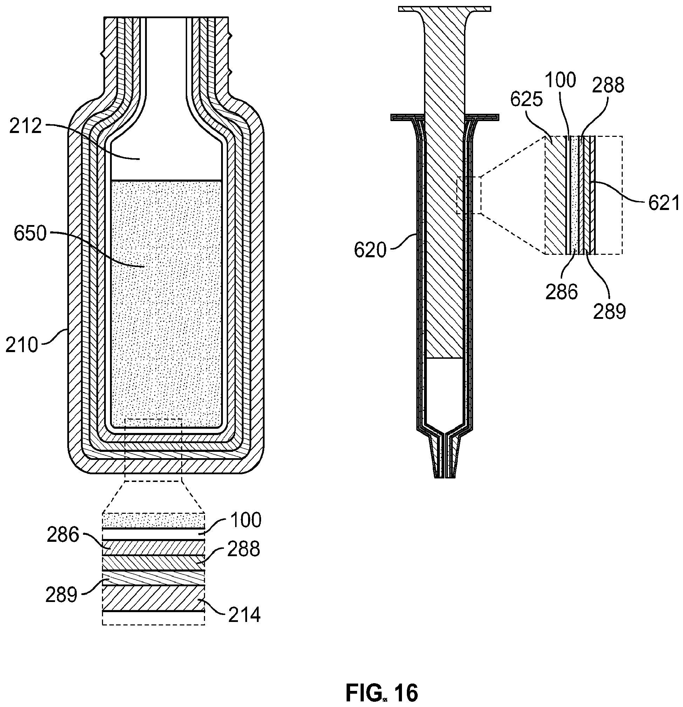

As shown in Figure 16, embodiments of the vessel 210 of the small-dose medicine package 600 may comprise a coating set 285 comprising a barrier coating or layer 288 and optionally one or more of a tie coating or layer 289 and a pH protective coating or layer 286. Like the anti-microbial coating 100 described above, this coating set 285 may be applied to at least a portion of the interior surface 214 of the vessel, e.g. the interior surfaces of the sidewall 215 and/or the bottom wall 216 of the vessel. This coating set 285 may be provided in addition to the anti-microbial coating 100, e.g. as illustrated in Figure 16, or alone, i.e. independent of an antimicrobial coating (not illustrated). Generally, when applied in combination with an anti-microbial coating 100, the anti-microbial coating is applied as the innermost layer, i.e. the layer that is in contact with the fluid 650 stored within the lumen 212. Note that Figure 16 is not intended to be drawn to scale and that the various coatings may be applied as very thin coatings relative to the thickness of the vessel wall 214.

In some embodiments, one or more surfaces of the applicator 620 may also comprise an anti-microbial coating 100. For example, where the small-dose medicine package 600 comprises a plunger-operated applicator 620, the interior surface of at least a portion of the barrel wall 621 and/or the exterior surface of at least a portion of the barrel wall may be provided with an anti-microbial coating 100 as described herein. In some embodiments, and as illustrated in Figure 15, the interior surface of at least a portion of the barrel wall 621 may be provided with an anti-microbial coating 100 as described herein. In some embodiments, the exterior surface of at least a portion of the barrel wall 621 may be provided with an anti-microbial coating 100 as described herein. In some embodiments, both the interior surface of at least a portion of the barrel wall 621 and the exterior surface of at least a portion of the barrel wall may be provided with an anti-microbial coating 100 as described herein.

In some embodiments of the small-dose medicine package 600, the cap 612 may initially seal the opening of the vessel 210 in a manner that prevents moisture and/or atmospheric gas (e.g. oxygen) and/or bacteria from entering into the lumen 212 (for example through the incorporation of one or more gaskets which may be compressed between the body of the cap and the vessel). That initial seal is then broken by the end user upon the first opening of the small-dose medicine package 600, which typically corresponds with the first use of (i.e., dispensing of product from) the bottle. In other embodiments, the small-dose medicine package 600 may comprise a seal, e.g. a film, foil, or laminate, which extends over the opening of the vessel and which may typically be sealed to an upper surface or top flange of the neck portion 218 so as to prevent moisture and/or atmospheric gas (e.g. oxygen) and/or bacteria from entering into the lumen 212. That seal is removed by the end user upon the first opening of the small-dose medicine package 600, which typically corresponds with the first use of (i.e., dispensing of product from) the bottle. In some embodiments, the small-dose medicine package 600 may comprise both initial seals. Regardless, however, where the small-dose medicine package 600 is in its initial sealed state, moisture and/or atmospheric gas (e.g. oxygen) may still enter into the lumen 212 of the vessel 210 through the vessel wall 214, which can result in deterioration of the fluid 650 contained within the lumen before the package is ever opened by the end user. Embodiments of the present invention therefore may comprise an oxygen barrier coating 288 that reduces the ingress of oxygen into the lumen 212 compared to a vessel without the oxygen barrier coating. The resulting increased oxygen barrier properties may serve to increase the pre-opening shelf life of the package.

During use, the applicator 620, and more particularly the dispensing tip 623 of the applicator (and often a portion of the main body 622 adjacent the dispensing tip), is placed into the recipient's mouth and the medical fluid dispensed directly into the recipient's mouth. As a result, the applicator 620 may come into contact with bacteria, which can then enter into the lumen 212 of the vessel 210 in which the remainder of the medical fluid is stored for future use, thereby contaminating the medical fluid 650 contained within the lumen. Embodiments of the present invention comprise an anti-microbial coating 100 that is effective to inhibit the growth of microbes such as bacteria in the medical fluid 650 contained within the lumen 212 of the vessel 210 (e.g. as compared to a vessel without the anti-microbial coating) and/or to inactivate or kill bacteria introduced into the lumen of the small-dose medicine package 600, and/or to increase the shelf-life of the small-dose medicine package after first use.

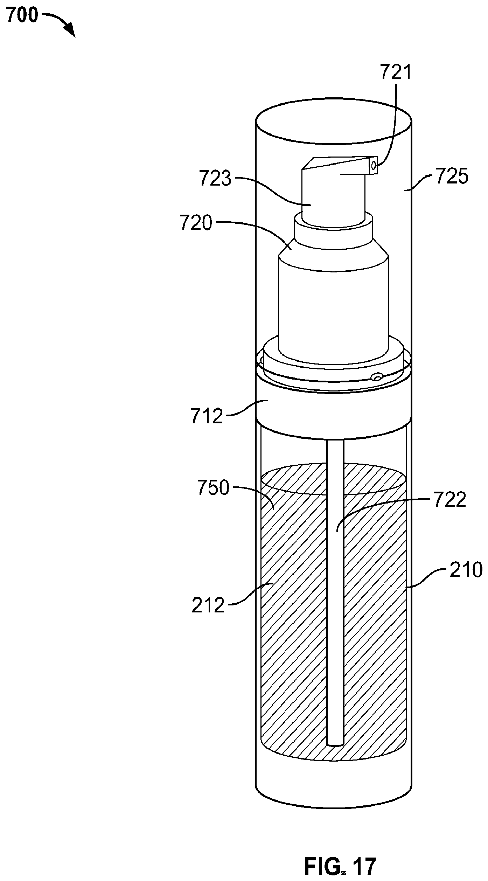

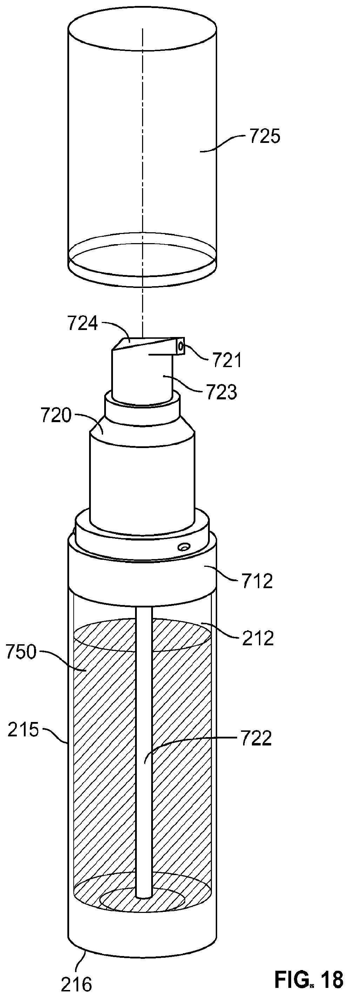

Another example of a multi-use package according to an embodiment of the present disclosure, and in particular a pump bottle 700, is shown in Figures 17-20. The pump bottle 700 comprises a vessel 210 having a wall 214, and more particularly one or more sidewalls 215 and a bottom wall 216, the wall (e.g. the one or more sidewalls and the bottom wall together) defining and at least partially enclosing a lumen 212. The sidewall 215 may comprise a main body portion 217 and a neck portion 218 having a reduced diameter relative to the main body portion, with the main body portion and the neck portion being connected by a transition region 219. Opposite the bottom wall 216 is an opening through which a fluid stored within the lumen may be dispensed from the vessel.

The pump bottle 700 may also comprise a pump cap 712, which is securable to the vessel 210, and in particular which may be securable to the neck portion 218 of the vessel. The pump cap 712 may be secured to the vessel 210 in any of a variety of manners. In some embodiments, for example, the exterior surface of the neck portion 218 may comprise a threaded portion configured to mate with a threaded portion on the interior surface of the pump cap 712 in order to secure the pump cap to the neck portion of the vessel 210.

The pump bottle 700 may further comprise a pump applicator 720. The pump applicator 720 may form part of and/or be attached to the pump cap 712. The pump applicator 720 comprises an outlet 721 configured to dispense an amount of a fluid contained within the lumen 212 of the vessel 210, typically into a user's hand. The pump applicator 720 may also comprise a dip tube 722 which extends into the lumen 212 of the vessel 210 and desirably into close proximity with the bottom wall 216 of the vessel, and through which the fluid stored in the lumen of the vessel travels en route to the outlet 721. The pump applicator 720 further comprises an actuating element 723 by which a user may dispense an amount of fluid from the outlet 721. In some embodiments, including that illustrated in Figures 17-18 for example, the actuating element 723 may be a piston that is manually operated by a user pushing down on an upper surface 724 of the pump applicator 720.

The pump bottle 700 may also comprise a cap 725, also referred to as a hood, that covers the outlet 721 of the pump applicator 720 when the bottle is not in use.

The pump bottle 700 further comprises a fluid 750, for example a cosmetic fluid, within the lumen 212. In some embodiments, the cosmetic fluid 750 may be a cream or lotion such as a moisturing and/or conditioning cream or lotion (including e.g. a baby cream or lotion), a skin care cream or lotion, an anti-aging cream or lotion, a pore cleansing cream or lotion, a shaving cream or lotion, or the like. In some embodiments, the fluid 750 may be a medical fluid, such as an ointment, salve, or medical cream. In some embodiments, the fluid 750 may be a fragrance composition, such as a perfume or cologne.

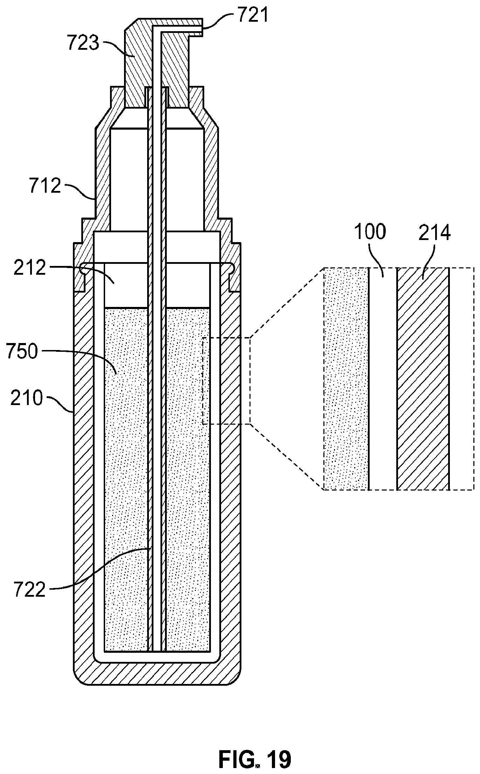

As shown in Figure 19, embodiments of the pump bottle 700 comprise an anti-microbial coating 100 on at least a portion of the interior surface of the vessel wall 214 (e.g. on the interior surfaces of the sidewall 215 and/or bottom wall 216), i.e. the surfaces that are in contact with the fluid stored within the lumen 212. Note that Figure 19 is not intended to be drawn to scale and that the anti-microbial coating 100 may be applied as a very thin coating relative to the thickness of the vessel wall 214.

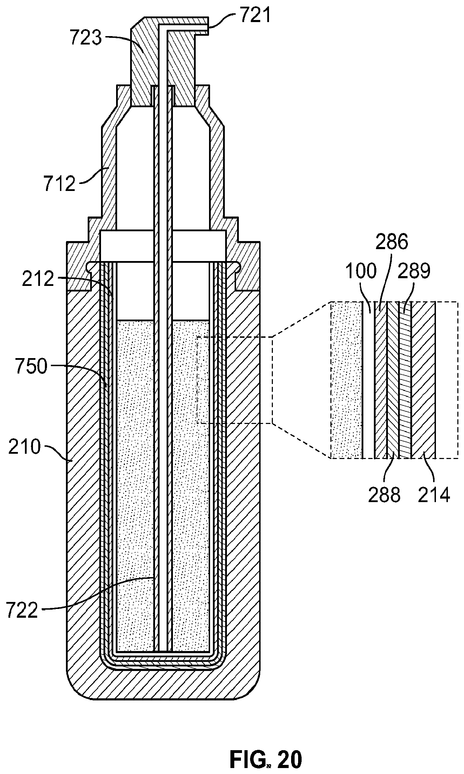

As shown in Figure 20, embodiments of the pump bottle 700 may also comprise a coating set 285 that includes a barrier coating or layer 288 and optionally one or more of a tie coating or layer 289 and a pH protective coating or layer 286. Like the anti-microbial coating 100 described above, this coating set 285 may be applied to at least a portion of the interior surface of the vessel wall 214, e.g. the interior surfaces of the sidewall(s) 15 and the bottom wall 16 of the vessel. This coating set 285 may be provided in addition to the anti-microbial coating 100, e.g. as illustrated in Figure 20, or alone, i.e. independent of an anti-microbial coating (not illustrated). Generally, when applied in combination with an anti-microbial coating 100, the anti-microbial coating is applied as the innermost layer, i.e. the layer that is in contact with the fluid 750 stored within the lumen 212. Note that Figure 20 is not intended to be drawn to scale and that the various coatings may be applied as very thin coatings relative to the thickness of the vessel wall 214.

In some embodiments of the pump bottle 700, the cap 712 or a combination of the cap and the hood 725 may initially seal the vessel 210 in a manner that prevents moisture and/or atmospheric gas (e.g. oxygen) and/or bacteria from entering the lumen 212 (for example through the incorporation of one or more gaskets which may be compressed between the body of the cap and the vessel). That initial seal is then broken by the end user upon the first opening of the pump bottle package 700, which typically corresponds with the first use of (i.e., dispensing of product from) the bottle. In other embodiments, the pump bottle 700 may comprise a seal, e.g. a film, foil, or laminate, which extends over the opening of the vessel 210 and which may typically be sealed to an upper surface or top flange of the neck portion 218 so as to prevent moisture and/or atmospheric gas (e.g. oxygen) and/or bacteria from entering into the lumen 212. That seal is removed by the end user upon the first opening of the pump bottle package 700, which typically corresponds with the first use of (i.e., dispensing of product from) the bottle. In those embodiments, the seal may be covered by a separate cap and the pump cap 712 may be detached from the vessel 210 and/or provided separately such that a user attaches the pump cap to the vessel once he/she has removed the seal. Regardless, however, where the pump bottle 700 is in its initial sealed state, moisture and/or atmospheric gas (e.g. oxygen) may still enter into the lumen 212 of the vessel 210 through the vessel wall(s) 214, which can result in deterioration of the fluid 750 within the lumen before the package is ever opened by the end user. Embodiments of the present invention therefore may comprise an oxygen barrier coating 288 that reduces the ingress of oxygen into the lumen 212 compared to a vessel without the oxygen barrier coating. The resulting increased oxygen barrier properties may serve to increase the pre-opening shelf life of the package.

In use, a user typically places his/her hand in close proximity to the outlet 721 of the pump applicator 720 and operates the actuator 723, e.g. pushes down on the upper surface 724 of the pump applicator, to dispense a desired amount of fluid 750 from the lumen of the vessel 210. Often the user's hand will come into contact with the outlet 721 of the pump applicator 720, particularly because operation of the actuator 723 may cause the outlet to move downward toward the user's hand. In use therefore the pump applicator 720 may come into contact with bacteria present on the user's hand, which can then enter into the lumen 212 of the vessel 210 in which the remainder of the fluid is stored for future use, thereby contaminating the fluid 750. Embodiments of the present invention therefore comprise an anti-microbial coating 100 that is effective to inhibit the growth of microbes such as bacteria in the fluid 750 contained within the lumen 212 of the vessel 210 (e.g. as compared to a vessel without the anti-microbial coating) and/or to inactivate or kill bacteria introduced into the lumen of the pump bottle 700, and/or to increase the shelf-life of the pump bottle package after first use.

Another, non-illustrated example of a multi-use package according to an embodiment of the present disclosure, is a cosmetic jar. A cosmetic jar comprises a vessel having one or more sidewalls 215 and a bottom wall 216, the one or more sidewalls and the bottom wall together defining and at least partially enclosing a lumen 212. Opposite the bottom wall 216 is an opening sized and configured so that a user may dip his/her fingers (which thereby serve as an applicator) or an applicator into the lumen 212 in order to take up an amount of the cosmetic product, e.g. a cream or the like, contained therein. The cosmetic jar may further comprise a cap that is removable and resealable on the vessel.

The cosmetic jar further comprises a fluid, e.g. a cosmetic cream or lotion, contained within the vessel lumen.

Embodiments of the cosmetic jar comprise an anti-microbial coating 100 on at least a portion of the interior surface of the vessel wall 214 (e.g. the interior surfaces of the one or more sidewalls 215 and the bottom wall 216), i.e. the surfaces that are in contact with the fluid stored within the lumen 212. Embodiments of the cosmetic jar may also comprise a coating set 285 that includes a barrier coating or layer 288 and optionally one or more of a tie coating or layer 289 and a pH protective coating or layer 286. Like the anti-microbial coating 100 described above, this coating set 285 may be applied to at least a portion of the interior surface of the vessel wall 214, e.g. the interior surfaces of the sidewalls 215 and/or the bottom wall 216 of the vessel. This coating set 285 may be provided in addition to the anti-microbial coating 100 or alone, i.e. independent of an anti-microbial coating. Generally, when applied in combination with an anti-microbial coating 100, the anti-microbial coating is applied as the innermost layer, i.e. the layer that is in contact with the fluid stored within the lumen 212.

In some embodiments of the cosmetic jar, the cap may initially seal the opening of the vessel 210 in a manner that prevents moisture and/or atmospheric gas (e.g. oxygen) and/or bacteria from entering into the lumen 212 (for example through the incorporation of one or more gaskets which may be compressed between the body of the cap and the vessel). That initial seal is then broken by the end user upon the first opening of the cosmetic jar which typically corresponds with the first use of (i.e., dispensing of product from) the jar. In other embodiments, the cosmetic jar may comprise a seal, e.g. a film, foil, or laminate, which extends over the opening of the vessel 210 and which may typically be sealed to an upper surface or top flange of the vessel sidewall(s) so as to prevent moisture and/or atmospheric gas (e.g. oxygen) and/or bacteria from entering into the lumen. That seal is removed by the end user upon the first opening of the cosmetic jar, which typically corresponds with the first use of (i.e., dispensing of product from) the jar. In some embodiments, the cosmetic jar may comprise both initial seals. However, where the cosmetic jar is in its initial sealed state, moisture and/or atmospheric gas (e.g. oxygen) may still enter into the lumen 212 of the vessel 2100 through the vessel wall 214, which can result in deterioration of the fluid contained within the lumen before the jar is ever opened by the end user. Embodiments of the present invention therefore may comprise an oxygen barrier coating 288 that reduces the ingress of oxygen into the lumen compared to a vessel without the oxygen barrier coating. The resulting increased oxygen barrier properties may serve to increase the pre-opening shelf life of the package.

In use, a user typically places his/her hand into the lumen 212 of the vessel 210 in order to extract the cosmetic product, e.g. cream or lotion, contained therein. At each use, therefore, bacteria present on the user's hand can easily enter into the lumen 212 of the vessel 210 in which the remainder of the product is stored for future use, thereby contaminating the product. Embodiments of the present invention therefore comprise an anti-microbial coating that is effective to inhibit the growth of microbes such as bacteria in the fluid contained within the lumen of the vessel (e.g. as compared to a vessel without the anti-microbial coating) and/or to inactivate or kill bacteria introduced into the lumen of the cosmetic jar, and/or to increase the shelf-life of the cosmetic jar product after first use.

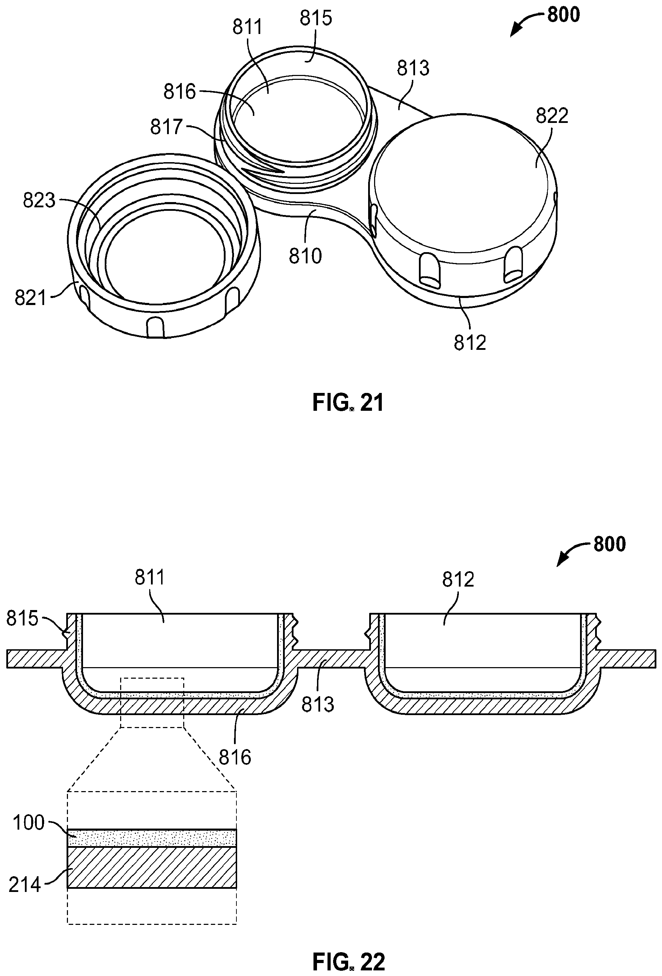

Another example of a multi-use package according to an embodiment of the present disclosure, and in particular an embodiment of a contact lens case 800, is shown in Figures 21-22. The contact lens case comprises a vessel 810 having a first reservoir 811 and a second reservoir 812. Each of the first and second reservoirs 811, 812 is defined by a side wall 815 and a bottom wall 816, the side wall and the bottom wall together defining the reservoir. Opposite the bottom wall 816 of each reservoir 811, 812 is an opening through which a contact lens may be placed into the reservoir. The first and second reservoirs 811, 812 are typically connected together by a connecting portion 813.

The contact lens case 800 may also comprise a first cap 821 and a second cap 822, each of the first and second caps 821, 822 being configured to close the opening of one of the first and second reservoirs 811, 812. The caps 821, 822 may be secured to the vessel 810 in any of a variety of manners. In the illustrated embodiment, for example, the exterior surface of the sidewall 815 of each reservoir 811, 812 may comprise a threaded portion 817 configured to mate with a threaded portion 823 on the interior surface of a cap 821, 822 in order to secure the cap to the vessel.

The contact lens case 800 differs from the other embodiments described herein in that the contact lens case is not a product package in which the lumen of a vessel is filled with a multi-use, cosmetic, and/or fragrance product, when purchased, but rather acts as a reusable storage container for contact lenses which is typically purchased with the reservoirs being empty. As such, the contact lense case 800 does not have a pre-opening shelf life and thus there is no need for an oxygen barrier coating of the sort described herein. However, at least a portion of the walls that define each of the first and second reservoirs 811, 812 and/or at least a portion of the interior surface, e.g. underside, of each of the first and second caps 821, 822 may be coated with an anti-microbial coating 100 of the sort described herein.

In use, each of the first and second reservoirs 811, 812 is typically partially filled with a contact lens solution, which may be configured to clean, rinse, and/or disinfect contact lenses, a contact lens is placed - with a user's fingers - into each reservoir for storage, and the first and second caps 821, 822 are secured onto the first and second reservoirs. Then, after a period of storage time, the user removes each of the first and second caps 821, 822 and extracts the contact lenses from the reservoirs 811, 812, again using his/her fingers. As a result, each of the first and second reservoirs 811, 812 may come into contact with bacteria from the user's hand both during insertion and removal of the contact lenses. Similarly, when a user removes each of the first and second caps 821, 822, the interior surface of the cap may come into contact with the user's hand and/or any of a variety of surfaces on which they may be placed, any of which may contain bacteria. Contact lens solution and/or contact lenses may thus be enclosed in a contaminated environment for storage.

Embodiments of the present invention comprise an anti-microbial coating 100 that is effective to inhibit the growth of microbes such as bacteria in the reservoirs 811, 812 of a contact lens case 800 (e.g. as compared to a contact lens case without the anti-microbial coating) and/or to inactivate or kill bacteria introduced into the reservoirs of the contact lens case, and/or to increase the shelf-life of the contact lens case after first use.

As shown in Figure 22, embodiments of the contact lens case 800 may comprise an anti-microbial coating 100 on at least a portion of the interior surface of the wall 214, e.g. the interior surfaces of the sidewall 215 and/or the bottom wall 216 of each reservoir 211, 212. Similarly, embodiments of the contact lens case 800 may comprise an anti-microbial coating 100 on the interior surface, e.g. underside, of each of the first and second caps 821, 822. Note that Figure 22 is not intended to be drawn to scale and that the anti-microbial coating 100 may be applied as a very thin coating relative to the thickness of the vessel and cap walls 214.

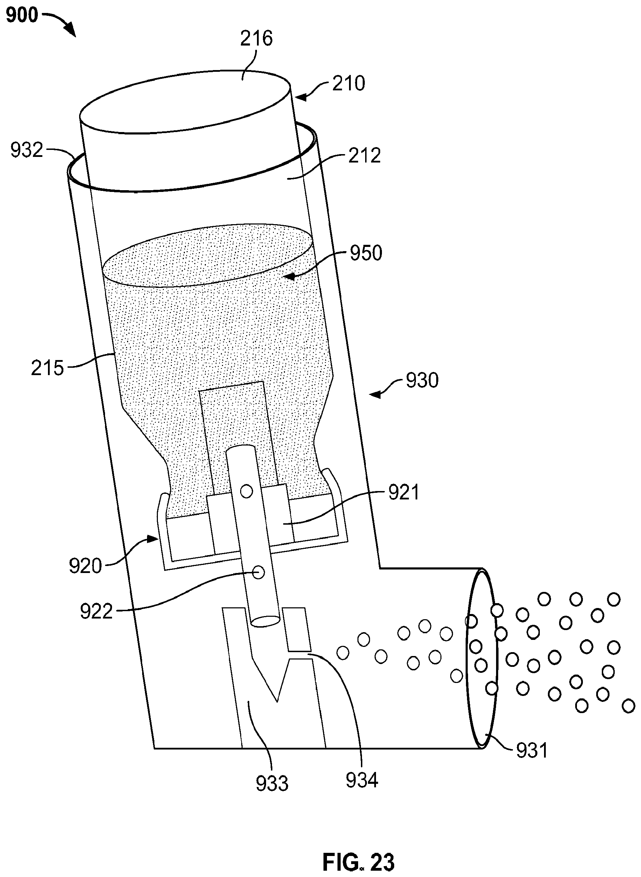

Another example of a multi-use package according to an embodiment of the present disclosure, and in particular a multi-dose inhaler or metered-dose inhaler (MDI) 900 is shown in Figure 23. The multi-dose inhaler 900 comprises a vessel 210, e.g. a cannister, having a side wall 215 and a bottom wall 216, the side wall and the bottom wall together defining and at least partially enclosing a lumen 212. The sidewall 215 may comprise a main body portion 217 and a neck portion 218 having a reduced diameter relative to the main body portion, with the main body portion and the neck portion being connected by a transition region 219. Opposite the bottom wall 216 is an opening through which a fluid stored within the lumen 212 may be dispensed from the vessel 210. The canister 210 may further comprise a metering valve 920 which may comprise a metering chamber 921 and a metering valve stem 922 that extends from the top of the canister. The metering valve 920 may serve as the opening of the canister through which fluid stored within the lumen may be dispensed from the vessel.

The canister 210 may also comprise a cap, which is securable to the vessel, and in particular which may be secured to the neck portion 218 of the vessel, and cover the meter valve stem 922, prior to first use.

The multi-dose inhaler 900 may further comprise an actuator 930. The actuator 930 may comprise a plastic body having a mouthpiece 931 at a first end and an opening 932 sized and configured to receive the canister 210 at the opposite end. The actuator 930 may further comprise a seat 933 configured to receive the metering valve stem 922 of the canister 210 and a nozzle 934 configured to spray the fluid contents out of the mouthpiece 931. The actuator 930 may also comprise a cap configured to cover the mouthpiece 931 when not in use.

The multi-dose inhaler 900 further comprises a fluid 950, for example a medical fluid, within the lumen 212 of the canister. In some embodiments, the medical fluid 950 may be a drug-containing solution that is formulated for administration into the lungs of a patient. In some embodiments, the drug-containing solution may comprise a respiratory drug, such as one configured to treat asthma, chronic obstructive pulmonary disease (COPD), or other respiratory diseases. In some embodiments, for example, the medical fluid 950 may comprise one or more bronchodilators, one or more corticosteroids, or a combination thereof. In some embodiments, the medical fluid 950 may comprise one or more mast cell stabilizers, such as cromoglicate or nedocromil, or one or more phospholipids. The fluid 950 may further comprise one or more propellants. In some embodiments, for instance, the fluid 950 may comprise a hydrofluorocarbon, e.g. hydrofluoroalkane, propellant.

In use, a canister 210 is inserted into the actuator 930 and the metering valve stem 922 of the canister placed into operably engagement with the seat 933 of the actuator. The mouthpiece 931 is placed into the mouth of the user and the actuator is operated by pressing down on the bottom wall 216 of the canister 210, which causes a metered dose of fluid to be discharged from the canister through the metering valve 920 and out of the actuator nozzle 934. The drug may be dissolved or suspended in the propellant. Discharge through the nozzle breaks up the volatile propellant into droplets, which are then rapidly evaporated, resulting in an aerosol of micrometer-sized medication particles that are then inhaled into the user's lungs.

Embodiments of the multi-dose inhaler 900 may comprise an anti-microbial coating 100 on the at least a portion of the interior surface of the vessel wall 214 (e.g. the interior surfaces of the sidewall 215 and/or the bottom wall 216 of the canister), i.e. the surfaces that are in contact with the fluid 950 stored within the lumen 212.

Embodiments of the multi-dose inhaler 900 may comprise an anti-microbial coating 100 on at least a portion of the interior surfaces of the actuator 930, including for instance the interior surfaces of the mouthpiece 931 and/or the interior surfaces of the actuator body that are positioned between the nozzle 934 and the mouthpiece 931.

In use, the mouthpiece 931 of the multi-dose inhaler 900 is placed into the user or patient's mouth, and the actuator 930 is actuated one or more times in order to dispense the medical fluid into the user or patient's lungs. In use therefore the actuator 930, and in particular the mouthpiece 931, may come into contact with the inside of the user or patient's with bacteria, which can then enter into the interior of the actuator 930 and/or the lumen 212 of the vessel 210 in which the remainder of the medical fluid 950 is stored for future use, thereby contaminating the actuator and/or the medical fluid. Embodiments of the present invention comprise an anti-microbial coating 100 that is effective to inhibit the growth of microbes such as bacteria in the medical fluid 950 contained within the lumen 212 of the vessel 210 (e.g. as compared to a vessel without the anti-microbial coating) and/or to inactivate or kill bacteria introduced into the lumen of the canister, and/or to increase the shelf-life of the canister after first use. Further, embodiments of the present invention may comprise an anti-microbial coating 100 that is effective to inhibit the growth of microbes such as bacteria in the actuator 930 (e.g. as compared to an actuator without the anti-microbial coating) and/or to inactivate or kill bacteria introduced into the interior of the actuator, and/or to extend the life of the actuator after first use.

Embodiments of the multi-dose inhaler 900 may also comprise a canister 210 having a coating set 285 that includes a barrier coating or layer 288 and optionally one or more of a tie coating or layer 289 and a pH protective coating or layer 286. Like the anti-microbial coating 100 described above, this coating set 285 may be applied to at least a portion of the interior surface of the vessel wall 214, e.g. the interior surfaces of the sidewall 215 and/or the bottom wall 216 of the vessel. This coating set 285 may be provided in addition to the anti-microbial coating 100 or alone, i.e. independent of an anti-microbial coating. Generally, when applied in combination with an anti-microbial coating 100, the anti-microbial coating is applied as the innermost layer, i.e. the layer that is in contact with the fluid stored within the lumen 212.

### Gas Barrier Coatings

As described above, embodiments of the present disclosure may include a gas barrier coating, such as an oxygen barrier coating. In some embodiments, the gas barrier coating may comprise an oxygen gas barrier and/or be part of a coating set, such as the "trilayer" coating set described below.

### Vessels and Coating Sets

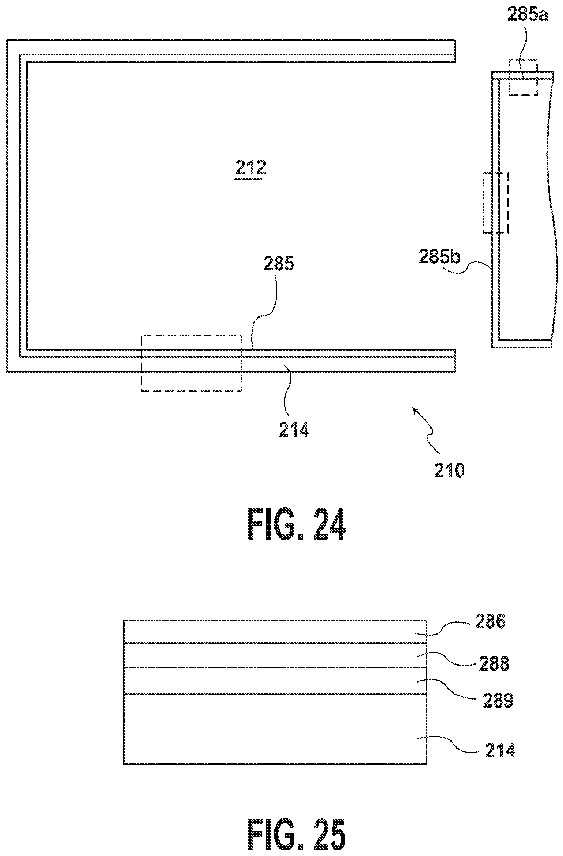

An aspect of the invention, illustrated most broadly by Fig. 24 and the detail view of Fig. 25, is a vessel 210 including a wall 214 enclosing a lumen 212 and a vessel coating or layer set 285 on at least a portion of the wall 214 facing the lumen 212. The vessel may be any of the multi-dose vessels described above or any other vessel configured to contain and/or containings a cosmetic or fragrance composition.

An embodiment of the vessel coating or layer set 285 is at least one tie coating or layer 289, at least one barrier coating or layer 288, and at least one pH protective coating or layer 286, illustrated in Figs. 24-25. This embodiment of the vessel coating or layer set is sometimes known as a "trilayer coating" in which the barrier coating or layer 288 of SiOₓ is protected against contents having a pH otherwise high enough to remove it by being sandwiched between the pH protective coating or layer 286 and the tie coating or layer 289, each an organic layer of SiOₓC_{y} as defined in this specification. A specific example of this trilayer coating is provided in this specification. The preferred contemplated thicknesses of the respective layers in nm (preferred ranges in parentheses) are given in the Trilayer Thickness Table.

| **Trilayer Thickness Table** | | |
|---|---|---|
| **Adhesion** | **Barrier** | **Protection** |
| 5-100 (5-20) if by PECVD | 20-200 (20-30) if by PECVD | 10-500 (100-200) |
| 1-20 (2-15) if by ALD | 1-20 (2-15) if by ALD | |

The trilayer coating set 285 includes as a first layer an adhesion or tie coating or layer 289 that improves adhesion of the barrier coating or layer to the substrate, i.e. vessel wall. The adhesion or tie coating or layer 289 is also believed to relieve stress on the barrier coating or layer 288, making the barrier layer less subject to damage from thermal expansion or contraction or mechanical shock. The adhesion or tie coating or layer 289 is also believed to decouple defects between the barrier coating or layer 288 and the substrate. This is believed to occur because any pinholes or other defects that may be formed when the adhesion or tie coating or layer 289 is applied tend not to be continued when the barrier coating or layer 288 is applied, so the pinholes or other defects in one coating do not line up with defects in the other. The adhesion or tie coating or layer 289 has some efficacy as a barrier layer, so even a defect providing a leakage path extending through the barrier coating or layer 289 is blocked by the adhesion or tie coating or layer 289.

The trilayer coating set 285 includes as a second layer a barrier coating or layer 288 that provides a barrier to oxygen that has permeated the vessel wall. The barrier coating or layer 288 also is a barrier to extraction of the composition of the barrel wall 214 by the contents of the lumen 214.

The trilayer coating set 285 includes as a third layer a pH protective coating or layer 286 that provides protection of the underlying barrier coating or layer 288 against contents of the vessel having a pH from 4 to 8. For a vessel wall that is in contact with the contents of the vessel from the time the package is manufactured to the time it is used, the pH protective coating or layer 286 prevents or inhibits attack of the barrier coating or layer 288 sufficiently to maintain an effective oxygen barrier over the intended pre-use shelf life of the package.

### Tie Coating or Layer

The tie coating or layer 289 has at least two functions. One function of the tie coating or layer 289 is to improve adhesion of a barrier coating or layer 288 to a substrate, in particular a thermoplastic substrate, although a tie layer can be used to improve adhesion to a glass substrate or to another coating or layer. For example, a tie coating or layer, also referred to as an adhesion layer or coating can be applied to the substrate and the barrier layer can be applied to the adhesion layer to improve adhesion of the barrier layer or coating to the substrate.

Another function of the tie coating or layer 289 has been discovered: a tie coating or layer 289 applied under a barrier coating or layer 288 can improve the function of a pH protective coating or layer 286 applied over the barrier coating or layer 288.

The tie coating or layer 289 can be composed of, comprise, or consist essentially of SiOₓC_{y}, in which x is between 0.5 and 2.4 and y is between 0.6 and 3. Alternatively, the atomic ratio can be expressed as the formula Si_{w}OₓC_{y}, The atomic ratios of Si, O, and C in the tie coating or layer 289 are, as several options:
Si 100 : O 50-150 : C 90-200 (i.e. w = 1, x = 0.5 to 1.5, y = 0.9 to 2);
Si 100 : O 70-130 : C 90-200 (i.e. w = 1, x = 0.7 to 1.3, y = 0.9 to 2)
Si 100: O 80-120 : C 90-150 (i.e. w = 1, x = 0.8 to 1.2, y = 0.9 to 1.5)
Si 100: O 90-120 : C 90-140 (i.e. w = 1, x = 0.9 to 1.2, y = 0.9 to 1.4), or
Si 100 : O 92-107 : C 116-133 (i.e. w = 1, x = 0.92 to 1.07, y = 1.16 to 1.33)

The atomic ratio can be determined by XPS. Taking into account the H atoms, which are not measured by XPS, the tie coating or layer 289 may thus in one aspect have the formula Si_{w}OₓC_{y}H_{z} (or its equivalent SiOₓC_{y}), for example where w is 1, x is from about 0.5 to about 2.4, y is from about 0.6 to about 3, and z is from about 2 to about 9. Typically, tie coating or layer 289 would hence contain 36% to 41% carbon normalized to 100% carbon plus oxygen plus silicon.

Optionally, the tie coating or layer can be similar or identical in composition with the pH protective coating or layer 286 described elsewhere in this specification, although this is not a requirement.

The tie coating or layer 289 is contemplated in any embodiment generally to be from 5 nm to 100 nm thick, preferably from 5 to 20 nm thick, particularly if applied by chemical vapor deposition. These thicknesses are not critical. Commonly but not necessarily, the tie coating or layer 289 will be relatively thin, since its function is to change the surface properties of the substrate.

### Barrier Layer

A barrier coating or layer 288 optionally can be deposited by plasma enhanced chemical vapor deposition (PECVD) or other chemical vapor deposition processes on the vessel wall, in particular a thermoplastic vessel wall, to prevent oxygen, carbon dioxide, or other gases from entering the vessel and/or to prevent leaching of the content of the vessel into or through the package wall.

The barrier coating or layer for any embodiment defined in this specification (unless otherwise specified in a particular instance) is a coating or layer, optionally applied by PECVD as indicated in U.S. Pat. No. 7,985,188. The barrier layer optionally is characterized as an "SiOₓ" coating, and contains silicon, oxygen, and optionally other elements, in which x, the ratio of oxygen to silicon atoms, is from about 1.5 to about 2.9, or 1.5 to about 2.6, or about 2. These alternative definitions of x apply to any use of the term SiOₓ in this specification.

The barrier coating 288 comprises or consists essentially of SiOx, wherein x is from 1.5 to 2.9, from 2 to 1000 nm thick, the barrier coating 288 of SiOx having an interior surface 220 facing the lumen 212 and an outer surface 222 facing the wall 214 article surface 254, the barrier coating 288 being effective to reduce the ingress of atmospheric gas into the lumen 212 compared to an uncoated vessel 250. One suitable barrier composition is one where x is 2.3, for example.

For example, the barrier coating or layer such as 288 of any embodiment can be applied at a thickness of at least 2 nm, or at least 4 nm, or at least 7 nm, or at least 10 nm, or at least 20 nm, or at least 30 nm, or at least 40 nm, or at least 50 nm, or at least 100 nm, or at least 150 nm, or at least 200 nm, or at least 300 nm, or at least 400 nm, or at least 500 nm, or at least 600 nm, or at least 700 nm, or at least 800 nm, or at least 900 nm. The barrier coating or layer can be up to 1000 nm, or at most 900 nm, or at most 800 nm, or at most 700 nm, or at most 600 nm, or at most 500 nm, or at most 400 nm, or at most 300 nm, or at most 200 nm, or at most 100 nm, or at most 90 nm, or at most 80 nm, or at most 70 nm, or at most 60 nm, or at most 50 nm, or at most 40 nm, or at most 30 nm, or at most 20 nm, or at most 10 nm, or at most 5 nm thick. Ranges of 20-200 nm, optionally 20-30 nm, are contemplated. Specific thickness ranges composed of any one of the minimum thicknesses expressed above, plus any equal or greater one of the maximum thicknesses expressed above, are expressly contemplated.

The thickness of the SiOₓ or other barrier coating or layer can be measured, for example, by transmission electron microscopy (TEM), and its composition can be measured by X-ray photoelectron spectroscopy (XPS).

A barrier coating or layer 286 of SiOₓ, in which x is between 1.5 and 2.9, is applied by plasma enhanced chemical vapor deposition (PECVD) directly or indirectly to the thermoplastic wall 214 (for example a tie coating or layer 289 can be interposed between them) so that in the filled vessel 210 the barrier coating or layer 286 is located between the inner or interior surface 220 of the thermoplastic wall 214 and the fluid 218.

The barrier coating or layer 286 of SiOₓ is supported by the thermoplastic wall 214. The barrier coating or layer 286 as described elsewhere in this specification, or in U.S. Patent No. 7,985,188, can be used in any embodiment.

Certain barrier coatings or layers 286 such as SiOₓ as defined here have been found to have the characteristic of being subject to being measurably diminished in barrier improvement factor in less than six months as a result of attack by certain relatively high pH contents of the coated vessel as described elsewhere in this specification, particularly where the barrier coating or layer directly contacts the contents. This issue can be addressed using a pH protective coating or layer as discussed in this specification.

The barrier coating or layer 286 of SiOₓ also can function as a primer coating or layer 283, as discussed elsewhere in this specification.

### pH Protective Coating or Layer

Barrier layers or coatings of SiOₓ are eroded or dissolved by some fluids, for example aqueous compositions having a pH above about 5. Since coatings applied by chemical vapor deposition can be very thin - tens to hundreds of nanometers thick - even a relatively slow rate of erosion can remove or reduce the effectiveness of the barrier layer in less time than the desired pre-use shelf life of a product package. This is particularly a problem for multi-use, cosmetic, and/or fragrance compositions having a pH of roughly 7, or more broadly in the range of 5 to 9. The higher the pH of the composition, the more quickly it erodes or dissolves the SiOₓ coating. Optionally, this problem can be addressed by protecting the barrier coating or layer 288, or other pH sensitive material, with a pH protective coating or layer 286.

Optionally, the pH protective coating or layer 286 can be composed of, comprise, or consist essentially of Si_{w}OₓC_{y}H_{z} (or its equivalent SiOₓC_{y}) or Si_{w}NₓC_{y}H_{z} or its equivalent Si(NH)ₓC_{y}), each as defined previously. The atomic ratio of Si : O : C or Si : N : C can be determined by XPS (X-ray photoelectron spectroscopy). Taking into account the H atoms, the pH protective coating or layer may thus in one aspect have the formula Si_{w}OₓC_{y}H_{z}, or its equivalent SiOₓC_{y}, for example where w is 1, x is from about 0.5 to about 2.4, y is from about to about 3, and z is from about 2 to about 9.

Typically, expressed as the formula Si_{w}OₓC_{y}, the atomic ratios of Si, O, and C are, as several options:
- Si 100 : O 50-150 : C 90-200 (i.e. w = 1, x = 0.5 to 1.5, y = 0.9 to 2);
- Si 100: O 70-130 : C 90-200 (i.e. w = 1, x = 0.7 to 1.3, y = 0.9 to 2);
- Si 100 : O 80-120 : C 90-150 (i.e. w = 1, x = 0.8 to 1.2, y = 0.9 to 1.5);
- Si 100: O 90-120 : C 90-140 (i.e. w = 1, x = 0.9 to 1.2, y = 0.9 to 1.4);
- Si 100 : O 92-107 : C 116-133 (i.e. w = 1, x = 0.92 to 1.07, y = 1.16 to 1.33);
- Si 100 : O 80-130 : C 90-150.

Alternatively, the pH protective coating or layer can have atomic concentrations normalized to 100% carbon, oxygen, and silicon, as determined by X-ray photoelectron spectroscopy (XPS) of less than 50% carbon and more than 25% silicon. Alternatively, the atomic concentrations are from 25 to 45% carbon, 25 to 65% silicon, and 10 to 35% oxygen. Alternatively, the atomic concentrations are from 30 to 40% carbon, 32 to 52% silicon, and 20 to 27% oxygen. Alternatively, the atomic concentrations are from 33 to 37% carbon, 37 to 47% silicon, and 22 to 26% oxygen.

The thickness of the pH protective coating or layer can be, for example:
- from 10 nm to 1000 nm;
- alternatively from 10 nm to 1000 nm;
- alternatively from 10 nm to 900 nm;
- alternatively from 10 nm to 800 nm;
- alternatively from 10 nm to 700 nm;
- alternatively from 10 nm to 600 nm;
- alternatively from 10 nm to 500 nm;
- alternatively from 10 nm to 400 nm;
- alternatively from 10 nm to 300 nm;
- alternatively from 10 nm to 200 nm;
- alternatively from 10 nm to 100 nm;
- alternatively from 10 nm to 50 nm;
- alternatively from 20 nm to 1000 nm;
- alternatively from 50 nm to 1000 nm;
- alternatively from 10 nm to 1000 nm;
- alternatively from 50 nm to 800 nm;
- alternatively from 100 nm to 700 nm;
- alternatively from 300 to 600 nm.

Optionally, the atomic concentration of carbon in the protective layer, normalized to 100% of carbon, oxygen, and silicon, as determined by X-ray photoelectron spectroscopy (XPS), can be greater than the atomic concentration of carbon in the atomic formula for the organosilicon precursor. For example, embodiments are contemplated in which the atomic concentration of carbon increases by from 1 to 80 atomic percent, alternatively from 10 to 70 atomic percent, alternatively from 20 to 60 atomic percent, alternatively from 30 to 50 atomic percent, alternatively from 35 to 45 atomic percent, alternatively from 37 to 41 atomic percent.

Optionally, the atomic ratio of carbon to oxygen in the pH protective coating or layer can be increased in comparison to the organosilicon precursor, and/or the atomic ratio of oxygen to silicon can be decreased in comparison to the organosilicon precursor.

Optionally, the pH protective coating or layer can have an atomic concentration of silicon, normalized to 100% of carbon, oxygen, and silicon, as determined by X-ray photoelectron spectroscopy (XPS), less than the atomic concentration of silicon in the atomic formula for the feed gas. For example, embodiments are contemplated in which the atomic concentration of silicon decreases by from 1 to 80 atomic percent, alternatively by from 10 to 70 atomic percent, alternatively by from 20 to 60 atomic percent, alternatively by from 30 to 55 atomic percent, alternatively by from 40 to 50 atomic percent, alternatively by from 42 to 46 atomic percent.

As another option, a pH protective coating or layer is contemplated in any embodiment that can be characterized by a sum formula wherein the atomic ratio C : O can be increased and/or the atomic ratio Si : O can be decreased in comparison to the sum formula of the organosilicon precursor.

The pH protective coating or layer 286 commonly is located between the barrier coating or layer 288 and the fluid 218 in the finished article. The pH protective coating or layer 286 is supported by the thermoplastic wall 214.

The pH protective coating or layer 286 optionally is effective to keep the barrier coating or layer 288 at least substantially undissolved as a result of attack by the fluid 218 for a period of at least six months.

The pH protective coating or layer can have a density between 1.25 and 1.65 g/cm³, alternatively between 1.35 and 1.55 g/cm³, alternatively between 1.4 and 1.5 g/cm³, alternatively between 1.4 and 1.5 g/cm³, alternatively between 1.44 and 1.48 g/cm³, as determined by X-ray reflectivity (XRR).

The pH protective coating or layer optionally can prevent or reduce the precipitation of a compound or component of a composition in contact with the pH protective coating or layer in comparison to the uncoated surface.

The pH protective coating or layer optionally can have an RMS surface roughness value (measured by AFM) of from about 5 to about 9, optionally from about 6 to about 8, optionally from about 6.4 to about 7.8. The Rₐ surface roughness value of the pH protective coating or layer, measured by AFM, can be from about 4 to about 6, optionally from about 4.6 to about 5.8. The Rₘₐₓ surface roughness value of the pH protective coating or layer, measured by AFM, can be from about 70 to about 160, optionally from about 84 to about 142, optionally from about 90 to about 130.

The interior surface of the pH protective optionally can have a contact angle (with distilled water) of from 90° to 110°, optionally from 80°to 120°, optionally from 70° to 130°, as measured by Goniometer Angle measurement of a water droplet on the pH protective surface, per ASTM D7334 - 08 "Standard Practice for Surface Wettability of Coatings, Substrates and Pigments by Advancing Contact Angle Measurement."

The passivation layer or pH protective coating or layer 286 optionally shows an O-Parameter measured with attenuated total reflection (ATR) of less than 0.4, measured as: $O - Parameter = \frac{Intensity at 1253 cm - 1}{Maximum intensity in the range 1000 to 1100 cm - 1}$

The O-Parameter is defined in U.S. Patent No. 8,067,070, which claims an O-parameter value of most broadly from 0.4 to 0.9. It can be measured from physical analysis of an FTIR amplitude versus wave number plot to find the numerator and denominator of the above expression, as shown in FIG. 6, which is the same as FIG. 5 of U.S. Patent No. 8,067,070, except annotated to show interpolation of the wave number and absorbance scales to arrive at an absorbance at 1253 cm-1 of .0424 and a maximum absorbance at 1000 to 1100 cm-1 of 0.08, resulting in a calculated O-parameter of 0.53. The O-Parameter can also be measured from digital wave number versus absorbance data.

U.S. Patent No. 8,067,070 asserts that the claimed O-parameter range provides a superior pH protective coating or layer, relying on experiments only with HMDSO and HMDSN, which are both non-cyclic siloxanes. Surprisingly, it has been found that if the PECVD precursor is a cyclic siloxane, for example OMCTS, O-parameters outside the ranges claimed in U.S. Patent No. 8,067,070 provide even better results than are obtained in U.S. Patent No. 8,067,070 with HMDSO.

Alternatively in some embodiments, the O-parameter has a value of from 0.1 to 0.39, or from 0.15 to 0.37, or from 0.17 to 0.35.

The passivation layer or pH protective coating or layer 286 optionally shows an N-Parameter measured with attenuated total reflection (ATR) of less than 0.7, measured as: $N - Parameter = \frac{Intensity at 840 cm - 1}{Intensity at 799 cm - 1} .$

The N-Parameter is also described in U.S. Patent No. 8,067,070, and is measured analogously to the O-Parameter except that intensities at two specific wave numbers are used - neither of these wave numbers is a range. U.S. Patent No. 8,067,070 claims a passivation layer with an N-Parameter of 0.7 to 1.6. Again, it has been determined that one may produce better coatings employing a pH protective coating or layer 286 having an N-Parameter lower than 0.7, as described above. Alternatively, the N-parameter has a value of at least 0.3, or from 0.4 to 0.6, or at least 0.53.

The rate of erosion, dissolution, or leaching (different names for related concepts) of the pH protective coating or layer 286, if directly contacted by the fluid 218, is less than the rate of erosion of the barrier coating or layer 288, if directly contacted by the fluid 218.

The thickness of the pH protective coating or layer is contemplated in any embodiment to be from 50-500 nm, with a preferred range of 100-200 nm.

The pH protective coating or layer 286 is effective to isolate the fluid 218 from the barrier coating or layer 288, at least for sufficient time to allow the barrier coating to act as a barrier during the pre-opening shelf life of the vessel 210.

It has also been found that certain pH protective coatings or layers of SiOₓC_{y} or Si(NH)ₓC_{y} formed from polysiloxane precursors, which pH protective coatings or layers have a substantial organic component, do not erode quickly when exposed to fluids, and in fact erode or dissolve more slowly when the fluids have higher pHs within the range of 5 to 9. For example, at pH 8, the dissolution rate of a pH protective coating or layer made from the precursor octamethylcyclotetrasiloxane, or OMCTS, is quite slow. These pH protective coatings or layers of SiOₓC_{y} or Si(NH)ₓC_{y} can therefore be used to cover a barrier layer of SiOₓ, retaining the benefits of the barrier layer by protecting it from the fluid in the multi-use, cosmetic, and/or fragrance product package. The protective layer is applied over at least a portion of the SiOₓ layer to protect the SiOₓ layer from contents stored in a vessel, where the contents otherwise would be in contact with the SiOₓ layer.

Although the present invention does not depend upon the accuracy of the following theory, it is further believed that effective pH protective coatings or layers for avoiding erosion can be made from siloxanes and silazanes as described in this disclosure. SiOₓC_{y} or Si(NH)ₓC_{y} coatings deposited from cyclic siloxane or linear silazane precursors, for example octamethylcyclotetrasiloxane (OMCTS), are believed to include intact cyclic siloxane rings and longer series of repeating units of the precursor structure. These coatings are believed to be nanoporous but structured and hydrophobic, and these properties are believed to contribute to their success as pH protective coatings or layers, and also protective coatings or layers. This is shown, for example, in U.S. Pat. No. 7,901,783.

SiOₓC_{y} or Si(NH)ₓC_{y} coatings also can be deposited from linear siloxane or linear silazane precursors, for example hexamethyldisiloxane (HMDSO) or tetramethyldisiloxane (TMDSO).

Optionally an FTIR absorbance spectrum of the pH protective coating or layer 286 of any embodiment has a ratio greater than 0.75 between the maximum amplitude of the Si- O-Si symmetrical stretch peak normally located between about 1000 and 1040 cm-1, and the maximum amplitude of the Si-O-Si assymmetric stretch peak normally located between about 1060 and about 1100 cm-1. Alternatively, in any embodiment, this ratio can be at least 0.8, or at least 0.9, or at least 1.0, or at least 1.1, or at least 1.2. Alternatively, in any embodiment, this ratio can be at most 1.7, or at most 1.6, or at most 1.5, or at most 1.4, or at most 1.3. Any minimum ratio stated here can be combined with any maximum ratio stated here.

Optionally, in any embodiment the pH protective coating or layer 286, in the absence of the medicament, has a non-oily appearance. This appearance has been observed in some instances to distinguish an effective pH protective coating or layer from a lubricity layer, which in some instances has been observed to have an oily (i.e. shiny) appearance.

Optionally, for the pH protective coating or layer 286 in any embodiment, the silicon dissolution rate by a 50 mM potassium phosphate buffer diluted in water for injection, adjusted to pH 8 with concentrated nitric acid, and containing 0.2 wt. % polysorbate-80 surfactant, (measured in the absence of the medicament, to avoid changing the dissolution reagent), at 40°C, is less than 170 ppb/day. (Polysorbate-80 is a common ingredient of pharmaceutical preparations, available for example as Tween^{®}-80 from Uniqema Americas LLC, Wilmington Delaware.)

Optionally, for the pH protective coating or layer 286 in any embodiment, the silicon dissolution rate is less than 160 ppb/day, or less than 140 ppb/day, or less than 120 ppb/day, or less than 100 ppb/day, or less than 90 ppb/day, or less than 80 ppb/day. Optionally, in any embodiment of Figures 24-26 the silicon dissolution rate is more than 10 ppb/day, or more than 20 ppb/day, or more than 30 ppb/day, or more than 40 ppb/day, or more than 50 ppb/day, or more than 60 ppb/day. Any minimum rate stated here can be combined with any maximum rate stated here for the pH protective coating or layer 286 in any embodiment.

Optionally, for the pH protective coating or layer 286 in any embodiment the total silicon content of the pH protective coating or layer and barrier coating, upon dissolution into a test composition with a pH of 8 from the vessel, is less than 66 ppm, or less than 60 ppm, or less than 50 ppm, or less than 40 ppm, or less than 30 ppm, or less than 20 ppm.

The protective coating or layer of Si_{w}OₓC_{y} or its equivalent SiOₓC_{y} also can have utility as a hydrophobic layer, independent of whether it also functions as a pH protective coating or layer. Suitable hydrophobic coatings or layers and their application, properties, and use are described in U.S. Patent No. 7,985,188. Dual functional protective / hydrophobic coatings or layers having the properties of both types of coatings or layers can be provided for in any embodiment of the present invention.

An embodiment can be carried out under conditions effective to form a hydrophobic pH protective coating or layer on the substrate. Optionally, the hydrophobic characteristics of the pH protective coating or layer can be set by setting the ratio of the O2 to the organosilicon precursor in the gaseous reactant, and/or by setting the electric power used for generating the plasma. Optionally, the pH protective coating or layer can have a lower wetting tension than the uncoated surface, optionally a wetting tension of from 20 to 72 dyne/cm, optionally from 30 to 60 dynes/cm, optionally from 30 to 40 dynes/cm, optionally 34 dyne/cm. Optionally, the pH protective coating or layer can be more hydrophobic than the uncoated surface.

### Atomic Layer Deposition Coating of Vessels

One or more of the layers described herein may be applied by atomic layer deposition coating. Coatings applied by atomic layer deposition are structurally (though not necessarily chemically) distinct from those applied by CVD or PECVD. In contrast to coatings applied by CVD or PECVD, coatings applied by atomic layer deposition consist of a plurality of monolayers of the deposited compound. Because each step deposit only a single monolayer, defects of the sort that can develop due to non-uniform growth during CVD or PECVD are avoided. The result is a coating having significantly higher density than that of a coating (of generally the same chemical composition) applied by CVD or PECVD. Because the coating consists of a plurality of monolayers of the deposited compound, the coating may also have a higher degree of compositional purity and consistency than coatings applied by PECVD.

In an atomic layer deposition process, sources, i.e., precursors, may be sequentially introduced in non-overlapping timeframes to deposit one material at a time. Once each possible adsorption site is occupied in a particular precursor flow, the precursor may be halted and a purge process may be completed before the next source material is introduced, with one timeframe for each precursor comprising one cycle. As the chamber is typically under a 1-20 mbar vacuum, the remaining precursor may be evacuated upon stopping flow. In this manner, the deposition process continues in a self-limited way in that there are only a finite number of sites on which the reactant can adsorb, so once they are filled, the growth stops until the next precursor is introduced, where the total material thickness is controlled by the number of cycles. This process may continue for each precursor, resulting in a coating or layer being deposited one atomic layer at a time. Accordingly, ALD is capable of growing very thin conformal films with excellent thickness uniformity and control, as well as increased density compared to other deposition techniques. Furthermore, precise composition control is enabled by the ALD process.

A plasma may be optionally utilized to enhance the material deposition, i.e., plasma enhanced atomic layer deposition (PEALD), also sometimes referred to as plasma-assisted atomic layer deposition, where the precursor dissociation may be increased using a plasma, enabling a lower growth temperature, which may be useful when applying coatings to certain thermoplastics.

ALD is useful for depositing high-density layers with low defect density. In an example, a thin SiOx film may be deposited by thermal and/or plasma enhanced ALD. The deposition temperature may be in the range of 30°C to 120°C. For instance, where thermal ALD is used, the deposition temperature may desirably be in the range of 80-120°C. Where PEALD is used, the temperature may be at least 30°C, e.g. between 30 °C and 80 °C or between 30 °C and 60 °C.

Precursors for the deposition of a SiOx film by ALD or PEALD include one or more silicon-containng precursor and one or more oxygen precusors. The silicon precursors may include, for example, aminosilanes; alkyl-aminosilanes, such as tetradimethyl-aminosilicon; 1,2-bis(diisopropylamino)disilane (for low temperature deposition, e.g. 50-60° C); diisopropylaminosilane; tris(dimethylamino)silane; bis(ethyl-methyl-amino)silane; and combinations thereof. Ozone may be used as an oxygen precursor in thermal ALD and O₂ plasma may be utilized with PEALD. Further, the silicon precursor (or precursors) may be pulsed to control the growth rate.

In another example, ALD and/or PEALD may be utilized to deposit other barrier layer materials such as silicon nitrides, silicon carbides, and aluminum oxides, or other such materials which may improve the gas barrier and/or material dissociation capabilities. Due to the slow and controlled growth rate of ALD, which may result in increased material adhesion, tie layers may not be needed.

### EXAMPLES

Although the examples provided below predominantly involve the coating of thermoplastic syringes and vials, which are not the subject of the present application, it is believed that a person of ordinary skill in the art can use the teaching of this specification to adjust the coating parameters as necessary in order to provide desired coating sets for the multi-use, cosmetic, and/or fragrance packages described herein.

### Examples 1-4 - Conditions for Production of pH Protective Layer

Some conditions used for production of pH Protective Layers are shown in Table 1.

**TABLE 1: OMCTS-BASED PLASMA pH PROTECTIVE COATING OR LAYER MADE WITH CARRIER GAS**

| **Example** | **pH protectiv e coating or layer Type** | **PH protective Monomer** | **pH protectiv e coating or layer Time (sec)** | **protective OMCTS Flow Rate (sccm)** | **protective O2 Flow Rate (sccm)** | **Carrier Gas (Ar) Flow Rate (sccm)** | **pH protectiv e coating or layer Power (Watts)** |
|---|---|---|---|---|---|---|---|
| 1 (Control) | Uncoated COC | n/a | n/a | n/a | n/a | n/a | n/a |
| 2 (Industry Standard) | Silicon oil on COC | n/a | n/a | n/a | n/a | n/a | n/a |
| 3 (without Oxygen) | L3 lubricity coating or layer over SiOₓ on COC | OMCTS | 10 sec | 3 | 0 | 65 | 6 |
| 4 (with Oxygen) | L2 pH protective coating or layer over SiOₓ on COC | OMCTS | 10 sec | 3 | 1 | 65 | 6 |

### Examples 5-8

Syringe samples were produced as follows. A COC 8007 extended barrel syringe was produced according to the Protocol for Forming COC Syringe Barrel. An SiOₓ barrier coating or layer was applied to the syringe barrels according to the Protocol for Coating COC Syringe Barrel Interior with SiOₓ. A pH protective coating or layer was applied to the SiOₓ coated syringes according to the Protocol for Coating COC Syringe Barrel Interior with OMCTS, modified as follows. Argon carrier gas and oxygen were used where noted in Table 2. The process conditions were set to the following, or as indicated in Table 2:
- OMCTS -3 sccm (when used)
- Argon gas -7.8 sccm (when used)
- Oxygen 0.38 sccm (when used)
- Power - 3 watts
- Power on time - 10 seconds
Syringes of Examples 5, 6, and 7 were tested to determine total extractable silicon levels (representing extraction of the organosilicon-based PECVD pH protective coating or layer) using the Protocol for Measuring Dissolved Silicon in a Vessel, modified and supplemented as shown in this example.

The silicon was extracted using saline water digestion. The tip of each syringe plunger was covered with PTFE tape to prevent extracting material from the elastomeric tip material, then inserted into the syringe barrel base. The syringe barrel was filled with two milliliters of 0.9% aqueous saline solution via a hypodermic needle inserted through the Luer tip of the syringe. This is an appropriate test for extractables because many prefilled syringes are used to contain and deliver saline solution. The Luer tip was plugged with a piece of PTFE beading of appropriate diameter. The syringe was set into a PTFE test stand with the Luer tip facing up and placed in an oven at 50°C for 72 hours.

Then, either a static or a dynamic mode was used to remove the saline solution from the syringe barrel. According to the static mode indicated in Table 2, the syringe plunger was removed from the test stand, and the fluid in the syringe was decanted into a vessel. According to the dynamic mode indicated in Table 2, the Luer tip seal was removed and the plunger was depressed to push fluid through the syringe barrel and expel the contents into a vessel. In either case, the fluid obtained from each syringe barrel was brought to a volume of 50ml using 18.2MΩ-cm deionized water and further diluted 2x to minimize sodium background during analysis. The CVH barrels contained two milliliters and the commercial barrels contained 2.32 milliliters.

Next, the fluid recovered from each syringe was tested for extractable silicon using the Protocol for Measuring Dissolved Silicon in a Vessel. The instrument used was a Perkin Elmer Elan DRC II equipped with a Cetac ASX-520 autosampler. The following ICP-MS conditions were employed:
- Nebulizer: Quartz Meinhardt
- Spray Chamber: Cyclonic
- RF (radio frequency) power: 1550 Watts
- Argon (Ar) Flow: 15.0 L/min
- Auxiliary Ar Flow: 1.2 L/min
- Nebulizer Gas Flow: 0.88 L/min
- Integration time: 80 sec
- Scanning mode: Peak hopping
- RPq (The RPq is a rejection parameter) for Cerium as CeO (m/z 156: < 2 %)

Aliquots from aqueous dilutions obtained from Syringes E, F, and G were injected and analyzed for Si in concentration units of micrograms per liter. The results of this test are shown in Table 2. While the results are not quantitative, they do indicate that extractables from the pH protective coating or layer are not clearly higher than the extractables for the SiOₓ barrier layer only. Also, the static mode produced far less extractables than the dynamic mode, which was expected.

**TABLE 2: OMCTS PH PROTECTIVE COATING OR LAYER (E and F)**

| **Example** | **OMCTS (sccm)** | **O₂ (sccm)** | **Ar (sccm)** |
|---|---|---|---|
| 5 | 3.0 | 0.38 | 7.8 |
| 6 | 3.0 | 0.38 | 7.8 |
| 7 (SiOₓonly) | n/a | n/a | n/a |
| 8 (silicon oil) | n/a | n/a | n/a |

### Examples 9-11

Syringe Examples 9, 10, and 11, employing three different pH protective coatings or layers, were produced in the same manner as for Examples 5-8 except as follows or as indicated in Table 3:
- OMCTS -2.5 sccm
- Argon gas -7.6 sccm (when used)
- Oxygen 0.38 sccm (when used)
- Power - 3 watts
- Power on time - 10 seconds

Syringe Example 9 had a three-component pH protective coating or layer employing OMCTS, oxygen, and carrier gas. Syringe Example 10 had a two component pH protective coating or layer employing OMCTS and oxygen, but no carrier gas. Syringe Example 11 had a one-component pH protective coating or layer (OMCTS only). Syringes of Examples 9-11 were then tested for lubricity as described for Examples 5-8.

The pH protective coatings or layers produced according to these working examples are also contemplated to function as protective coatings or layers to increase the shelf life of the vessels, compared to similar vessels provided with a barrier coating or layer but no pH protective coating or layer.

### TABLE 3: OMCTS pH protective coating or layer

- OMCTS -2.5 sccm
- Argon gas -7.6 sccm (when used)
- Oxygen 0.38 sccm (when used)
- Power - 3 watts
- Power on time - 10 seconds

### Examples 12-14

Examples 9-11 using an OMCTS precursor gas were repeated in Examples 12-14, except that HMDSO was used as the precursor in Examples 12-14. The results are shown in Table 4. The coatings produced according to these working examples are contemplated to function as pH protective coatings or layers, and also as protective coatings or layers to increase the shelf life of the vessels, compared to similar vessels provided with a barrier coating or layer but no pH protective coating or layer.

**TABLE 4: HMDSO pH protective coating or layer**

| **Example** | **HMDSO(sccm)** | **O₂ (sccm)** | **Ar (sccm)** |
|---|---|---|---|
| 12 | 2.5 | 0.38 | 7.6 |
| 13 | 2.5 | 0.38 | - |
| 14 | 2.5 | - | - |

The pH protective coatings or layers produced according to these working examples are also contemplated to function as protective coatings or layers to increase the shelf life of the vessels, compared to similar vessels provided with a barrier coating or layer but no pH protective coating or layer.

**TABLE 5**

| **Example** | **OMCTS (sccm)** | **Ar/O2 (sccm)** | **Power (Watts)** | **Dep. Time (sec)** | | **AFM RMS (nanometers)** |
|---|---|---|---|---|---|---|
| **15** | 2.0 | 10/0.38 | 3.5 | 10 | | |
| **16** | | | | | | |
| **17** | | | | | | 19.6, 9.9, 9.4 (Average=13.0) |
| | | | | | | |
| **21** | 2.0 | 10/0.38 | 4.5 | 10 | | |
| **22** | | | | | FIG. 7 | |
| **23** | | | | | | 12.5, 8.4, 6.1 (Average=6.3) |
| | | | | | | |
| **24** | 2.0 | 10/0 | 3.4 | 10 | | |
| **25** | | | | | | 1.9, 2.6, 3.0 (Average=2.3) |

**TABLE 6**

| | **SiOx/Lub** | **Coater** | **Mode** | **Siloxane Feed** | **Ar/O2** | **Power (W)** | **Dep. Time (Sec.)** |
|---|---|---|---|---|---|---|---|
| **Example 18 SiOₓ/Baseline OMCTS Lub** | **SiOₓ:** | Auto-Tube | Auto | HMDSO 52.5 in, 133.4 cm. | 0 sccm Ar, 90 sccm O₂ | 37 | 7 |
| | **Lubricity:** | Auto-S | Same | OMCTS, 2.0 sccm | 10 sccm Ar 0.38 sccm O₂ | 3,4 | 10 |
| | | | | | | | |
| **Example 19 SiOₓ/High Pwr OMCTS Lub** | **SiOₓ:** | Same | same | Same | Same | 37 | 7 |
| | **Lubricity:** | Same | Same | Same | Same | 4,5 | 10 |
| | | | | | | | |
| **Example 20 SiOₓ/No O₂ OMCTS Lub** | **SiOx:** | Auto-Tube | Same | Same | 0 sccm Ar, 90 sccm O2 | 37 | 7 |
| | **Lubricity:** | Auto-S | Same | Same | 10 sccm Ar 0 sccm O2 | 3,4 | 10 |

### Summary of Lubricity and/or Protective Measurements

Table 8 shows a summary of the above OMCTS coatings or layers

**TABLE 8: Summary Table of OMCTS PH PROTECTIVE COATING OR LAYER fromTables 1, 2, 3 and 5**

| **Example** | **OMCTS(sccm)** | **O2 (sccm)** | **Ar (sccm)** | **Power (Watt)** | **Dep Time (sec)** |
|---|---|---|---|---|---|
| **3** | 3.0 | 0.00 | 65 | 6 | 10 |
| **4** | 3.0 | 1.00 | 65 | 6 | 10 |
| **5** | 3.0 | 0.38 | 7.8 | 6 | 10 |
| **6** | 3.0 | 0.38 | 7.8 | 6 | 10 |
| **9** | 2.5 | 0.38 | 7.6 | 6 | 10 |
| **10** | 2.5 | 0.38 | 0.0 | 6 | 10 |
| **11** | 2.5 | 0.00 | 0.0 | 6 | 10 |
| **15** | 2.0 | 0.38 | 10 | 3.5 | 10 |
| **16** | 2.0 | 0.38 | 10 | 4.5 | 10 |
| **16A** | 2.0 | 0.00 | 10 | 3.4 | 10 |
| **18** | 2.0 | 0.38 | 10 | 3.4 | 10 |
| **19** | 2.0 | 0.38 | 10 | 4.5 | 10 |
| **20** | 2.0 | 0.00 | 10 | 3.4 | 10 |

### Comparative Example 26: Dissolution of SiOx Coating Versus pH

The Protocol for Measuring Dissolved Silicon in a Vessel is followed, except as modified here. Test solutions - 50 mM buffer solutions at pH 3, 6, 7, 8, 9, and 12 are prepared. Buffers are selected having appropriate pKa values to provide the pH values being studied. A potassium phosphate buffer is selected for pH 3, 7, 8 and 12, a sodium citrate buffer is utilized for pH 6 and tris buffer is selected for pH 9. 3 ml of each test solution is placed in borosilicate glass 5 ml pharmaceutical vials and SiOₓ coated 5 ml thermoplastic pharmaceutical vials. The vials are all closed with standard coated stoppers and crimped. The vials are placed in storage at 20 - 25°C and pulled at various time points for inductively coupled plasma spectrometer (ICP) analysis of Si content in the solutions contained in the vials, in parts per billion (ppb) by weight, for different storage times.

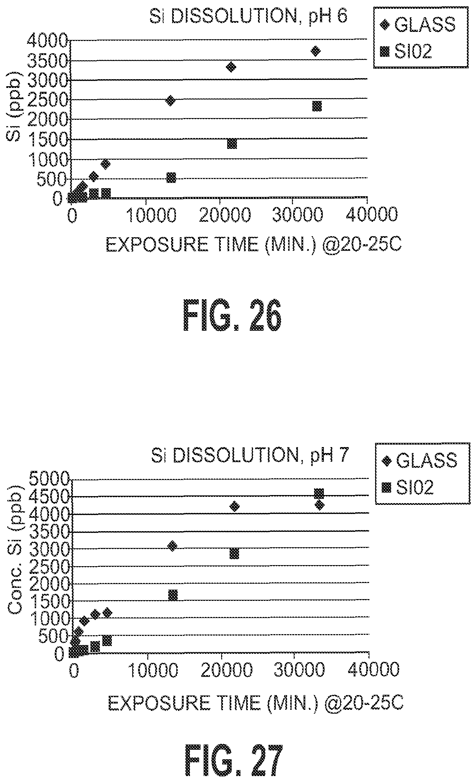

The Protocol for Determining Average Dissolution Rate Si content is used to monitor the rate of glass dissolution, except as modified here. The data is plotted to determine an average rate of dissolution of borosilicate glass or SiOₓ coating at each pH condition. Representative plots at pH 6 through 8 are FIGS 26-28.

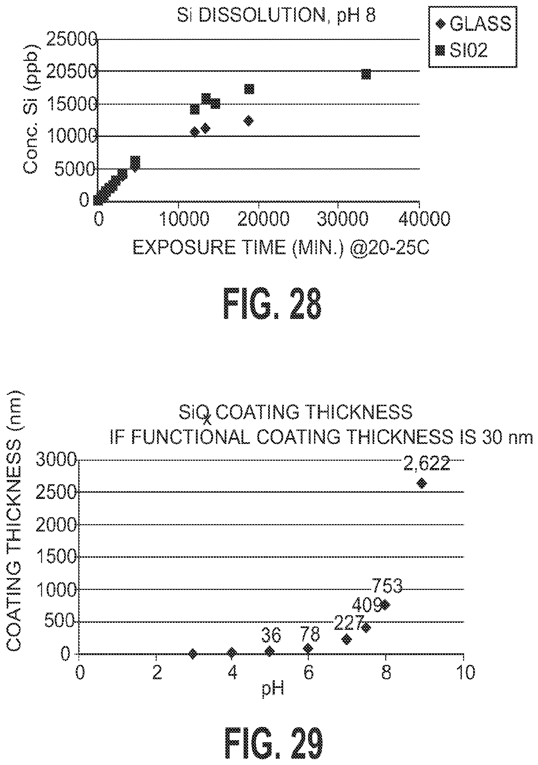

The rate of Si dissolution in ppb is converted to a predicted thickness (nm) rate of Si dissolution by determining the total weight of Si removed, then using a surface area calculation of the amount of vial surface (11.65 cm2) exposed to the solution and a density of SiOₓ of 2.2 g/cm3. FIG. 29 shows the predicted initial thickness of the SiOₓ coating required, based on the conditions and assumptions of this example (assuming a residual SiOₓ coating of at least 30 nm at the end of the desired shelf life of two years, and assuming storage at 20 to 25°C). As FIG. 29 shows, the predicted initial thickness of the coating is about 36 nm at pH 5, about 80 nm at pH 6, about 230 nm at pH 7, about 400 nm at pH 7.5, about 750 nm at pH 8, and about 2600 nm at pH 9.

The coating thicknesses in FIG. 29 represent atypically harsh case scenarios for pharma and biotech products. Most biotech products and many pharma products are stored at refrigerated conditions and none are typically recommended for storage above room temperature. As a general rule of thumb, storage at a lower temperature reduces the thickness required, all other conditions being equivalent.

The following conclusions are reached, based on this test. First, the amount of dissolved Si in the SiOₓ coating or glass increases exponentially with increasing pH. Second, the SiOₓ coating dissolves more slowly than borosilicate glass at a pH lower than 8. The SiOₓ coating shows a linear, monophasic dissolution over time, whereas borosilicate glass tends to show a more rapid dissolution in the early hours of exposure to solutions, followed by a slower linear dissolution. This may be due to surface accumulation of some salts and elements on borosilicate during the forming process relative to the uniform composition of the SiOₓ coating. This result incidentally suggests the utility of a SiOₓ coating on the wall of a borosilicate glass vial to reduce dissolution of the glass at a pH lower than 8. Third, PECVD applied barrier coatings for vials in which pharmaceutical preparations are stored will need to be adapted to the specific pharmaceutical preparation and proposed storage conditions (or vice versa), at least in some instances in which the pharmaceutical preparation interacts with the barrier coating significantly.

### Example 27

An experiment is conducted with vessels coated with SiOₓ coating + OMCTS pH protective coating or layer, to test the pH protective coating or layer for its functionality as a protective coating or layer. The vessels are 5 mL vials (the vials are normally filled with product to 5 mL; their capacity without headspace, when capped, is about 7.5 mL) composed of cyclic olefin co-polymer (COC, Topas^{®} 6013M-07).

Sixty vessels are coated on their interior surfaces with an SiOₓ coating produced in a plasma enhanced chemical vapor deposition (PECVD) process using a HMDSO precursor gas according to the Protocol for Coating Tube Interior with SiOₓ set forth above, except that equipment suitable for coating a vial is used. The following conditions are used.
- HMDSO flow rate: 0.47 sccm
- Oxygen flow rate: 7.5 sccm
- RF power: 70 Watts
- Coating time: 12 seconds (includes a 2-sec RF power ramp-up time)

Next the SiOₓ coated vials are coated over the SiOₓ with an SiOₓC_{y} coating produced in a PECVD process using an OMCTS precursor gas according to the Protocol for Coating COC Syringe Barrel Interior with OMCTS Lubricity Coating set forth above, except that the same coating equipment is used as for the SiOₓ coating. Thus, the special adaptations in the protocol for coating a syringe are not used. The following conditions are used.
- OMCTS flow rate: 2.5 sccm
- Argon flow rate: 10 sccm
- Oxygen flow rate: 0.7 sccm
- RF power: 3.4 Watts
- Coating time: 5 seconds

Eight vials are selected and the total deposited quantity of PECVD coating (SiOₓ + SiOₓC_{y}) is determined with a Perkin Elmer Optima Model 7300DV ICP-OES instrument, using the Protocol for Total Silicon Measurement set forth above. This measurement determines the total amount of silicon in both coatings, and does not distinguish between the respective SiOₓ and SiOₓC_{y} coatings. The results are shown below.

| Vial | Total Silicon ug/L |
|---|---|
| 1 | 13844 |
| 2 | 14878 |
| 3 | 14387 |
| 4 | 13731 |
| 5 | 15260 |
| 6 | 15017 |
| 7 | 15118 |
| 8 | 12736 |
| **Mean** | **14371** |
| **StdDev** | **877** |

### Quantity of SiOₓ + Lubricity layer on Vials

In the following work, except as indicated otherwise in this example, the Protocol for Determining Average Dissolution Rate is followed. Two buffered pH test solutions are used in the remainder of the experiment, respectively at pH 4 and pH 8 to test the effect of pH on dissolution rate. Both test solutions are 50 mM buffers using potassium phosphate as the buffer, diluted in water for injection (WFI) (0.1 um sterilized, filtered). The pH is adjusted to pH 4 or 8, respectively, with concentrated nitric acid.

25 vials are filled with 7.5 ml per vial of pH 4 buffered test solution and 25 other vials are filled with 7.5 ml per vial of pH 4 buffered test solution (note the fill level is to the top of the vial - no head space). The vials are closed using prewashed butyl stoppers and aluminum crimps. The vials at each pH are split into two groups. One group at each pH containing 12 vials is stored at 4°C and the second group of 13 vials is stored at 23°C

The vials are sampled at Days 1, 3, 6, and 8. The Protocol for Measuring Dissolved Silicon in a Vessel is used, except as otherwise indicated in this example.The analytical result is reported on the basis of parts per billion of silicon in the buffered test solutions of each vial. A dissolution rate is calculated in terms of parts per billion per day as described above in the Protocol for Determining Average Dissolution Rate. The results at the respective storage temperatures follow:

| | **Shelf Life Conditions 23º C** | |
|---|---|---|
| | **Vial SiOₓ + Lubricity Coating at pH4** | **Vial SiOₓ + Lubricity Coating at pH8** |
| Si Dissolution Rate(PPB/day) | 31 | 7 |

| | **Shelf Life Conditions 4º C** | |
|---|---|---|
| | **Vial SiOₓ + Lubricity Coating at pH4** | **Vial SiOₓ + Lubricity Coating at pH8** |
| Si Dissolution Rate (PPB/day) | 7 | 11 |

The observations of Si dissolution versus time for the OMCTS-based coating at pH 8 and pH 4 indicate the pH 4 rates are higher at ambient conditions. Thus, the pH 4 rates are used to determine how much material would need to be initially applied to leave a coating of adequate thickness at the end of the shelf life, taking account of the amount of the initial coating that would be dissolved. The results of this calculation are:

| | **Vial SiOₓ + Lubricity Coatingat pH4** |
|---|---|
| Si Dissolution Rate (PPB/day) | 31 |
| Mass of Coating Tested (Total Si) | 14,371 |
| Shelf Life (days) at 23º C | 464 |
| Shelf Life (years) at 23º C | 1.3 |
| Required Mass of Coating (Total Si) - 2 years | 22,630 |
| Required Mass of Coating (Total Si) - 3 years | 33,945 |

### Shelf Life Calculation

Based on this calculation, the OMCTS protective layer needs to be about 2.5 times thicker - resulting in dissolution of 33945 ppb versus the 14,371 ppb representing the entire mass of coating tested - to achieve a 3-year calculated shelf life.

### Example 28

The results of Comparative Example 26 and Example 27 above can be compared as follows, where the "pH protective coating or layer" is the coating of SiOₓC_{y} referred to in

### Example BB.

| | **Shelf Life Conditions - - pH8 and 23º C** | |
|---|---|---|
| | **Vial SiOₓ** | **Vial SiOₓ + Lubricity Coating** |
| Si Dissolution Rate (PPB/day) | 1,250 | 7 |

This data shows that the silicon dissolution rate of SiOₓ alone is reduced by more than 2 orders of magnitude at pH 8 in vials also coated with SiOₓC_{y} coatings.

### Example 29

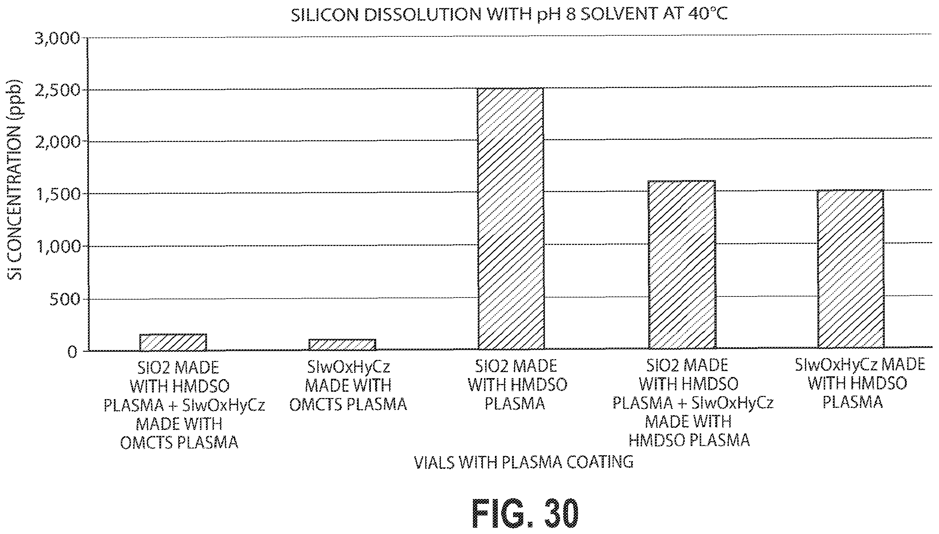

Another comparison is shown by the following data from several different experiments carried out under similar accelerated dissolution conditions, of which the 1- day data is also presented in FIG. 30.

| | **Silicon Dissolution with pH 8 at 40°C (ug/L)** | | | | | | |
|---|---|---|---|---|---|---|---|
| **Vial Coating Description** | **1day** | **2days** | **3days** | **4 days** | **7 days** | **10days** | **15 days** |
| A. SiOₓ made with HMDSO Plasma + Si_{w}OₓC_{y} or its equivalent SiOₓC_{y} made with OMCTS Plasma | 165 | 211 | 226 | 252 | 435 | 850 | 1,364 |
| B. SiwOxCy or its equivalent SiOxCy made with OMCTS Plasma | 109 | 107 | 76 | 69 | 74 | 158 | 198 |
| C. SiOx made with HMDSO Plasma | 2,504 | 4,228 | 5,226 | 5,650 | 9,292 | 10,177 | 9,551 |
| D. SiOx made with HMDSO Plasma + SiwOxCy or its equivalent SiOₓC_{y} made with HMDSO Plasma | 1,607 | 1,341 | 3,927 | 10,182 | 18,148 | 20,446 | 21,889 |
| E. Si_{w}OₓC_{y} or its equivalent SiOₓC_{y} made with HMDSO Plasma | 1,515 | 1,731 | 1,813 | 1,743 | 2,890 | 3,241 | 3,812 |

FIG. 30 and Row A (SiOₓ with OMCTS coating) versus C (SiOₓ without OMCTS coating) show that the OMCTS pH protective coating or layer is also an effective protective coating or layer to the SiOₓ coating at pH 8. The OMCTS coating reduced the one-day dissolution rate from 2504 ug/L ("u" or µ or the Greek letter "mu" as used herein are identical, and are abbreviations for "micro") to 165 ug/L. This data also shows that an HMDSO-based Si_{w}OₓC_{y} (or its equivalent SiOₓC_{y}) overcoat (Row D) provided a far higher dissolution rate than an OMCTS-based Si_{w}OₓC_{y} (or its equivalent SiOₓC_{y}) overcoat (Row A). This data shows that a substantial benefit can be obtained by using a cyclic precursor versus a linear one.

### Example 30

Samples 1-6 as listed in Table 9 were prepared as described in Example AA, with further details as follows.

A cyclic olefin copolymer (COC) resin was injection molded to form a batch of 5ml vials. Silicon chips were adhered with double-sided adhesive tape to the internal walls of the vials. The vials and chips were coated with a two-layer coating by plasma enhanced chemical vapor deposition (PECVD). The first layer was composed of SiOₓ with barrier properties as defined in the present disclosure, and the second layer was an SiOₓC_{y} pH protective coating or layer.

A precursor gas mixture comprising OMCTS, argon, and oxygen was introduced inside each vial. The gas inside the vial was excited between capacitively coupled electrodes by a radio-frequency (13.56 MHz) power source. The monomer flow rate (Fm) in units of sccm, oxygen flow rate (Fo) in units of sccm, argon flowrate in sccm, and power (W) in units of watts are shown in Table 9.

A composite parameter, W/FM in units of kJ/kg, was calculated from process parameters W, Fm, Fo and the molecular weight, M in g/mol, of the individual gas species. W/FM is defined as the energy input per unit mass of polymerizing gases. Polymerizing gases are defined as those species that are incorporated into the growing coating such as, but not limited to, the monomer and oxygen. Non-polymerizing gases, by contrast, are those species that are not incorporated into the growing coating, such as but not limited to argon, helium and neon.

In this test, PECVD processing at high W/FM is believed to have resulted in higher monomer fragmentation, producing organosiloxane coatings with higher cross- link density. PECVD processing at low W/FM, by comparison, is believed to have resulted in lower monomer fragmentation producing organosiloxane coatings with a relatively lower cross-link density.

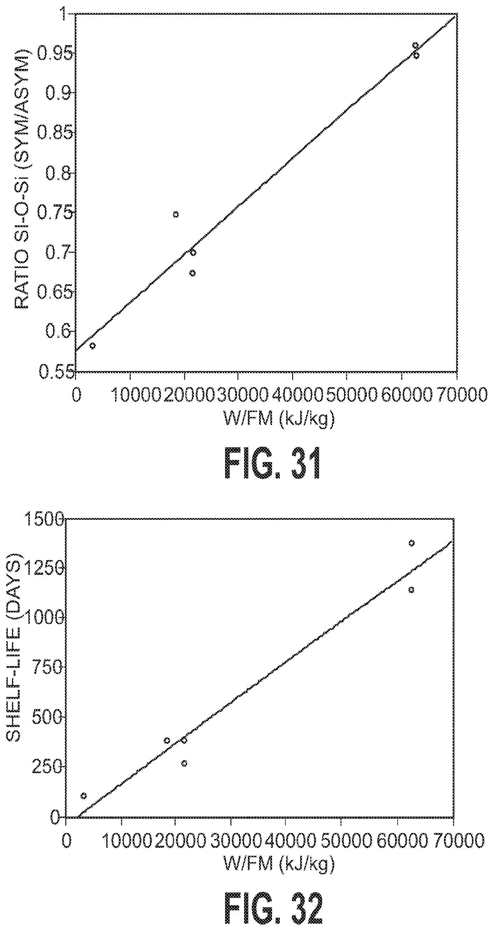

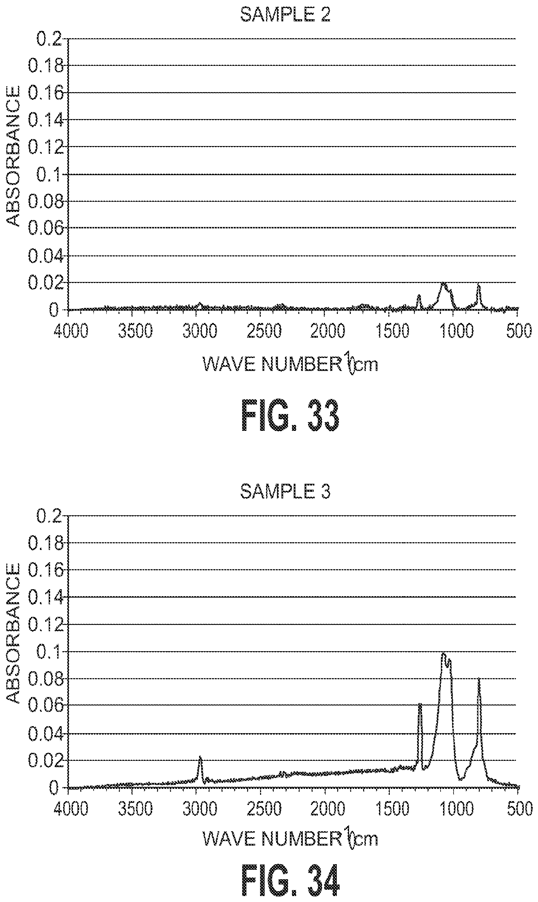

The relative cross-link density of samples 5, 6, 2, and 3 was compared between different coatings by measuring FTIR absorbance spectra. The spectra of samples 5, 6, 2, and 3 are provided in FIGS. 33 to 36. In each spectrum, the ratio of the peak absorbance at the symmetric stretching mode (1000-1040 cm⁻¹) versus the peak absorbance at the asymmetric stretching mode (1060-1100 cm⁻¹) of the Si-O-Si bond was measured, and the ratio of these two measurements was calculated, all as shown in Table 9. The respective ratios were found to have a linear correlation to the composite parameter W/FM as shown in FIG. 31.

A qualitative relation - whether the coating appeared oily (shiny, often with irridescence) or non-oily (non-shiny) when applied on the silicon chips - was also found to correlate with the W/FM values in Table 9. Oily appearing coatings deposited at lower W/FM values, as confirmed by Table 9, are believed to have a lower crosslink density, as determined by their lower sym/asym ratio, relative to the non-oily coatings that were deposited at higher W/FM and a higher cross-link density. The only exception to this general rule of thumb was sample 2 in Table 9. It is believed that the coating of sample 2 exhibited a non-oily appearance because it was was too thin to see. Thus, an oilyness observation was not reported in Table 9 for sample 2. The chips were analyzed by FTIR in transmission mode, with the infrared spectrum transmitted through the chip and sample coating, and the transmission through an uncoated null chip subtracted.

Non-oily organosiloxane layers produced at higher W/FM values, which protect the underlying SiOₓ coating from aqueous solutions at elevated pH and temperature, were preferred because they provided lower Si dissolution and a longer shelf life, as confirmed by Table 9. For example, the calculated silicon dissolution by contents of the vial at a pH of 8 and 40°C was reduced for the non-oily coatings, and the resulting shelf life was 1381 days in one case and 1147 days in another, as opposed to the much shorter shelf lives and higher rates of dissolution for oily coatings. Calculated shelf life was determined as shown for Example AA. The calculated shelf life also correlated linearly to the ratio of symmetric to asymmetric stretching modes of the Si-O- Si bond in organosiloxane pH protective coatings or layers.

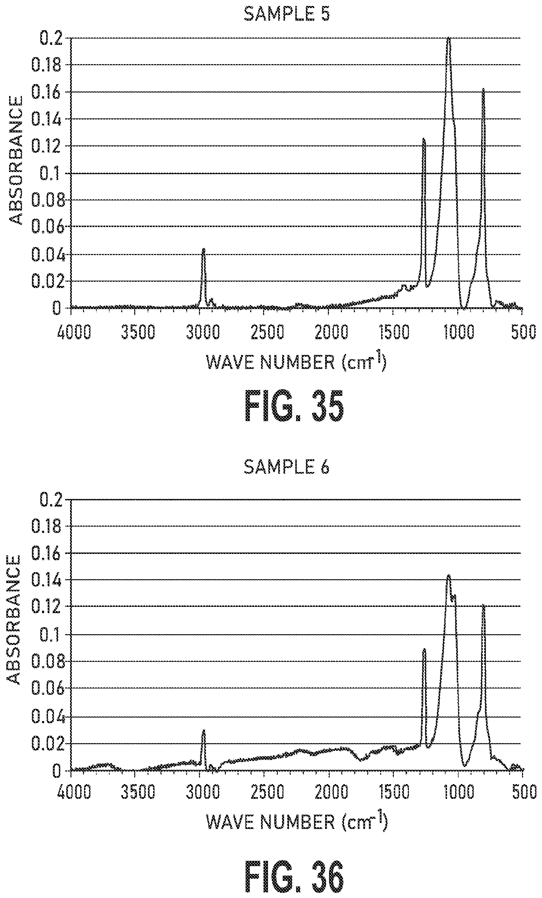

Sample 6 can be particularly compared to Sample 5. An organosiloxane, pH protective coating or layer was deposited according to the process conditions of sample 6 in Table 9. The coating was deposited at a high W/FM. This resulted in a non-oily coating with a high Si-O-Si sym/asym ratio of 0.958, which resulted in a low rate of dissolution of 84.1 ppb/day (measured by the Protocol for Determining Average Dissolution Rate) and long shelf life of 1147 days (measured by the Protocol for Determining Calculated Shelf Life). The FTIR spectra of this coating is shown in Figure 35, which exhibits a relatively similar asymmetric Si-O-Si peak absorbance compared to the symmetric Si-O-Si peak absorbance. This is an indication of a higher cross-link density coating, which is a preferred characteristic for pH protection and long shelf life.

An organosiloxane pH protective coating or layer was deposited according to the process conditions of sample 5 in Table 9. The coating was deposited at a moderate W/FM. This resulted in an oily coating with a low Si-O-Si sym/asym ratio of 0.673, which resulted in a high rate of dissolution of 236.7 ppb/day (following the Protocol for Determining Average Dissolution Rate) and shorter shelf life of 271 days (following the Protocol for Determining Calculated Shelf Life). The FTIR spectrum of this coating is shown in FIG. 13, which exhibits a relatively high asymmetric Si-O-Si peak absorbance compared to the symmetric Si-O-Si peak absorbance. This is an indication of a lower cross-link density coating, which is contemplated in any embodiment to be an unfavorable characteristic for pH protection and long shelf life.

Sample 2 can be particularly compared to Sample 3. A pH protective coating or layer was deposited according to the process conditions of sample 2 in Table 9. The coating was deposited at a low W/FM. This resulted in a coating that exhibited a low Si- O-Si sym/asym ratio of 0.582, which resulted in a high rate of dissolution of 174 ppb/day and short shelf life of 107 days. The FTIR spectrum of this coating is shown in Figure 36, which exhibits a relatively high asymmetric Si-O-Si peak absorbance compared to the symmetric Si-O-Si peak absorbance. This is an indication of a lower cross-link density coating, which is an unfavorable characteristic for pH protection and long shelf life.

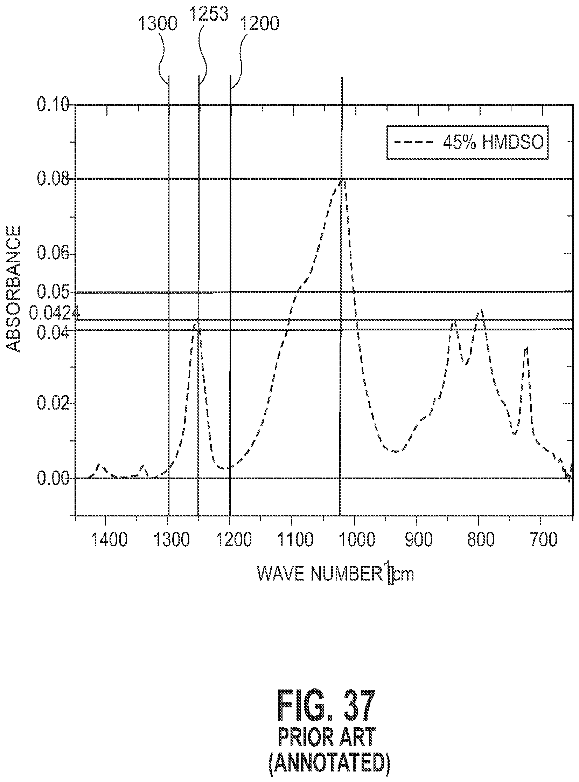

An organosiloxane, pH pH protective coating or layer was deposited according to the process conditions of sample 3 in Table 9. The coating was deposited at a high W/FM. This resulted in a non-oily coating with a high Si-O-Si sym/asym ratio of 0.947, which resulted in a low rate of Si dissolution of 79.5 ppb/day (following the Protocol for Determining Average Dissolution Rate) and long shelf life of 1381 days (following the Protocol for Determining Calculated Shelf Life). The FTIR spectrum of this coating is shown in Figure 37, which exhibits a relatively similar asymmetric Si-O-Si peak absorbance compared to the symmetric Si-O-Si peak absorbance. This is an indication of a higher cross-link density coating, which is a preferred characteristic for pH protection and long shelf life.

**TABLE 9**

| | Process Parameters | | | | | Si Dissolution @ pH8/40ºC | | | FTIR Absorbance | | | |
|---|---|---|---|---|---|---|---|---|---|---|---|---|
| Flow Rate Samples | OMCTS | Ar | O₂ Flow Rate | Power (W) | W/FM (kJ/kg) | Total Si (ppb) | Shelf life (days) | Rate of Dissolution (ppb/day) | Si-O-Si sym stretch (1000-1040 cm⁻¹) | Si-O-Si asym stretch (1060-1100 cm⁻¹) | Ratio Si-O-Si (sym/asym) | Oilyness |
| 1 | 3 | 10 | 0.5 | 14 | 21613 | 43464 | 385 | 293.18 | 0.153 | 0.219 | 0.700 | YES |
| 2 | 3 | 20 | 0.5 | 2 | 3088 | 7180 | 107 | 174.08 | 0.011 | 0.020 | 0.582 | NA |
| 3 | 1 | 20 | 0.5 | 14 | 62533 | 42252.17 | 1381 | 79.53 | 0.093 | 0.098 | 0.947 | NO |
| 4 | 2 | 15 | 0.5 | 8 | 18356 | 27398 | 380 | 187.63 | 0.106 | 0.141 | 0.748 | YES |
| 5 | 3 | 20 | 0.5 | 14 | 21613 | 24699 | 271 | 236.73 | 0.135 | 0.201 | 0.673 | YES |
| 6 | 1 | 10 | 0.5 | 14 | 62533 | 37094 | 1147 | 84.1 | 0.134 | 0.140 | 0.958 | NO |

### Example 31

An experiment similar to Example 27 was carried out, modified as indicated in this example and in Table 10 (where the results are tabulated). 100 5 mL COP vials were made and coated with an SiOₓ barrier layer and an OMCTS-based pH protective coating or layer as described previously, except that for Sample PC194 only the pH protective coating or layer was applied. The coating quantity was again measured in parts per billion extracted from the surfaces of the vials to remove the entire pH protective coating or layer, as reported in Table 10.

In this example, several different coating dissolution conditions were employed. The test solutions used for dissolution contained either 0.02 or 0.2 wt.% polysorbate-80 surfactant, as well as a buffer to maintain a pH of 8. Dissolution tests were carried out at either 23°C or 40°C. Multiple syringes were filled with each test solution, stored at the indicated temperature, and analyzed at several intervals to determine the extraction profile and the amount of silicon extracted. An average dissolution rate for protracted storage times was then calculated by extrapolating the data obtained according to the Protocol for Determining Average Dissolution Rate. The results were calculated as described previously and are shown in Table 10. Of particular note, as shown on Table 10, were the very long calculated shelf lives of the filled packages provided with a PC 194 pH protective coating or layer:
- 21045 days (over 57 years) based on storage at a pH of 8, 0.02 wt.% polysorbate-80 surfactant, at 23°C;
- 38768 days (over 100 years) based on storage at a pH of 8, 0.2 wt.% polysorbate-80 surfactant, at 23°C;
- 8184 days (over 22 years) based on storage at a pH of 8, 0.02 wt.% polysorbate-80 surfactant, at 40°C; and
- 14732 days (over 40 years) based on storage at a pH of 8, 0.2 wt.% polysorbate-80 surfactant, at 40°C.

Referring to Table 10, the longest calculated shelf lives corresponded with the use of an RF power level of 150 Watts and a corresponding high W/FM value. It is believed that the use of a higher power level causes higher cross-link density of the pH protective coating or layer.

**TABLE 10**

| **Sample** | **OMCTS Flow Rate (sccm)** | **Argon Flow Rate (sccm)** | **O₂ Flow Rate (sccm)** | **Power (W)** | **Plasma Duration (sec)** | **W/FM (kJ/kg)** | **Total Si (ppb) (OMCTS) layer)** | **Calculated Shelf-life (days)** | **Average Rate of Dissolution (ppb/day)** |
|---|---|---|---|---|---|---|---|---|---|
| | | | | | | | | | |

| | Process Parameters | | | | | | Si Dissolution @ pH8/23ºC/0.02% Tween^{®}-80 | | |
|---|---|---|---|---|---|---|---|---|---|
| PC194 | 0.5 | 20 | 0.5 | 150 | 20 | 1223335 | 73660 | 21045 | 3.5 |
| 018 | 1.0 | 20 | 0.5 | 18 | 15 | 77157 | 42982 | 1330 | 32.3 |
| | | | | | | | | | |

| | Process Parameters | | | | | | Si Dissolution @ pH8/23ºC/0.2% Tween^{®}-80 | | |
|---|---|---|---|---|---|---|---|---|---|
| PC194 | 0.5 | 20 | 0.5 | 150 | 20 | 1223335 | 73660 | 38768 | 1.9 |
| 018 | 1.0 | 20 | 0.5 | 18 | 15 | 77157 | 42982 | 665 | 64.6 |
| 048 | 4 | 80 | 2 | 35 | 20 | 37507 | 56520 | 1074 | 52.62 |
| | | | | | | | | | |

| | Process Parameters | | | | | | Si Dissolution @ pH8/40ºC/0.02% Tween^{®}-80 | | |
|---|---|---|---|---|---|---|---|---|---|
| PC194 | 0.5 | 20 | 0.5 | 150 | 20 | 1223335 | 73660 | 8184 | 9 |
| 018 | 1.0 | 20 | 0.5 | 18 | 15 | 77157 | 42982 | 511 | 84 |
| | | | | | | | | | |

| | Process Parameters | | | | | | Si Dissolution @ pH8/40ºC/0.2% Tween^{®}-80 | | |
|---|---|---|---|---|---|---|---|---|---|
| PC194 | 0.5 | 20 | 0.5 | 150 | 20 | 1223335 | 73660 | 14732 | 5 |
| 018 | 1.0 | 20 | 0.5 | 18 | 15 | 77157 | 42982 | 255 | 168 |

### Example 32

Another series of experiments similar to those of Example 31 are run, showing the effect of progressively increasing the RF power level on the FTIR absorbance spectrum of the pH protective coating or layer. The results are tabulated in Table 11, which in each instance shows a symmetric / assymmetric ratio greater than between the maximum amplitude of the Si-O-Si symmetrical stretch peak normally located between about 1000 and 1040 cm-1, and the maximum amplitude of the Si-O-Si assymmetric stretch peak normally located between about 1060 and about 1100 cm-1. Thus, the symmetric / assymmetric ratio is 0.79 at a power level of 20 W, 1.21 or 1.22 at power levels of 40, 60, or 80 W, and 1.26 at 100 Watts under otherwise comparable conditions.

The 150 Watt data in Table 11 is taken under somewhat different conditions than the other data, so it is not directly comparable with the 20 - 100 Watt data discussed above. The FTIR data of samples 6 and 8 of Table 11 was taken from the upper portion of the vial and the FTIR data of samples 7 and 9 of Table 11 was taken from the lower portion of the vial. Also, the amount of OMCTS was cut in half for samples 8 and 9 of Table 11, compared to samples 6 and 7. Reducing the oxygen level while maintaining a power level of 150 W raised the symmetric / asymmetric ratio still further, as shown by comparing samples 6 and 7 to samples 8 and 9 in Table 11.

It is believed that, other conditions being equal, increasing the symmetric / asymmetric ratio increases the shelf life of a vessel filled with a material having a pH exceeding 5.

Table 12 shows the calculated O-Parameters and N-Parameters (as defined in U.S. Pat. No. 8,067,070) for the experiments summarized in Table 11. As Table 12 shows, the O-Parameters ranged from 0.134 to 0.343, and the N-Parameters ranged from 0.408 to 0.623 - all outside the ranges claimed in U.S. Pat. No. 8,067,070.

**TABLE 11**

| **Sample s ID** e | **OMCT S Flow Rate (sccm)** | **Argon Flow Rate (sccm)** | **O₂ Flow Rate (sccm)** | **Power (W)** | **Plasma Duration (sec)** | **W/FM (kJ/kg)** | **Symmetric Stretch Peak at 1000-1040 cm-¹** | **AssymetricStretch Peak at 1060-1100 cm-¹** | **Symmetric / Assymetric Ratio** |
|---|---|---|---|---|---|---|---|---|---|
| | | | | | | | | | |

| | Process Parameters | | | | | | FTIR Results | | |
|---|---|---|---|---|---|---|---|---|---|
| 1 | 1 | 20 | 0.5 | 20 | 20 | 85,730 | 0.0793 | 0.1007 | 0.79 |
| 2 | 1 | 20 | 0.5 | 40 | 20 | 171,460 | 0.0619 | 0.0507 | 1.22 |
| 3 | 1 | 20 | 0.5 | 60 | 20 | 257,190 | 0 .1092 | 0.0904 | 1.21 |
| 4 | 1 | 20 | 0.5 | 80 | 20 | 342,919 | 0.1358 | 0.1116 | 1.22 |
| 5 | 1 | 20 | 0.5 | 100 | 20 | 428,649 | 0.209 | 0.1658 | 1.26 |
| 6 | 1 | 20 | 0.5 | 150 | 20 | 642,973 | 0.2312 | 0.1905 | 1.21 |
| 7 | 1 | 20 | 0.5 | 150 | 20 | 642,973 | 0.2324 | 0.1897 | 1.23 |
| 8 | 0.5 | 20 | 0.5 | 150 | 20 | 1,223,335 | 0.1713 | 0.1353 | 1.27 |
| 9 | 0.5 | 20 | 0.5 | 150 | 20 | 1,223,335 | 0.1475 | 0.1151 | 1.28 |

**TABLE 12**

| **Samples ID** | **OMCTS Flow Rate (sccm)** | **Argon Flow Rate (sccm)** | **O₂ Flow Rate (sccm)** | **Power (W)** | **Plasma Duration (sec)** | **W/FM (kJ/kg)** | **O-Parameter** | **N-Parameter** |
|---|---|---|---|---|---|---|---|---|
| Process Parameters | | | | | | | | |
| 1 | 1 | 20 | 0.5 | 20 | 20 | 85,730 | 0.343 | 0.436 |
| 2 | 1 | 20 | 0.5 | 40 | 20 | 171,460 | 0.267 | 0.408 |
| 3 | 1 | 20 | 0.5 | 60 | 20 | 257,190 | 0.311 | 0.457 |
| 4 | 1 | 20 | 0.5 | 80 | 20 | 342,919 | 0.270 | 0.421 |
| 5 | 1 | 20 | 0.5 | 100 | 20 | 428,649 | 0.177 | 0.406 |
| 6 | 1 | 20 | 0.5 | 150 | 20 | 642,973 | 0.151 | 0.453 |
| 7 | 1 | 20 | 0.5 | 150 | 20 | 642,973 | 0.151 | 0.448 |
| 8 | 0.5 | 20 | 0.5 | 150 | 20 | 1,223,335 | 0.134 | 0.623 |
| 9 | 0.5 | 20 | 0.5 | 150 | 20 | 1,223,335 | 0.167 | 0.609 |

### Example 33

The purpose of this example was to evaluate the recoverability or drainage of a slightly viscous aqueous solution from glass, COP and coated vials.

This study evaluated the recovery of a 30 cps (centipoise) carbohydrate solution in water-for-injection from (A) an uncoated COP vial, (B) an SiOₓ + pH protective layer coated COP vial prepared according to the above Protocol for Coating Syringe Barrel Interior with SiOₓ, followed by the Protocol for Coating Syringe Barrel Interior with OMCTS PH protective Coating or Layer, and (C) a glass vial.

2.0 ml of the carbohydrate solution was pipetted into 30 vials each of glass, COP and pH protective coated vials. The solution was aspirated from the vials with a 10 ml syringe, through a 23 gauge, 1.5" needle. The vials were tipped to one side as the solution was aspirated to maximize the amount recovered. The same technique and similar withdrawal time was used for all vials. The vials were weighed empty, after placing 2.0 ml of the solution to the vial and at the conclusion of aspirating the solution from the vial. The amount delivered to the vial (A) was determined by subtracting the weight of the empty vial from the weight of the vial with the 2.0 ml of solution. The weight of solution not recovered (B) was determined by subtracting the weight of the empty vial from the weight of the vials after aspirating the solution from the vial. The percent unrecovered was determined by dividing B by A and multiplying by 100.

It was observed during the aspiration of drug product that the glass vials remained wetted with the solution. The COP vial repelled the liquid and as the solution was aspirated from the vials. This helped with recovery but droplets were observed to bead on the sidewalls of the vials during the aspiration. The pH protective coated vials also repelled the liquid during aspiration but no beading of solution on the sidewalls was observed.

The conclusion was that pH protective coated vials do not wet with aqueous solutions as do glass vials, leading to superior recovery of drug product relative to glass. pH protective coated vials were not observed to cause beading of solution on sidewall during aspiration of aqueous products therefore coated vials performed better than uncoated COP vials in product recovery experiments.

The hydrophobic characteristics of the pH protective coating or layer may have significant benefit/use in the multi-use, cosmetic, and/or fragrance packages of the present disclosure, regardless of whether or not the pH protective coating or layer is applied over a barrier coating or layer and/or intended to protect any such barrier coating or layer from dissolution. For instance, a hydrophobic coating or layer may help ensure that more of a water-based fluid contained within the lumen of a vessel is available for extraction, e.g. by the applicator, because it does not stick to the walls of the vessel.

### Example 34

Syringe samples were produced as follows. A COC 8007 extended barrel syringe was produced according to the Protocol for Forming COC Syringe Barrel. An SiOₓ coating or layer was applied to some of the syringes according to the Protocol for coating COC Syringe Barrel Interior with SiOₓ. A pH protective coating or layer was applied to the SiOₓ coated syringes according to the Protocol for Coating COC Syringe Barrel Interior with OMCTS Lubricity Coating, modified as follows. The OMCTS was supplied from a vaporizer, due to its low volatility. Argon carrier gas was used. The process conditions were set to the following:
- OMCTS - 3 sccm
- Argon gas - 65 sccm
- Power - 6 watts
- Time - 10 seconds

The coater was later determined to have a small leak while producing the samples identified in the Table, which resulted in an estimated oxygen flow of 1.0 sccm. The samples were produced without introducing oxygen.

The coatings produced according to these working examples are contemplated to function as primer coatings or layers, and also as protective coatings or layers to increase the shelf life of the vessels, compared to similar vessels provided with a barrier coating or layer but no pH protective coating or layer.

### PECVD Process for Trilayer Coating

The PECVD trilayer coating described in this specification can be applied, for example, as follows for a 1 to 5 mL vessel. Two specific examples are 1 mL thermoplastic resin syringe and a 5 mL thermoplastic resin drug vial. Though the present application is not specifically directed to these syringes and vials, larger or smaller vessels for various multi-use, cosmetic, and/or fragrance packages, will call for adjustments in parameters that a person of ordinary skill can carry out in view of the teaching of this specification.

The apparatus used is the PECVD apparatus with rotating quadrupole magnets as described generally in this specification.

The general coating parameter ranges, with preferred ranges in parentheses, for a trilayer coating for a 1 mL syringe barrel are shown in the PECVD Trilayer Process General Parameters Tables (1 mL syringe and 5 mL vial).

| **PECVD Trilayer Process General Parameters Table (1 mL syringe)** | | | | |
|---|---|---|---|---|
| **Parameter** | **Units** | **Tie** | **Barrier** | **pH Protective** |
| Power | W | 40-90 (60-80) | 140 | 40-90 (60-80) |
| TMDSO Flow | sccm | 1-10 (3-5) | None | 1-10 (3-5) |
| HMDSO Flow | sccm | None | 1.56 | None |
| O₂ Flow | sccm | 0.5-5 (1.5-2.5) | 20 | 0.5-5 (1.5-2.5) |
| Argon Flow | sccm | 40-120 (70-90) | 0 | 40-120 (70-90) |
| Ramp Time | seconds | None | None | None |
| Deposition Time | seconds | 0.1-10 (1-3) | 20 | 0.1-40 (15-25) |
| Tube Pressure | Torr | 0.01-10 (0.1-1.5) | 0.59 | 0.01-10 (0.1-1.5) |

| **PECVD Trilayer Process General Parameters Table (5 mL vial)** | | | | |
|---|---|---|---|---|
| **Parameter** | **Units** | **Adhesion** | **Barrier** | **Protection** |
| Power | W | 40-90 (60-80) | 140 | 40-90 (60-80) |
| TMDSO Flow | sccm | 1-10 (3-5) | None | 1-10 (3-5) |
| HMDSO Flow | sccm | None | 1.56 | None |
| O₂ Flow | sccm | 0.5-5 (1.5-2.5) | 20 | 0.5-5 (1.5-2.5) |
| Argon Flow | sccm | 40-120 (70-90) | 0 | 40-120 (70-90) |
| Ramp Time | seconds | None | None | None |
| Deposition Time | seconds | 0.1-10 (1-3) | 20 | 0.1-40 (15-25) |
| Tube Pressure | Torr | 0.01-10 (0.1-1.5) | 0.59 | 0.01-10 (0.1-1.5) |

### Example 35

Examples of specific coating parameters that have been used for a 1 mL syringe and 5 mL vial are shown in the PECVD Trilayer Process Specific Parameters Tables (1 mL syringe and 5 mL vial):

| **PECVD Trilayer Process Specific Parameters Table (1 mL syringe)** | | | | |
|---|---|---|---|---|
| **Parameter** | **Units** | **Tie** | **Barrier** | **pH Protective** |
| Power | W | 70 | 140 | 70 |
| TMDSO Flow | sccm | 4 | None | 4 |
| HMDSO Flow | sccm | None | 1.56 | None |
| O₂ Flow | sccm | 2 | 20 | 2 |
| Argon Flow | sccm | 80 | 0 | 80 |
| Ramp Time | seconds | None | None | None |
| Deposition Time | seconds | 2.5 | 20 | 10 |
| Tube Pressure | Torr | 1 | 0.59 | 1 |

| **PECVD Trilayer Process Specific Parameters Table (5 mL vial)** | | | | |
|---|---|---|---|---|
| **Parameter** | **Units** | **Adhesion** | **Barrier** | **Protection** |
| Power | W | 20 | 40 | 20 |
| TMDSO Flow | sccm | 2 | 0 | 2 |
| HMDSO Flow | sccm | 0 | 3 | 0 |
| O₂ Flow | sccm | 1 | 50 | 1 |
| Argon Flow | sccm | 20 | 0 | 20 |
| Ramp Time | seconds | 0 | 2 | 2 |
| Deposition Time | seconds | 2.5 | 10 | 10 |
| Tube Pressure | Torr | 0.85 | 1.29 | 0.85 |

The O-parameter and N-parameter values for the pH protective coating or layer applied to the 1 mL syringe as described above are 0.34 and 0.55, respectively.

The O-parameter and N-parameter values for the pH protective coating or layer applied to the 5 mL vial are 0.24 and 0.63, respectively.

### Example 36

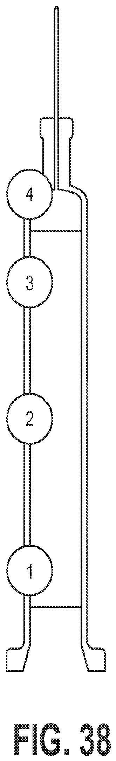

Referring to Fig. 38 and Table, Example 36, the thickness uniformity at four different points along the length of a 1 mL syringe with a staked needle (present during PECVD deposition) and the indicated trilayer coating (avg. thicknesses: 38 nm adhesion or tie coating or layer; 55 nm barrier coating or layer, 273 nm pH protective coating or layer) is shown. The table shows individual layer thicknesses at the four marked points, showing adequate thickness of each layer at each point along the high profile syringe barrel.

**TABLE, Example 36**

| **Syringe Location** | **Adhesion** | **Barrier** | **Protection** |
|---|---|---|---|
| 1 | 46 | 75 | 343 |
| 2 | 38 | 55 | 273 |
| 3 | 86 | 47 | 493 |
| 4 | 42 | 25 | 287 |

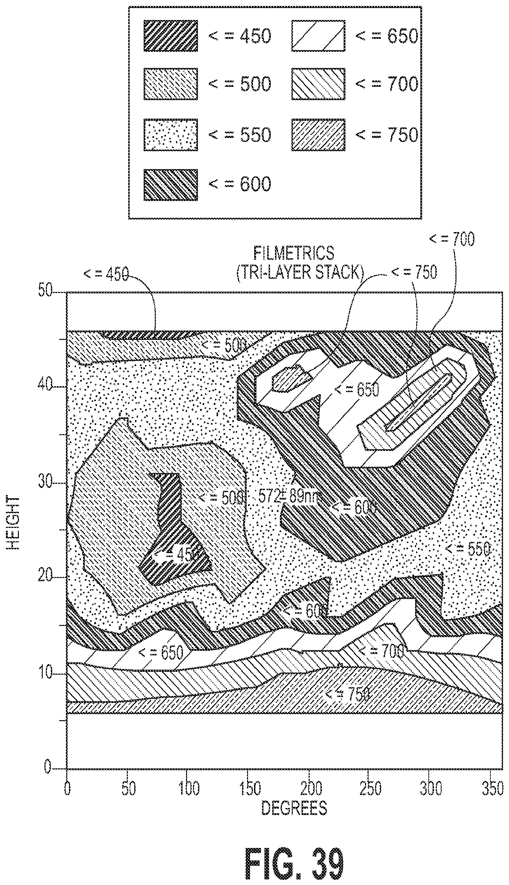

Referring to FIG. 39, the plot maps the coating thickness over the portion of the cylindrical inner surface of the barrel shown in FIG. 38, as though unrolled to form a rectangle. The overall range of thickness of the trilayer coating is 572 plus or minus 89 nm.

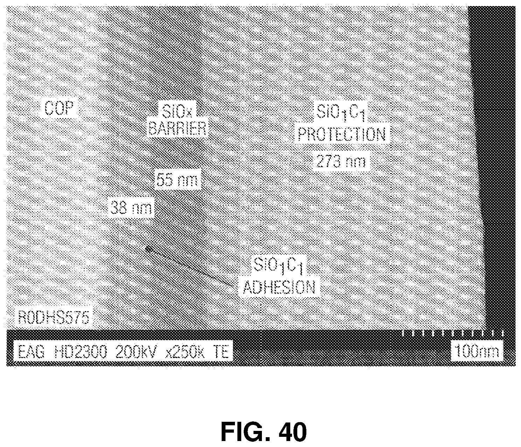

FIG. 40 is a photomicrograph showing a cross-section of the trilayer coating on a COP syringe substrate at the point 2 shown in FIG. 38.

A syringe having a coating similar to the trilayer coating of FIGS. 38-40 is tested for shelf life, using the silicon dissolution and extrapolation method described in this specification, compared to syringes having a bilayer coating (similar to the trilayer coating except lacking the tie coating or layer) and a monolayer coating which is just the pH protective coating or layer directly applied to the thermoplastic barrel of the syringe, with no barrier layer. The test solution was a 0.2% Tween, pH 8 phosphate buffer. The extrapolated shelf lives of the monolayer and trilayer coatings were similar and very long - on the order of 14 years. The shelf life of the syringes having a bilayer coating were much lower - less than two years. In other words, the presence of a barrier layer under the pH protective layer shortened the shelf life of the coating substantially, but the shelf life was restored by providing a tie coating or layer under the barrier layer, sandwiching the barrier coating or layer with respective SiOₓC_{y} layers. The barrier layer is necessary to establish a gas barrier, so the monolayer coating would not be expected to provide adequate gas barrier properties by itself. Thus, only the trilayer coating had the combination of gas barrier properties and a long shelf life, even while in contact with a solution that would attack an exposed barrier coating or layer.

### Example 37

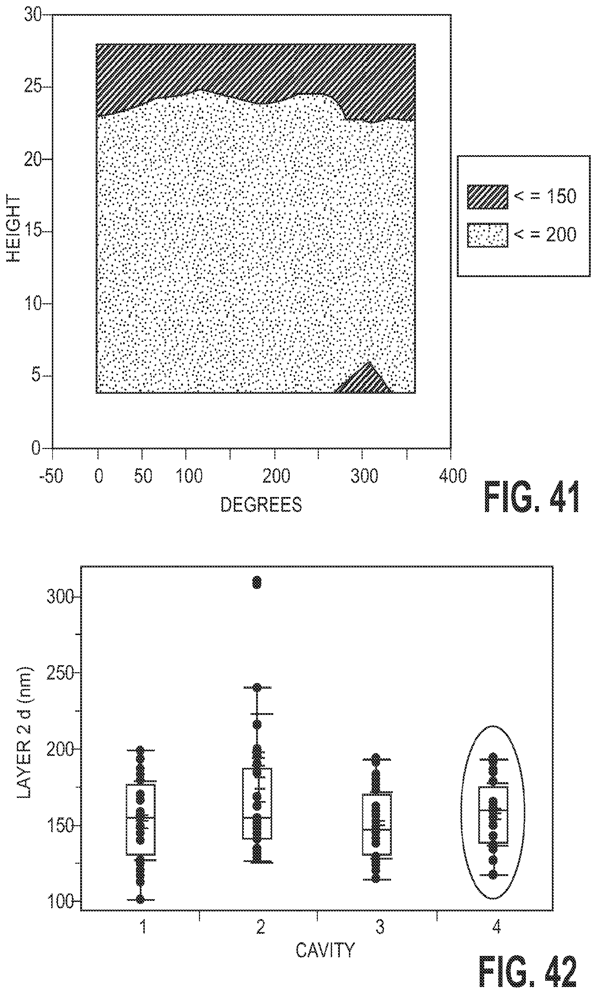

FIGS. 41 and 42 show a trilayer coating distribution for the 5 mL vial, which is much shorter in relation to its inner diameter and thus easier to coat uniformly, showing very little variation in coating thickness, with the great majority of the surface coated between 150 and 250 nm thickness of the trilayer, with only a small proportion of the container coated with between 50 and 250 nm of the trilayer.

### Example 38

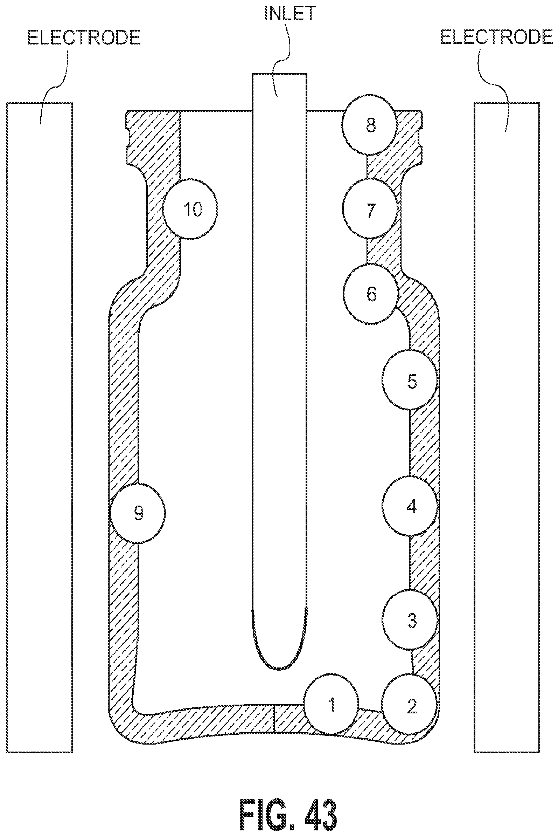

Figure 43 shows the breakdown of coating thickness (nm) by vial location. The Vial Coating Distribution Table shows the uniformity of coating.

| **Vial Coating Distribution Table** | | | | |
|---|---|---|---|---|
| **Vial Location** | **Adhesion** | **Barrier** | **Protection** | **Total Trilayer,nm** |
| 1 | 13 | 29 | 77 | 119 |
| 2 | 14 | 21 | 58 | 93 |
| 3 | 25 | 37 | 115 | 177 |
| 4 | 35 | 49 | 158 | 242 |
| 5 | 39 | 49 | 161 | 249 |
| 6 | 33 | 45 | 148 | 226 |
| 7 | 31 | 29 | 153 | 213 |
| 8 | 48 | 16 | 218 | 282 |
| 9 | 33 | 53 | 155 | 241 |
| 10 | 31 | 29 | 150 | 210 |
| Average | 30 | 36 | 139 | 205 |

### Example 39

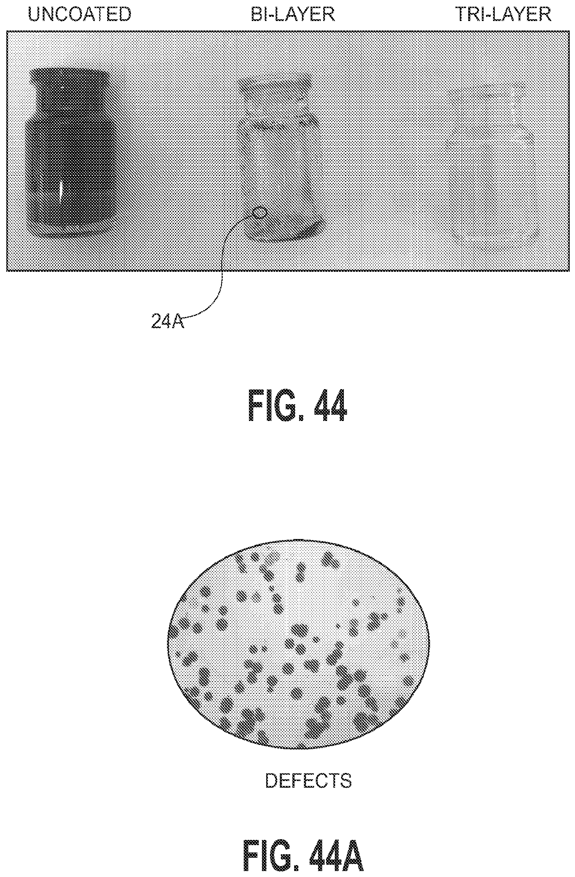

Fig. 44 is a visual test result showing the integrity of the trilayer vial coating described above. The three 5 mL cyclic olefin polymer (COC) vials of FIGS. 44 and 44A were respectively:
- uncoated (left vial),
- coated with the bilayer coating described in this specification (a barrier coating or layer plus a pH protective coating or layer - the second and third components of the trilayer coating) (center vial); and
- coated with the trilayer coating as described above (right vial).

The three vials were each exposed to 1 N potassium hydroxide for four hours, then exposed for 24 hours to a ruthenium oxide (RuO4) stain that darkens any exposed part of the thermoplastic vial unprotected by the coatings. The high pH potassium hydroxide exposure erodes any exposed part of the barrier coating or layer at a substantial rate, greatly reduced, however by an intact pH protective coating or layer. In particular, the high pH exposure opens up any pinholes in the coating system. As FIG. 44 shows, the uncoated vial is completely black, showing the absence of any effective coating. The bilayer coating was mostly intact under the treatment conditions, but on microscopic inspection has many pinholes (illustrated by FIG. 44A) where the ruthenium stain reached the thermoplastic substrate through the coating. The overall appearance of the bilayer coating clearly shows visible "soiled" areas where the stain penetrated. The trilayer coating, however, protected the entire vial against penetration of the stain, and the illustrated vial remains clear after treatment. This is believed to be the result of sandwiching the barrier coating or layer between two layers of SiOxCy, which both protects the barrier layer against direct etching and against undercutting and removal of flakes of the barrier layer.

### Protocol for Total Silicon Measurement

This protocol is used to determine the total amount of silicon coatings present on the entire vessel wall. A supply of 0.1 N potassium hydroxide (KOH) aqueous solution is prepared, taking care to avoid contact between the solution or ingredients and glass. The water used is purified water, 18 MΩ quality. A Perkin Elmer Optima Model 7300DV ICP-OES instrument is used for the measurement except as otherwise indicated.

Each device (vial, syringe, tube, or the like) to be tested and its cap and crimp (in the case of a vial) or other closure are weighed empty to 0.001 g, then filled completely with the KOH solution (with no headspace), capped, crimped, and reweighed to 0.001g. In a digestion step, each vial is placed in an autoclave oven (liquid cycle) at 121°C for 1 hour. The digestion step is carried out to quantitatively remove the silicon coatings from the vessel wall into the KOH solution. After this digestion step, the vials are removed from the autoclave oven and allowed to cool to room temperature. The contents of the vials are transferred into ICP tubes. The total Si concentration is run on each solution by ICP/OES following the operating procedure for the ICP/OES.

The total Si concentration is reported as parts per billion of Si in the KOH solution. This concentration represents the total amount of silicon coatings that were on the vessel wall before the digestion step was used to remove it.

The total Si concentration can also be determined for fewer than all the silicon layers on the vessel, as when an SiOx barrier layer is applied, an SiOxCy second layer (for example, a lubricity layer or a primer coating or layer) is then applied, and it is desired to know the total silicon concentration of just the SiOxCy layer. This determination is made by preparing two sets of vessels, one set to which only the SiOx layer is applied and the other set to which the same SiOx layer is applied, followed by the SiOxCy layer or other layers of interest. The total Si concentration for each set of vessels is determined in the same manner as described above. The difference between the two Si concentrations is the total Si concentration of the SiOₓC_{y} second layer.

### Protocol for Measuring Dissolved Silicon in a Vessel

In some of the working examples, the amount of silicon dissolved from the wall of the vessel by a test solution is determined, in parts per billion (ppb), for example to evaluate the dissolution rate of the test solution. This determination of dissolved silicon is made by storing the test solution in a vessel provided with an SiOx and/or SiOxCy coating or layer under test conditions, then removing a sample of the solution from the vessel and testing the Si concentration of the sample. The test is done in the same manner as the Protocol for Total Silicon Measurement, except that the digestion step of that protocol is replaced by storage of the test solution in the vessel as described in this protocol. The total Si concentration is reported as parts per billion of Si in the test solution

### Protocol for Determining Average Dissolution Rate

The average dissolution rates reported in the working examples are determined as follows. A series of test vessels having a known total total silicon measurement are filled with the desired test solution analogous to the manner of filling the vials with the KOH solution in the Protocol for Total Silicon Measurement. (The test solution can be a physiologically inactive test solution as employed in the present working examples or a physiologically active pharmaceutical preparation intended to be stored in the vessels to form a pharmaceutical package). The test solution is stored in respective vessels for several different amounts of time, then analyzed for the Si concentration in parts per billion in the test solution for each storage time. The respective storage times and Si concentrations are then plotted. The plots are studied to find a series of substantially linear points having the steepest slope.

The plot of dissolution amount (ppb Si) versus days decreases in slope with time, even though it does not appear thatthe Si layer has been fully digested by the test solution.

For the PC194 test data in Table 10 below, linear plots of dissolution versus time data are prepared by using a least squares linear regression program to find a linear plot corresponding to the first five data points of each of the experimental plots. The slope of each linear plot is then determined and reported as representing the average dissolution rate applicable to the test, measured in parts per billion of Si dissolved in the test solution per unit of time.

### Protocol for Determining Calculated Shelf Life

The calculated shelf life values reported in the working examples are determined by extrapolation of the total silicon measurements and average dissolution rates, respectively determined as described in the Protocol for Total Silicon Measurement and the Protocol for Determining Average Dissolution Rate. The assumption is made that under the indicated storage conditions the SiOxCy primer coating or layer will be removed at the average dissolution rate until the coating is entirely removed. Thus, the total silicon measurement for the vessel, divided by the dissolution rate, gives the period of time required for the test solution to totally dissolve the SiOxCy coating. This period of time is reported as the calculated shelf life. Unlike commercial shelf life calculations, no safety factor is calculated. Instead, the calculated shelf life is the calculated time to failure.

It should be understood that because the plot of ppb Si versus hours decreases in slope with time, an extrapolation from relatively short measurement times to relatively long calculated shelf lives is believed to be a "worst case" test that tends to underestimate the calculated shelf life actually obtainable.

## Claims

1. A package comprising:
a vessel comprising one or more walls that enclose at least a portion of a lumen;
a fluid within the lumen, the fluid being present in an amount that is configured for a plurality of doses or applications; and
an anti-microbial coating on an interior surface of the one or more walls, wherein the anti-microbial coating is in contact with the fluid, **characterised in that** the anti-microbial coating comprises
zinc oxide (ZnO) applied by PECVD, ALD, or PEALD from a feed gas or feed gases comprising zinc acetate, diethyl zinc, or a combination thereof, and an oxidant;
titanium dioxide (TiO₂) applied by PECVD or ALD from a feed gas or feed gases comprising titanium tetrachloride and an oxidant; or
silver oxide (Ag₂O) applied by PECVD or ALD from a feed gas or feed gases comprising an organosilver compound and an oxidant, wherein the organosilver compound has the composition:
Ag(Hfac)(PR₃)
in which Hfac is 1,1,1,5,5,5-hexafluoroacetylacetonate, P is phosphine, and R is methyl, ethyl, or a combination thereof;
wherein the anti-microbial coating is effective to inhibit the growth of microbes, such as bacteria, in the fluid within the lumen.

2. The package of claim 1, wherein the fluid is a drug or medical product, a cosmetic product, or a skin care product.

3. The package of claim 1 or 2, wherein the fluid is an aseptic or sterile fluid.

4. The package of any preceding claim, wherein the package further comprises an applicator for the fluid, and wherein the applicator is susceptible to bacterial contamination.

5. The package of any preceding claim, wherein the anti-microbial coating has been applied by PECVD.

6. The package of any one of claims 1-4, in which the anti-microbial coating consists essentially of a plurality of atomic monolayers, wherein the anti-microbial coating or layer has been applied by atomic layer deposition (ALD) or by plasma-enhanced atomic layer deposition (PEALD).

7. The package of claim 5, in which the anti-microbial coating comprises zinc oxide (ZnO) applied by PECVD from a feed gas comprising the zinc acetate, diethyl zinc, or a combination thereof, and the oxidant; or
titanium dioxide (TiO₂) applied by PECVD from a feed gas comprising the titanium tetrachloride and the oxidant; or
silver oxide (Ag₂O) applied by PECVD from a feed gas comprising the organosilver compound and an oxidant.

8. The package of any one of claims 1-4, in which the anti-microbial coating comprises
zinc oxide (ZnO) applied by ALD or PEALD using feed gases comprising the zinc acetate, diethyl zinc, or a combination thereof, and the oxidant; or
titanium dioxide (TiO₂) applied by ALD or PEALD using feed gases comprising the titanium tetrachloride, titanium isopropoxide, or combination thereof, and the oxidant; or
silver oxide (Ag₂O) applied by PECVD using feed gases comprising the organosilver compound and the oxidant.

9. The package of any preceding claim, in which the oxidant is selected from O₂, O₃, H₂O, H₂O₂, N₂O, NO₂, air, or a combination thereof.

10. The package of claim 9, in which the oxidant is O₂.

11. The package of any preceding claim, which is a multi-dose eye drop bottle comprising:
a dropper tip at the opening of the lumen;
an ophthalmic medical fluid within the lumen.

12. Use of the package of any preceding claim to prevent microbial contamination of the fluid product within the lumen after first use, such as by bacteria that may enter the fluid via an applicator or dropper tip.

13. A method of preventing contamination of a fluid product, optionally an aseptic or sterile fluid product, after first use, the method comprising:
providing the fluid product in a package comprising an anti-microbial coating in accordance with any one of claims 1-11.

14. The method of claim 13, wherein the fluid product is an ophthalmic medical fluid product, the method comprising:
providing the ophthalmic medical fluid product in a multi-dose eye drop bottle in accordance with claim 11.

## Patentansprüche

1. Eine Verpackung, umfassend:
einen Behälter, der eine oder mehrere Wände umfasst, die zumindest einen Teil eines Lumens umschließen;
einer Flüssigkeit innerhalb des Lumens, wobei die Flüssigkeit in einer Menge vorliegt, die für mehrere Dosen oder Anwendungen ausgelegt ist; und
eine antimikrobielle Beschichtung auf einer Innenfläche der einen oder mehreren Wände, wobei die antimikrobielle Beschichtung mit der Flüssigkeit in Kontakt steht, **dadurch gekennzeichnet, dass** die antimikrobielle Beschichtung umfasst:
Zinkoxid (ZnO), das mittels PECVD, ALD oder PEALD aus einem oder mehreren Speisegasen aufgebracht wird, die Zinkacetat, Diethylzink oder eine Kombination davon sowie ein Oxidationsmittel umfassen;
Titandioxid (TiO₂), das mittels PECVD oder ALD aus einem oder mehreren Speisegasen aufgebracht wird, die Titantetrachlorid und ein Oxidationsmittel umfassen; oder
Silberoxid (Ag₂O), das mittels PECVD oder ALD aus einem oder mehreren Speisegasen aufgebracht wird, die eine Organosilberverbindung und ein Oxidationsmittel umfassen, wobei die Organosilberverbindung die folgende Zusammensetzung aufweist:
Ag(Hfac)(PR₃)
worin Hfac 1,1,1,5,5,5-Hexafluoracetylacetonat ist, P Phosphin ist und R Methyl, Ethyl oder eine Kombination davon ist;
wobei die antimikrobielle Beschichtung das Wachstum von Mikroben, wie beispielsweise Bakterien, in der Flüssigkeit innerhalb des Lumens wirksam hemmt.

2. Die Verpackung nach Anspruch 1, wobei die Flüssigkeit ein Arzneimittel oder Medizinprodukt, ein Kosmetikprodukt oder ein Hautpflegeprodukt ist.

3. Die Verpackung nach Anspruch 1 oder 2, wobei die Flüssigkeit eine aseptische oder sterile Flüssigkeit ist.

4. Die Verpackung nach einem der vorstehenden Ansprüche, wobei die Verpackung ferner einen Applikator für die Flüssigkeit umfasst und wobei der Applikator anfällig für bakterielle Kontamination ist.

5. Die Verpackung nach einem der vorstehenden Ansprüche, wobei die antimikrobielle Beschichtung mittels PECVD aufgebracht wurde.

6. Die Verpackung nach einem der Ansprüche 1 bis 4, wobei die antimikrobielle Beschichtung im Wesentlichen aus einer Vielzahl von monoatomaren Schichten besteht, wobei die antimikrobielle Beschichtung oder Schicht durch Atomlagenabscheidung (ALD) oder durch plasmaunterstützte Atomlagenabscheidung (PEALD) aufgebracht wurde.

7. Die Verpackung nach Anspruch 5, wobei die antimikrobielle Beschichtung Zinkoxid (ZnO), das mittels PECVD aus einem Speisegas aufgebracht wurde, welches das Zinkacetat, Diethylzink oder eine Kombination davon sowie das Oxidationsmittel umfasst; oder
Titandioxid (TiO₂), das mittels PECVD aus einem Speisegas aufgebracht wurde, welches das Titantetrachlorid und ein Oxidationsmittel umfasst; oder
Silberoxid (Ag₂O), das mittels PECVD aus einem Speisegas aufgebracht wurde, das die Organosilberverbindung und ein Oxidationsmittel umfasst.

8. Das Verfahren nach einem der Ansprüche 1 bis 4, wobei die antimikrobielle Beschichtung umfasst
Zinkoxid (ZnO), das mittels ALD oder PEALD unter Verwendung von Speisegasen aufgebracht wurde, welche das Zinkacetat, Diethylzink oder eine Kombination davon sowie das Oxidationsmittel umfassen; oder
Titandioxid (TiO₂), das mittels ALD oder PEALD unter Verwendung von Speisegasen aufgebracht wurde, welche das Titantetrachlorid, Titanisopropoxid oder eine Kombination davon sowie das Oxidationsmittel umfassen; oder
Silberoxid (Ag₂O), das mittels PECVD unter Verwendung von Speisegasen aufgebracht wurde, welche die Organosilberverbindung und das Oxidationsmittel umfassen.

9. Die Verpackung nach einem der vorstehenden Ansprüche, bei der das Oxidationsmittel aus O₂, O₃, H₂O, H₂O₂, N₂O, NO₂, Luft oder einer Kombination davon ausgewählt ist.

10. Die Verpackung nach Anspruch 9, wobei das Oxidationsmittel O₂ ist.

11. Die Verpackung nach einem der vorstehenden Ansprüche, welche eine Mehrdosen-Augentropfenflasche ist, umfassend:
eine Tropfspitze an der Öffnung des Lumens;
eine ophthalmologische medizinische Flüssigkeit innerhalb des Lumens.

12. Verwendung der Verpackung nach einem der vorstehenden Ansprüche zur Verhinderung einer mikrobiellen Kontamination des Flüssigkeitsprodukts im Lumen nach dem ersten Gebrauch, beispielsweise durch Bakterien, die über einen Applikator oder eine Tropfspitze in die Flüssigkeit gelangen können.

13. Ein Verfahren zur Verhinderung einer Kontamination eines Flüssigkeitsprodukts, optional eines aseptischen oder sterilen Flüssigkeitsprodukts, nach der ersten Verwendung, wobei das Verfahren umfasst:
Bereitstellen des Flüssigkeitsprodukts in einer Verpackung, die eine antimikrobielle Beschichtung umfasst, gemäß einem der Ansprüche 1 bis 11.

14. Das Verfahren nach Anspruch 13, wobei das Flüssigkeitsprodukt ein ophthalmologisches medizinisches Flüssigkeitsprodukt ist, wobei das Verfahren umfasst:
Bereitstellen des ophthalmologischen medizinischen Flüssigkeitsprodukts in einer Mehrdosen-Augentropfenflasche gemäß Anspruch 11.

## Revendications

1. Un emballage comprenant :
un récipient comprenant une ou plusieurs parois qui délimitent au moins une partie d'une lumière ;
un fluide à l'intérieur de la lumière, le fluide étant présent en une quantité qui est configurée pour une pluralité de doses ou d'applications ; et
un revêtement antimicrobien sur une surface intérieure de la ou des parois, ledit revêtement antimicrobien étant en contact avec le fluide, **caractérisé en ce que**
le revêtement antimicrobien
comprend
de l'oxyde de zinc (ZnO) appliqué par PECVD, ALD ou PEALD à partir d'un gaz d'alimentation ou de gaz d'alimentation comprenant de l'acétate de zinc, du diéthylzinc, ou une combinaison de ceux-ci, et un oxydant ; du dioxyde de titane (TiO₂) appliqué par PECVD ou ALD à partir d'un gaz d'alimentation ou de gaz d'alimentation comprenant du tétrachlorure de titane et un oxydant ; ou de l'oxyde d'argent (Ag₂O) appliqué par PECVD ou ALD à partir d'un gaz d'alimentation ou de gaz d'alimentation comprenant un composé organoargentique et un oxydant, dans lequel le composé organoargentique a la composition :
Ag(Hfac)(PR₃)
dans laquelle Hfac est le 1,1,1,5,5,5-hexafluoroacétylacétonate, P est la phosphine, et R est un méthyle, un éthyle, ou une combinaison de ceux-ci ;
dans lequel le revêtement antimicrobien est efficace pour inhiber la croissance de microbes, tels que des bactéries, dans le fluide à l'intérieur de la lumière.

2. L'emballage selon la revendication 1, dans lequel le fluide est un médicament ou un produit médical, un produit cosmétique, ou un produit de soin de la peau.

3. L'emballage selon la revendication 1 ou 2, dans lequel le fluide est un fluide aseptique ou stérile.

4. L'emballage selon l'une quelconque des revendications précédentes, dans lequel l'emballage comprend en outre un applicateur pour le fluide, et dans lequel l'applicateur est susceptible de contamination bactérienne.

5. L'emballage selon l'une quelconque des revendications précédentes, dans lequel le revêtement antimicrobien a été appliqué par PECVD.

6. L'emballage selon l'une quelconque des revendications 1 à 4, dans lequel le revêtement antimicrobien est constitué essentiellement d'une pluralité de monocouches atomiques, dans lequel le revêtement ou la couche antimicrobien a été appliqué par dépôt de couches atomiques (ALD) ou par dépôt de couches atomiques assisté par plasma (PEALD).

7. L'emballage selon la revendication 5, dans lequel le revêtement antimicrobien comprend de l'oxyde de zinc (ZnO) appliqué par PECVD à partir d'un gaz d'alimentation comprenant l'acétate de zinc, le diéthylzinc, ou une combinaison de ceux-ci, et l'oxydant ; ou
du dioxyde de titane (TiO₂) appliqué par PECVD à partir d'un gaz d'alimentation comprenant le tétrachlorure de titane et l'oxydant ; ou
de l'oxyde d'argent (Ag₂O) appliqué par PECVD à partir d'un gaz d'alimentation comprenant le composé organoargentique et un oxydant.

8. L'emballage selon l'une quelconque des revendications 1 à 4, dans lequel le revêtement antimicrobien comprend
de l'oxyde de zinc (ZnO) appliqué par ALD ou PEALD en utilisant des gaz d'alimentation comprenant l'acétate de zinc, le diéthylzinc, ou une combinaison de ceux-ci, et l'oxydant ; ou
du dioxyde de titane (TiO₂) appliqué par ALD ou PEALD en utilisant des gaz d'alimentation comprenant le tétrachlorure de titane, l'isopropoxyde de titane, ou une combinaison de ceux-ci, et l'oxydant ; ou
de l'oxyde d'argent (Ag₂O) appliqué par PECVD en utilisant des gaz d'alimentation comprenant le composé organoargentique et l'oxydant.

9. L'emballage selon l'une quelconque des revendications précédentes, dans lequel l'oxydant est choisi parmi O₂, O₃, H₂O, H₂O₂, N₂O, NO₂, l'air, ou une combinaison de ceux-ci.

10. L'emballage de la revendication 9, dans lequel l'oxydant est O₂.

11. L'emballage selon l'une quelconque des revendications précédentes, qui est un flacon compte-gouttes multidose pour les yeux comprenant :
un embout compte-gouttes à l'ouverture de la lumière ;
un fluide médical ophtalmique à l'intérieur de la lumière.

12. Utilisation de l'emballage selon l'une quelconque des revendications précédentes pour prévenir la contamination microbienne du produit fluide à l'intérieur de la lumière après la première utilisation, notamment par des bactéries susceptibles de pénétrer dans le fluide par l'intermédiaire d'un applicateur ou d'un embout compte-gouttes.

13. Procédé pour empêcher la contamination d'un produit fluide, éventuellement un produit fluide aseptique ou stérile, après une première utilisation, le procédé comprenant :
la fourniture du produit fluide dans un emballage comprenant un revêtement antimicrobien selon l'une quelconque des revendications 1 à 11.

14. Le procédé selon la revendication 13, dans lequel le produit fluide est un produit fluide médical ophtalmique, le procédé comprenant :
la fourniture du produit fluide médical ophtalmique dans un flacon compte-gouttes multidose pour les yeux selon la revendication 11.
